# EUROPÄISCHE PATENTSCHRIFT

(11) **EP 3 272 300 B1**
(45) Veröffentlichungstag und Bekanntmachung des Hinweises auf die Patenterteilung: **11.03.2020**
(21) Anmeldenummer: 17178422.6
(22) Anmeldetag: 28.06.2017
(51) Int. Cl.: A61B 17/88, B01F 13/04, B01F 15/00, B05C 17/005, B05C 17/01, B65D 81/32, B65D 83/00

(54) **KNOCHENZEMENT-APPLIKATOR MIT DREIWEGE-VENTIL ZUR DRUCKENTLASTUNG**
BONE CEMENT APPLICATOR WITH THREE-WAY VALVE FOR PRESSURE RELIEF
APPLICATEUR DE CIMENT OSSEUX À L'AIDE D'UNE VANNE TROIS VOIES DESTINÉE À LA DÉCHARGE DE PRESSION

(30) Priorität: 21.07.2016 DE 102016113467
(43) Veröffentlichungstag der Anmeldung: 24.01.2018
(73) Patentinhaber: Heraeus Medical GmbH, 61273 Wehrheim (DE)
(72) Erfinder: Vogt, Sebastian Dr., 99092 Erfurt (DE); Kluge, Thomas Dr., 56179 Vallendar (DE)
(74) Vertreter: Schultheiss & Sterzel Patentanwälte PartG mbB

(56) Entgegenhaltungen:
- EP-A1- 2 447 184
- WO-A1-2007/000066
- DE-A1-102005 017 599
- DE-A1-102011 010 763
- DE-U1-202014 102 416
- US-A1- 2010 262 152

## Beschreibung

Die Erfindung betrifft einen Knochenzement-Applikator zur Applikation eines Knochenzements im Bereich der Wirbelsäule, der bevorzugt auch zum Lagern der Ausgangskomponenten geeignet ist.

Die Erfindung betrifft auch ein Verfahren zur Applikation eines Knochenzements, insbesondere eines pastenförmigen Mehrkomponenten-Polymethylmethacrylat-Knochenzementteigs, mit einem solchen Knochenzement-Applikator.

Gegenstand der Erfindung ist insbesondere ein einfacher, kostengünstig herzustellender Knochenzement-Applikator für die Vertebroplastie mit pastenförmigen Mehrkomponenten-Polymethylmethacrylat-Knochenzementen, mit dem hochviskose pastenförmige Ausgangskomponenten des Polymethylmethacrylat-Knochenzements auch mit manuell bedienbaren Auspressvorrichtungen gemischt und ausgetragen werden können.

Konventionelle Polymethylmethacrylat-Knochenzemente (PMMA-Knochenzemente) sind aus einer pulverförmigen Komponente und einer flüssigen Monomerkomponente zusammengesetzt (K.-D. Kühn: Knochenzemente für die Endoprothetik: Ein aktueller Vergleich der physikalischen und chemischen Eigenschaften handelsüblicher PMMA-Zemente. Springer-Verlag Berlin Heidelberg New York, 2001). Diese Polymethylmethacrylat-Knochenzemente werden nach Vermischung des Zementpulvers mit der flüssigen Monomerkomponente im noch nicht ausgehärteten, pastenförmigen Zustand als Zementteig appliziert. Bei Verwendung von Mischsystemen befindet sich der Zementteig im Fall von Pulver-Flüssigkeits-Zementen in einer Kartusche. Aus dieser Kartusche wird der Zementteig durch Bewegung eines Austragskolbens heraus gedrückt. Die Austragskolben haben üblicherweise einen Durchmesser zwischen 30 mm bis 40 mm und damit an der Außenseite, an welcher der Stößel der Auspressvorrichtung beim Auspressvorgang angreift, eine Fläche von 7,0 cm² bis 12,5 cm². Die Bewegung des Austragskolbens wird durch manuell bedienbare, mechanische Auspressvorrichtungen bewirkt, die auch als Applikatoren bezeichnet werden. Diese manuellen Auspressvorrichtungen erreichen normalerweise eine Auspresskraft im Bereich von ungefähr 1,5 kN bis 3,5 kN.

Eine neuere Entwicklung stellen pastenförmige Zweikomponenten-Knochenzemente dar, wie sie beispielsweise aus der DE 10 2007 050 762 B3, der DE 10 2008 030 312 A1 und der DE 10 2007 052 116 B4 bekannt sind. Bei diesen Zweikomponenten-Knochenzementen werden zwei pastenförmige Ausgangskomponenten in zwei separaten Kartuschen mit zwei separaten Austragskolben aufbewahrt. Bei der Applikation werden beide Pasten durch Bewegung der Austragkolben aus den Kartuschen in einen statischen Mischer gepresst und nach erfolgter Vermischung durch ein Austragsrohr ausgetragen. Bei geeigneter Zusammensetzung der pastenförmigen Ausgangskomponenten wird nach der Vermischung der beiden Ausgangskomponenten sofort ein klebfreier, applikationsbereiter Zementteig erhalten. Wartezeiten bis zum Erreichen der Klebfreiheit des Zementteigs, die bei bisherigen konventionellen Polymethylmethacrylat-Knochenzementen immer zwangsläufig auftreten, entfallen dadurch. Wertvolle OP-Zeit kann dadurch gespart werden.

Bei der Applikation der bisherigen, konventionellen PMMA-Knochenzemente, die aus einer flüssigen Monomerkomponente und einer separat dazu gelagerten Zementpulverkomponente als Ausgangskomponenten bestehen, wird nach der Vermischung der beiden Ausgangskomponenten in Zementiersystemen beziehungsweise Vakuumzementiersystemen der gebildete Zementteig mit Hilfe von manuell bedienbaren Auspressvorrichtungen ausgepresst. Diese einfachen mechanischen Auspressvorrichtungen nutzen insbesondere Klemmstangen zum Auspressen, die durch einen manuell zu betätigenden Kipphebel angetrieben werden. Die manuell angetriebenen Auspressvorrichtungen sind seit Jahrzehnten weltweit erprobt und stellen bisher den Stand der Technik dar. Vorteilhaft an diesen Auspressvorrichtungen ist, dass der medizinische Anwender über die aufzubringende Handkraft ein Gefühl für den Eindringwiderstand des Knochenzementteigs in die Knochenstrukturen (Spongiosa) hat.

Bei hochviskosen pastenförmigen Ausgangskomponenten unter Verwendung von Kartuschen, bei denen die Austragskolben an den äußeren Kolbenseiten, an denen die Stößel der Auspressvorrichtungen angreifen, eine gesamte Fläche im Bereich von 7,0 cm² bis 12,5 cm² haben, sind diese Vorrichtungen nicht oder nur mit einem sehr großen Kraftaufwand manuell zu bedienen. Dieser große Kraftaufwand ist medizinischen Anwendern im OP nicht zuzumuten.

Aus dem Kleb- und Dichtstoffbereich sind auch elektrisch angetriebene Auspressvorrichtungen bekannt. Diese Vorrichtungen können sowohl mit Akkumulatoren und mit Batterien als auch mit Hilfe einer stationären Stromversorgung angetrieben werden. Diese Vorrichtungen können mit ihren zum Teil sehr großen Auspresskräften besonders zähe pastenförmige Massen auspressen. Nachteilig ist jedoch bei der Verwendung von Elektromotoren, dass diese Buntmetalle enthalten und kostenintensiv in der Beschaffung sind. Im steril zu haltenden OP-Bereich müssen derartige Vorrichtungen aufwendig sterilisiert oder sogar ausgetauscht werden. Bei einer elektrischen Verkabelung kann die Bewegung des Anwenders im OP behindert werden.

Weiterhin wurden auch pneumatische Vorrichtungen vorgeschlagen. Diese Geräte erfordern einen stationären oder mobilen Druckluftanschluss (US 2 446 501 A; DE 20 2005 010 206 U1). Dazu sind Druckluftschläuche notwendig, welche die Bewegung des Anwenders behindern können.

Alternativ dazu ist auch die Verwendung von Druckgaspatronen zur Bereitstellung von Druckgas möglich. Dazu wurden Vorrichtungen vorgeschlagen, bei denen der Druckgaszufluss durch ein Ventil und zusätzlich durch ein zweites Ventil der Strom der viskosen Masse gesteuert werden (US 2004/0074927 A1; US 6 935 541 B1). Bei diesen Vorrichtungen sind die Gaspatronen in den Vorrichtungen integriert. Bei derartigen an Druckluft angeschlossenen oder Druckgaspatronen enthaltenden Systemen ist immer eine Druckgasquelle erforderlich, ohne die die Systeme nicht mehr anwendbar sind.

In der Vertebroplastie wird die Applikation von Knochenzement mit einem Röntgenverfahren in situ verfolgt. Bei Applikationsvorrichtungen für die Vertebroplastie wird üblicherweise ein Schlauch eingesetzt, über dessen Spitze der Knochenzement appliziert werden kann, damit der Anwender außerhalb des Bereichs der Röntgenstrahlung arbeiten kann. Hierzu können am Schlauch des Weiteren ein Trokar oder eine Kanüle angeordnet sein. Derartige Systeme sind beispielsweise aus US 7 112 205 B2, US 8 038 682 B2, US 8 308 731 B2, DE 10 2005 045 227 A1, EP 1 074 231 B1, EP 1 596 736 B1, US 9 005 209 B2 und WO 2008/097855 A2 bekannt.

Alternativ können auch andere Aufbauten verwendet werden, um den Anwender von der Röntgenstrahlung fernzuhalten, wie beispielsweise in den Dokumenten US 6 676 663 B2, US 7 008 433 B2, US 8 348 494 B2, EP1 464 292 B1, EP 1 614 403 B1, US 2008/319445 A9 und WO 2008/038322 A2 beschrieben.

Ein Knochenzement-Applikator für die Vertebroplastie zum Applizieren von Knochenzement mit einem Schlauch, einem Trokar und einem Mischer ist aus der US 2008/0086143 A1 bekannt. Der Knochenzement-Applikator umfasst zwei nebeneinander angeordnete Kartuschen, in denen die Ausgangskomponenten auch gelagert werden. Der Knochenzement-Applikator wird unmittelbar vor der Anwendung zusammengesetzt. Bei derartigen Knochenzement-Applikatoren für die Vertebroplastie wird auf die Ausgangskomponenten des Knochenzements mit Hilfe einer Auspressvorrichtung, die Austragskolben in den Kartuschen vortreiben, ein Druck ausgeübt und mit Hilfe des Drucks die Ausgangskomponenten aus den Kartuschen und durch den Schlauch ausgetrieben. Dabei werden die Ausgangskomponenten meist zuvor in einem vorgeschalteten statischen Mischer gemischt. Dies führt dazu, dass sich die den Knochenzementfluss begrenzenden Teile des Knochenzement-Applikators (die Kartuschen, das Gehäuse des Mischers und der Schlauch) elastisch verformen. Wenn der Vortrieb der Austragskolben gestoppt wird, kommt es durch die elastische Kraft dieser Teile zu einer Volumenkontraktion dieser Teile, so dass auch dann noch Knochenzement durch die Applikationsöffnung des Schlauchs beziehungsweise Trokars austritt. Hierdurch kann es zu einer Kontamination des OP-Saals oder des Anwenders mit dem Knochenzement kommen oder es wird ungewollt eine zu große Menge des Knochenzements appliziert. Zudem muss, wenn der Volumenstrom des Knochenzementteigs wieder gestartet werden soll, zunächst wieder der Druck im Knochenzement aufgebaut werden, damit dieser durch die Applikationsöffnung austritt. Dadurch verzögert sich wiederum der Zeitpunkt nach dem Beginn des Vortreibens der Austragskolben, ab dem tatsächlich der Knochenzementteig appliziert werden kann, was ebenfalls unerwünscht ist. Aufgrund der großen Zähigkeit des Knochenzementteigs und der Ausgangskomponenten, insbesondere wenn pastöse Ausgangskomponenten verwendet werden, sind alle diese Effekte relativ stark ausgeprägt. Dem kann durch die Verwendung massiver und teurer metallisches Gehäuseteile entgegengewirkt werden. Diese Teile müssen nach der Anwendung gereinigt und für eine weitere Anwendung sterilisiert werden oder sie müssen aufwendig recycelt werden.

Die US 8,544,683 B2 offenbart ein Kartuschensystem, das zur Beimischen einer geringen Menge zu einer Hauptausgangskomponente geeignet ist. Bei dem Kartuschensystem ist neben einer Kartusche eine zweite kleinere Kartusche angeordnet, wobei bei einem Vortrieb eines Austragskolbens in der größeren Kartusche auch ein Austragskolben in der kleineren Kartusche über ein gemeinsames Verbindungselement angetrieben wird. Das System ist zum Mischen der zähen pastösen Ausgangskomponenten von PMMA-Knochenzement jedoch nicht geeignet.

Ein koaxiales Kartuschensystem, das ein spezielles Kolbensystem enthält, wird im Patent EP1 392 450 B1 beschrieben. Das Kartuschensystem findet Anwendung in der Baustoffchemie für die Lagerung und Vermischung von pastenförmigen Zweikomponentendichtungsmassen. Das dort offenbarte Kolbensystem hat einen zylinderförmigen Austragskolben für die zentrale Kartusche und einen ringförmigen Austragskolben für die zweite, koaxial angeordnete Kartusche. Beide Austragskolben werden hinter den Dichtungsflächen über ein Auflageelement angetrieben, das auf der Rückseite mehrere Anlageflächen für den Stößel der Auspressvorrichtung besitzt. Das Auflageelement enthält bogenförmige Schneiden. Bei axialer Einwirkung des Stößels einer Auspressvorrichtung werden beide Austragskolben vorwärts in Richtung Kartuschenkopf bewegt. Dabei werden die in den koaxialen Kartuschen enthaltenen pastenförmigen Komponenten in Richtung Kartuschenkopf gedrückt. Gleichzeitig zerteilen zwei Schneiden die Wand der inneren koaxialen Kartusche in zwei Teile. Nachteilig ist an diesem System, dass zwangsläufig gleichzeitig zwei Schneidprozesse ablaufen. Das bedeutet, dass für beide Schneidprozesse Energie aufgebracht werden muss, die dadurch nicht zum eigentlichen Vortrieb der beiden pastöse Komponenten zur Verfügung steht. Die Vermischung von pastenförmigen Ausgangskomponenten für PMMA-Knochenzemente benötigt aufgrund der im Austragsrohr angeordneten statischen Mischer und der hohen Viskosität der Ausgangskomponenten sehr viel Vortriebsenergie, der bei größeren Kartuschen nicht mehr manuell, gefahrlos und nicht mit herkömmlichen Auspressvorrichtungen aufzubringen ist. Ein Verlust an Vortriebsenergie durch zwei gleichzeitig stattfindende Schneidprozesse kann daher insbesondere bei hoch viskosen pastenförmigen Komponenten problematisch sein. Zudem sind Koaxialkartuschen nicht ohne weiteres mit der zähen pastenförmigen Haupt-Ausgangskomponente eines PMMA-Knochenzements zu befüllen. Insbesondere wenn nur geringe Mengen des PMMA-Knochenzements enthalten sein sollen, werden die freien Querschnitte der äußeren Koaxialkartusche für die Hauptausgangskomponente so klein, dass sie nicht mit herkömmlichen Verfahren zu befüllen ist.

Im Patent FR 1 468 507 wird ein Kartuschensystem offenbart, bei dem in einer Kartusche ein schlauchförmiger Lagerbehälter angeordnet ist. Dieser ist am Kartuschenende an einem Punkt mit der Kartusche verbunden. In der Kartusche ist ein Austragskolben angeordnet, der eine Öffnung besitzt, in der ein Teil des schlauchförmigen Lagerbehälters angeordnet ist, wobei die Öffnung kleiner ist als der Durchmesser des schlauchförmigen Lagerbehälters. Bei der Vorwärtsbewegung des Austragskolbens in Richtung Kartuschenkopf wird einerseits die in der Kartusche enthaltene Masse ausgepresst und andererseits wird aus dem schlauchförmigen Behälter die darin enthaltene Masse durch Ausquetschen in Richtung Kartuschenkopf bewegt. Für die Funktion des Ausquetschens ist es wichtig, dass der schlauchförmige Lagerbehälter am Ende der Kartusche fixiert ist, damit beim Ausquetschen der schlauchförmige Lagerbehälter sich nicht mit dem Austragskolben nach vorne in Richtung Kartuschenkopf mit bewegt, ohne dass die darin enthaltene Masse ausgedrückt wird. Nachteilig ist an dem vorgeschlagenen System, dass durch das einfache Ausquetschen an einer Öffnung zwangsläufig mehr oder weniger große Reste der auszupressenden Masse im schlauchförmigen Lagerbehälter verbleiben. Beim Ausquetschen wirft der Schlauch unkontrollierbar und unvorhersehbare Falten, in denen Reste des auszuquetschenden Materials zurückbleiben. Dadurch ist die Verwendung dieses Lagerungssystems bei Mehrkomponenten-Knochenzementpasten nicht oder nur bedingt möglich, weil für die reproduzierbare Aushärtung des Knochenzements exakt definierte Gehalte der mindestens zwei Initiatorkomponenten erforderlich sind, die getrennt in der ersten Ausgangskomponente und in der zweiten Ausgangskomponente angeordnet sind. Eine Variation des Mischungsverhältnisses muss daher immer soweit als möglich vermieden werden. Zudem müssten die Kartuschen mit den Resten der Ausgangskomponenten aufgrund der chemischen Zusammensetzung der Ausgangskomponenten aufwendig entsorgt werden.

Die WO 2007/000066 A1 offenbart ein Ventilelement, bei dem eine Paste, die mit einem statischen Mischer gemischt wurde, bei geschlossenem Ventil von dem Mischer in eine Kammer geleitet wird. Aus der US 2010/0262152 A1 ist ein System zum Mischen von Knochenzementpulver mit einer Monomerflüssigkeit bekannt, bei dem über ein Ventil ein Vakuum in einem Mischraum erzeugt werden kann, um die Monomerflüssigkeit in das Zementpulver zu saugen.

Die Aufgabe der Erfindung besteht also darin, die Nachteile des Stands der Technik zu überwinden. Insbesondere soll ein einfacher und kostengünstig zu fertigender Knochenzement-Applikator für die Vertebroplastie für pastenförmige Mehrkomponenten-Polymethylmethacrylat-Knochenzemente und ein Verfahren zum Applizieren eines Zementteigs mit einem Knochenzement-Applikator für die Vertebroplastie bereitgestellt werden, der einfach aufgebaut ist und kostengünstig zu fertigen ist, wobei es beim Stoppen des Zementflusses nicht zu einem Nachlaufen des Knochenzementteigs kommen soll. Ferner soll der Knochenzement-Applikator nach Unterbrechung des Flusses des Knochenzementteigs möglichst schnell wieder einsetzbar sein. Es soll eine Kontamination der Umgebung und des Anwenders mit Knochenzementteig möglichst ausgeschlossen werden.

Der Erfindung liegt insbesondere die Aufgabe zu Grunde, einen einfachen Knochenzement-Applikator für einen Zweikomponenten-PMMA-Knochenzement (beziehungsweise für einen Zweikomponenten-PMMA-Spinezement) für die Vertebroplastie zu entwickeln. Der Knochenzement-Applikator soll möglichst einfach aus Kunststoff gefertigt werden können und sich dadurch als Produkt zur einmaligen Anwendung eignen. Das Auspressen des gemischten Zementteigs soll mit einer konventionellen, manuell angetriebenen Auspressvorrichtung, wie sie bisher bei PMMA-Knochenzementen zur Zementierung von Knie- und Hüft-TEP (totale Endoprothese des Hüftgelenks) üblich ist, möglich sein. Der Knochenzement-Applikator soll so beschaffen sein, dass ein sofortiger Notstop des fließenden Knochenzementteigs möglich ist, ohne dass eine Kontamination des OPs (Operationssaals) mit dem Knochenzementteig beziehungsweise ein Weiterfließen des Knochenzementteigs erfolgt.

Des Weiteren soll der Knochenzement-Applikator als einmalig zu verwendendes ready-to-use-System in einfachster Weise mit einer minimalen Anzahl von Montageschritten innerhalb weniger Sekunden einsatzbereit machbar sein und nach Verbindung mit manuell anzutreibenden medizinischen Auspressvorrichtungen sofort nach Beginn der manueller Betätigung der Auspressvorrichtung einen homogen gemischten Zementteig erzeugen und an der Applikationsöffnung eines Schlauchs austragen, möglichst auch dann wenn der Fluss des Knochenzements kurzzeitig unterbrochen wurde. Die in den OPs bisher für die konventionellen Polymethylmethacrylat-Knochenzemente genutzten manuell zu bedienenden Auspressvorrichtungen mit jeweils einer Schubstange beziehungsweise mit jeweils einem Stößel und gegebenenfalls einem Teller sollen für den Austrag des Zweikomponenten-Polymethylmethacrylat-Knochenzements beziehungsweise des Knochenzementteigs mit dem zu entwickelnden Knochenzement-Applikator genutzt werden können. Dadurch soll die Beschaffung von speziellen Auspressvorrichtungen für den Austrag von pastenförmigen Zweikomponenten-Polymethylmethacrylat-Knochenzementen vermieden werden.

Bevorzugt soll der zu entwickelnde Knochenzement-Applikator keine zwei miteinander verbundenen und synchron vorzutreibende Schubstangen beziehungsweise Stößel erfordern, damit die gesamte Vorrichtung nicht wesentlich aufwendiger, länger und größer wird als die bisher für die konventionellen Pulver-Flüssigkeits-Polymethylmethacrylat-Knochenzemente üblichen Mischsysteme. Es soll eine einfache Lösung gefunden werden, die es erlaubt, möglichst mit nur einer Schubstange beziehungsweise nur einem Stößel und gegebenenfalls einem daran befestigten Teller zwei oder mehr pastenförmige Ausgangskomponenten synchron und manuell aus dem Knochenzement-Applikator auszutreiben. Die pastenförmigen Ausgangskomponenten des Knochenzements sollen bevorzugt getrennt innerhalb des Knochenzement-Applikators sicher gelagert werden können. Zur Anwendung sollen beide pastenförmigen Ausgangskomponenten sicher zusammengeführt werden können.

Es soll auch möglich sein, dass die beiden Ausgangskomponenten quantitativ aus den Kartuschen ausgepresst werden können, damit das Mischungsverhältnis der Initiatorkomponenten reproduziert werden kann, um damit reproduzierbare Verarbeitungseigenschaften des gemischten Zementteigs und der mechanischen Eigenschaften des ausgehärteten Knochenzements zu gewährleisten.

Der Knochenzement-Applikator soll auch ein geringes Volumen des homogen gemischten Zementteigs von ca. 50 ml beziehungsweise maximal 80 ml austragen können, ohne dass größere Restmengen (mehr als 20 ml) in dem System verbleiben und aufwendig entsorgt werden müssen. Größere Volumina des Zementteigs werden für Anwendungen in der Vertebroplastie nicht angestrebt. Die genannten geringen Mengen sind nämlich für die Anwendung in der Vertebroplastie ausreichend.

Eine erste spezielle Ausführung des Knochenzement-Applikators soll es erlauben, zwei Pasten im Volumenverhältnis größer oder gleich 95 zu 5 oder bevorzugt größer oder gleich 98 zu 2 auszupressen und bevorzugt auch zu lagern. Diese erste Ausführung des Knochenzement-Applikators ist dabei für solche pastenförmige Mehrkomponenten-Polymethylmethacrylat-Knochenzemente vorgesehen, bei denen sich eine geringvolumige pastenförmige Ausgangskomponente sehr leicht mit einer großvolumigen pastenförmige Ausgangskomponente vermischen lässt, wobei sich die geringvolumige pastenförmige Ausgangskomponente in der großvolumigen pastenförmige Ausgangskomponente innerhalb von wenigen Sekunden löst.

Als Ausgangskomponenten sollen bevorzugt pastenförmige Ausgangskomponenten verwendet werden, die unmittelbar nach dem Auspressen anwendbar sind, bei denen also keine Zeit zum Anquellen des PMMA-Knochenzements notwendig ist. Der Knochenzement-Applikator soll so beschaffen sein, dass eine Verwechselung der relevanten Montageschritte durch den Anwender konstruktiv soweit wie möglich ausgeschlossen ist und der Knochenzement-Applikator auch von weitgehend ungeschultem Personal anwendbar ist. Weiterhin soll ein Verfahren zum Vermischen der pastenförmigen Ausgangskomponenten und zum Austragen des homogen gemischten Zementteigs bereitgestellt werden.

Die Aufgaben der Erfindung werden gelöst durch einen Knochenzement-Applikator zur Applikation eines Knochenzementteigs im Bereich der Wirbelsäule, der Knochenzement-Applikator aufweisend
zumindest eine röhrenförmige Kartusche mit einem Innenraum, wobei Ausgangskomponenten des Knochenzements im Innenraum der zumindest einen Kartusche enthalten sind,
zumindest einen Austragskolben zum Austreiben der Ausgangskomponenten aus der zumindest einen Kartusche durch eine Öffnung der zumindest einen Kartusche, wobei der zumindest eine Austragskolben in Längsrichtung im Inneren der zumindest einen Kartusche beweglich ist,
einen Schlauch,
eine Applikationsöffnung, durch die der Knochenzementteig zu applizieren ist, ein von außen bedienbares Dreiwege-Ventil, das im Schlauch oder an einer der zumindest einen Kartusche zugewandten Seite des Schlauchs angeordnet ist, wobei das Dreiwege-Ventil mit der Öffnung der zumindest einen Kartusche in Fluidverbindung steht,
einen Auffangbehälter zum Aufnehmen von Knochenzementteig, der an dem Dreiwege-Ventil angeordnet ist, wobei das Dreiwege-Ventil derart aufgebaut und in dem Knochenzement-Applikator angeordnet ist, dass es in einer ersten Stellung eine Fluidverbindung zwischen der Applikationsöffnung und der Öffnung der zumindest einen Kartusche bereitstellt und einen Durchführung zum Auffangbehälter verschließt und in einer zweiten Stellung eine Fluidverbindung zwischen der Applikationsöffnung und dem Auffangbehälter bereitstellt und einen Durchgang zur Öffnung der zumindest einen Kartusche verschließt.

Der Knochenzement-Applikator ist erfindungsgemäß bevorzugt für die Vertebroplastie vorgesehen und damit für die Vertebroplastie einsetzbar und geeignet.

Die Ausgangskomponenten des Knochenzements, insbesondere des Zweikomponenten- beziehungsweise Mehrkomponenten-Polymethylmethacrylat-Knochenzements, sind bevorzugt fluid, besonders bevorzugt pastenförmig.

Das Dreiwege-Ventil ist erfindungsgemäß bevorzugt manuell bedienbar.

Es kann vorgesehen sein, dass der Schlauch die Applikationsöffnung aufweist, insbesondere einen Luer-System-Adapter oder einen Trokar mit der Applikationsöffnung aufweist, und/oder eine der Applikationsöffnung gegenüberliegenden Anschlussöffnung aufweist.

Die Applikationsöffnung ist in Fluidverbindung mit dem Schlauch.

Auf der Seite der Kartusche bedeutet, dass die Anordnung bezogen auf die Flussrichtung des Knochenzementteigs beziehungsweise der Ausgangskomponenten bei geeigneter Stellung des Dreiwege-Ventils erfolgt, das heißt, auf der Seite erfolgt, von der aus der Knochenzementteig beziehungsweise die Ausgangskomponenten einfließt.

In dem Schlauch oder an dem der Applikationsöffnung gegenüberliegendem Ende des Schlauchs ist das Dreiwege-Ventil angeordnet.

Das Dreiwege-Ventil oder das der Applikationsöffnung gegenüberliegende Ende des Schlauchs ist in Fluidverbindung mit der Öffnung der zumindest einen Kartusche.

Es kann bevorzugt vorgesehen sein, dass zwischen der zumindest einen Kartusche und dem Dreiwege-Ventil oder zwischen der zumindest einen Kartusche und dem der Applikationsöffnung gegenüberliegende Ende des Schlauchs ein Mischrohr mit einem Mischer angeordnet ist.

Der zumindest eine Austragskolben der zumindest einen Kartusche ist vorzugsweise in dem der Öffnung der zumindest einen Kartusche gegenüberliegenden Ende der zumindest einen Kartusche angeordnet.

Es kann erfindungsgemäß vorgesehen sein, dass zwischen der Öffnung der zumindest einen Kartusche und dem Schlauch oder zwischen der Öffnung der zumindest einen Kartusche und dem Dreiwege-Ventil ein Mischer zum Mischen des Knochenzements, insbesondere ein statischer Mischer, angeordnet ist, wobei vorzugsweise das Dreiwege-Ventil zwischen dem Mischer und dem Schlauch angeordnet ist, wobei das Dreiwege-Ventil in der ersten Stellung eine Fluidverbindung zwischen der Applikationsöffnung und dem Mischer bereitstellt und in der zweiten Stellung den Durchgang zum Mischer verschließt.

Hiermit wird erreicht, dass die Ausgangskomponenten aus der zumindest einen Kartusche besser durchmischt werden. Durch die erfindungsgemäße Anordnung des Dreiwege-Ventils wird ferner erreicht, dass der Druck des Knochenzementteigs im Mischer erhalten bleibt und somit unmittelbar nach einem erneuten Öffnen des Dreiwege-Ventils wieder Knochenzementteig durch den Schlauch und die Applikationsöffnung ausgetrieben werden kann, ohne dass der Druck im Mischer neu aufgebaut werden müsste. Dadurch kann also eine schnellere Bereitstellung von Knochenzementteig nach dem Öffnen des Dreiwege-Ventils erreicht werden.

Mit der vorliegenden Erfindung wird auch vorgeschlagen, dass der Knochenzement-Applikator mit Hilfe einer manuell bedienbaren Auspressvorrichtung bedienbar ist und der zumindest eine Austragskolben mit manueller Kraft in der zumindest einen Kartusche bewegbar ist, wobei der Querschnitt des Innenraums der einen Kartusche maximal 3,5 cm² beträgt, bevorzugt maximal 2,5 cm² beträgt, oder der Querschnitt aller Innenräume der Kartuschen zusammen maximal 3,5 cm² beträgt, bevorzugt maximal 2,5 cm² beträgt, und/oder die Schubfläche des zumindest einen Austragskolbens maximal 3,5 cm² beträgt, bevorzugt maximal 2,5 cm² beträgt.

Dass der zumindest eine Austragskolben mit manueller Kraft in der zumindest einen Kartusche bewegbar ist, bedeutet, dass der zumindest eine Austragskolben mit einer manuell angetriebenen Auspressvorrichtung in der zumindest einen Kartusche bewegbar ist.

Durch diese maximalen Querschnitts- beziehungsweise Antriebsflächen, die die zum Austreiben und Mischen der Ausgangskomponenten des Knochenzements notwendige Kraft begrenzen, kann erreicht werden, dass der zähe Knochenzementteig aus der zumindest einen Kartusche mit manueller Kraft durch den Schlauch und gegebenenfalls den Mischer ausgetrieben werden kann. Dadurch kann ein manuell angetriebener beziehungsweise antreibbarer Knochenzement-Applikator bereitgestellt werden, der ohne einen Anschluss für eine externe Energiequelle und auch ohne eine interne Energiequelle auskommt und somit immer und unabhängig von den äußeren Gegebenheiten Einsatzbereit ist, beziehungsweise es kann eine Auspressvorrichtung verwendet werden, die ohne einen Anschluss für eine externe Energiequelle und auch ohne eine interne Energiequelle auskommt und somit immer und unabhängig von den äußeren Gegebenheiten Einsatzbereit ist.

Des Weiteren kann vorgesehen sein, dass der Schlauch zumindest bereichsweise flexibel ist und/oder die Applikationsöffnung in einem Anschluss mit einem Innengewinde, insbesondere in einem Luer-System-Adapter, oder in einem Trokar angeordnet ist.

Hiermit wird erreicht, dass der Knochenzement-Applikator auch in schwer zugänglichen Bereichen einsetzbar ist. Ferner wird über den Schlauch und gegebenenfalls den Trokar erreicht, dass der Eintrag des Knochenzements mit einem Röntgenverfahren überwacht werden kann, ohne dass der Bediener des Knochenzement-Applikators der Röntgenstrahlung unmittelbar ausgesetzt ist. Der Trokar kann über ein Innengewinde, insbesondere über ein Luer-System, mit dem restlichen Schlauch verbunden sein. Mit Hilfe eines Luer-System-Adapters wird eine universelle Anschließbarkeit des Knochenzement-Applikators beziehungsweise von dessen Bauteilen erreicht.

Bevorzugt kann auch vorgesehen sein, dass der Auffangbehälter nach außen für den Knochenzementteig undurchlässig ist, vorzugsweise der Auffangbehälter flüssigkeitsdicht oder flüssigkeitsdicht und gasdicht ist, und/oder der Auffangbehälter ein Volumen aufweist, das mindestens so groß ist wie die Hälfte des Volumens des Schlauchs, bevorzugt mindestens so groß ist wie das Volumen des Schlauchs.

Hiermit kann vermieden werden, dass der Knochenzementteig und dessen Bestandteile nach außen gedrückt werden und dadurch den OP-Saal oder den Anwender verschmutzen beziehungsweise kontaminieren.

Ferner kann vorgesehen sein, dass die zumindest eine Kartusche an der Rückseite ein Befestigungselement zur Befestigung einer Auspressvorrichtung aufweist.

Hiermit wird eine stabile Befestigung der Auspressvorrichtung mit dem Knochenzement-Applikator ermöglicht.

Gemäß einer bevorzugten ersten Ausführung der vorliegenden Erfindung wird ein Knochenzement-Applikator vorgeschlagen, aufweisend
eine erste röhrenförmige Kartusche mit einem ersten zylindrischen Innenraum, wobei in dem Innenraum eine erste Ausgangskomponente eines Knochenzements enthalten
ist,
einen ersten Austragskolben der axial beweglich im ersten Innenraum der ersten Kartusche angeordnet ist und der zum Austreiben der ersten Ausgangskomponente aus der ersten Kartusche durch eine dem ersten Austragskolben gegenüberliegende Öffnung in einem Kartuschenkopf der ersten Kartusche vorgesehen ist,
eine zweite röhrenförmige Kartusche, die innerhalb der ersten röhrenförmigen Kartusche angeordnet ist, wobei in der zweiten Kartusche eine zweite Ausgangskomponente des Knochenzements enthalten ist und ein zweiter Austragskolben angeordnet ist, wobei mit dem zweiten Austragskolben die zweite Ausgangskomponente aus der zweiten Kartusche durch eine gegenüberliegende Öffnung der zweiten Kartusche im Bereich des Kartuschenkopfs der ersten Kartusche auszutreiben ist,
wobei vom Kartuschenkopf aus gesehen hinter dem ersten Austragskolben und dem zweiten Austragskolben eine axial im Innenraum der ersten Kartusche vortreibbare Pressvorrichtung mit einer Klemmkante zum Zusammenpressen der zweiten Kartusche angeordnet ist, wobei die Pressvorrichtung derart in Richtung des Kartuschenkopfs vortreibbar ist, dass während der Bewegung der Pressvorrichtung die zweite Kartusche axial fortlaufend zusammengepresst wird und dabei der erste Austragskolben und der zweite Austragskolben in Richtung des Kartuschenkopfs vorgetrieben werden.

Hiermit wird erreicht, dass durch die Anordnung zumindest einer inneren zweiten Kartusche in einer größeren äußeren ersten Kartusche, wobei die innere zweite Kartusche mit einer Pressvorrichtung, die innerhalb der äußeren ersten Kartusche läuft und dabei sowohl die Austragskolben in den Kartuschen vorschiebt als auch die Wand der inneren zweiten Kartusche zusammendrückt und damit aus dem Weg des Stößels der Auspressvorrichtung drückt, gelingt, einen Knochenzement-Applikator für einen Zweikomponenten-Knochenzement beziehungsweise Mehrkomponenten-Knochenzement bereitzustellen, mit dem auch eine geringfügige Menge zumindest einer zweiten Ausgangskomponente mit einer ersten Hauptausgangskomponente homogen und reproduzierbar mit dem gewünschten Mischungsverhältnis gemischt und anschließend appliziert werden kann. Der Knochenzement-Applikator ist dadurch auch zur Lagerung der Ausgangskomponenten geeignet und kann zudem kostengünstig gefertigt werden. Trotz der kostengünstigen Ausführung ist der Knochenzement-Applikator einfach einzusetzen und kann auch mit manuell angetriebenen Auspressvorrichtungen verwendet werden. Zugleich ist die Gefahr für eine Blockade des Knochenzement-Applikators gering, so dass ein besonders betriebssicherer Knochenzement-Applikator bereitgestellt wird. Ferner wurde überraschend gefunden, dass auf diese Weise eine schmale Kartusche mit nur einer Pressvorrichtung als Antrieb zum Vortreiben der Austragskolben zum Vortreiben der beiden Ausgangskomponenten verwendet werden kann, mit der auch gleichzeitig die Wand oder die Wände der zumindest einen inneren (zweiten) Kartusche seitlich weggedrückt werden kann. Dadurch kann die Kraft, die notwendig ist, um die Ausgangskomponenten zu mischen und auszutreiben, minimiert werden, so dass eine mit manueller Kraft anzutreibende Auspressvorrichtung zusammen mit dem Knochenzement-Applikator eingesetzt werden kann, um die Ausgangskomponenten aus den Kartuschen auszutreiben und miteinander zu mischen. Diese erste Ausführung ist also zur Beimischung geringer Mengen einer zweiten Ausgangskomponente des Knochenzements geeignet.

Die zumindest eine Kartusche wird bei dieser erfindungsgemäßen ersten Ausführung also durch zwei Kartuschen, nämlich die erste und die zweite Kartusche realisiert. Der zumindest eine Austragskolben wird dementsprechend durch den ersten und den zweiten Austragskolben realisiert.

Der Innenraum der ersten (äußeren) Kartusche hat vorzugsweise eine zylindrische Geometrie. Das gilt auch für den Innenraum der zweiten (inneren) Kartusche vor der Verformung der Wand der zweiten Kartusche mit der Pressvorrichtung. Die zylindrische Form ist die einfachste, mit der sich die Innenräume der Kartuschen realisieren lassen. Unter einer zylindrischen Form ist geometrisch die Form eines allgemeinen Zylinders mit einer beliebigen Grundfläche zu verstehen, also nicht nur ein Zylinder mit einer kreisförmigen Grundfläche. Die begrenzende Innenwand des Innenraums kann also ein Zylinder mit beliebiger Grundfläche sein und der Mantel kann ein Zylinder mit beliebiger Grundfläche sein, das heißt auch mit nicht kreisförmiger oder runder Grundfläche. Erfindungsgemäß wird jedoch eine zylindrische Geometrie mit rotationssymmetrischer und insbesondere kreisrunder Grundfläche für den Innenraum der ersten Kartusche bevorzugt, da diese am einfachsten zu fertigen sind. Die Wand der zweiten Kartusche kann derart an der Innenwand der ersten Kartusche befestigt sein, dass eine zylindrische Symmetrie des zweiten Innenraums von der kreisförmigen Grundfläche abweicht. Die Pressvorrichtung und der erste Austragskolben können auch einteilig oder fest miteinander verbunden ausgeführt sein.

Der erste Austragskolben schließt bevorzugt dicht mit der Innenwand des ersten zylindrischen Innenraums ab und der zweite Austragskolben schließt dicht mit der Innenwand des zweiten zylindrischen Innenraums ab. Der zweite zylindrische Innenraum ist dabei nicht verformt oder nur am rückseitigen Ende verformt. Besonders bevorzugt schließt der erste Austragskolben dicht mit der Außenwand der zweiten Kartusche in dem Bereich ab, in denen die Außenwand der zweiten Kartusche den Innenraum der ersten Kartusche begrenzt.

Bei erfindungsgemäßen Knochenzement-Applikatoren nach der ersten Ausführung kann vorgesehen sein, dass die zweite Kartusche mit ihrer Außenwand an der Innenwand der ersten Kartusche anliegt und an der Innenwand der ersten Kartusche befestigt ist.

Dabei kann bevorzugt vorgesehen sein, dass die zweite Kartusche mit ihrer Außenwand mit der Innenwand der ersten Kartusche vorne im Bereich des Kartuschenkopfs und hinten, hinter dem zweiten Austragskolben befestigt ist, wobei vorzugsweise die zweite Kartusche mit ihrer Außenwand mit der Innenwand der ersten Kartusche entlang der gesamten Länge der zweiten Kartusche befestigt ist.

Hierdurch wird verhindert, dass sich die zweite Kartusche unkontrolliert in der ersten Kartusche bewegt und dadurch eine Undichtigkeit der sich verformenden zweiten Kartusche entsteht. Eine Verbindung beziehungsweise Befestigung der zweiten Kartusche entlang der gesamten Länge der zweiten Kartusche ist dazu besonders gut geeignet. Zudem kann dies auch einfach gefertigt werden.

Des Weiteren kann vorgesehen sein, dass beim Vortreiben der Pressvorrichtung der erste Austragskolben und der zweite Austragskolben parallel zueinander vorgetrieben werden, vorzugsweise der erste Austragskolben und der zweite Austragskolben auf gleicher Höhe in Richtung des Kartuschenkopfs laufen.

Hiermit wird eine gleichmäßige Mischung der Ausgangskomponenten erreicht, bei der das Mischungsverhältnis gleichbleibend ist und damit die Eigenschaften des zu mischenden Knochenzements.

Es kann ferner vorgesehen sein, dass die Klemmkante gegen die Längsachse der ersten Kartusche geneigt ist, vorzugsweise mit einem Winkel von wenigstens 40° senkrecht zur Längsachse in Richtung der Innenwand der ersten Kartusche geneigt ist, besonders bevorzugt mit einem Winkel zwischen 40° und 80° senkrecht zur Längsachse in Richtung der Innenwand der ersten Kartusche geneigt ist.

Hierdurch wird die Wand der zweiten Kartusche über eine größere geneigte Fläche verformt, wodurch der Vortrieb vereinfacht wird und die Verformung gleichmäßiger erfolgen kann. Gleichzeitig ist die Verformung aber ausreichend, um den zweiten Austragskolben rückseitig durch die sich verformende Wand der zweiten Kartusche gleichmäßig anzutreiben.

Mit der ersten Ausführung der Erfindung wird auch vorgeschlagen, dass die Klemmkante beim Vortreiben der Pressvorrichtung die zweite Kartusche beziehungsweise die Wand der zweiten Kartusche gegen die Innenwand der ersten Kartusche quetscht.

Damit wird die Wand der Kartusche so weit als möglich aus dem Wirkungsbereich eines nachfolgenden Stößels einer Auspressvorrichtung gedrückt, so dass diese nicht dessen Bewegung behindern kann.

Des Weiteren kann vorgesehen sein, dass die Klemmkante mindestens 30 Prozent der Fläche des Querschnitts der zweiten Kartusche überdeckt, bevorzugt mindestens 60% der Fläche des Querschnitts der zweiten Kartusche überdeckt.

Hiermit wird eine ausreichende Umformung erreicht, damit der zweite Austragskolben durch die sich verformende Wand der zweiten Kartusche angetrieben werden kann. Ferner wird so auch die Wand der zweiten Kartusche ausreichend weit aus dem Wirkungsbereich eines nachfolgenden Stößels einer Auspressvorrichtung gedrückt, so dass diese nicht dessen Bewegung behindern kann.

Des Weiteren kann vorgesehen sein, dass zwischen der Pressvorrichtung und der Innenwand der ersten Kartusche im Bereich der zweiten Kartusche ein Spalt vorgesehen ist, der genauso breit oder breiter ist als die Dicke der Wand der zweiten Kartusche.

Hierdurch kann die Pressvorrichtung über die verformte zweite Kartusche laufen, ohne dass das Material der Wand der zweiten Kartusche in sich komprimiert werden müsste und ohne dass eine Verformung der ersten Kartusche notwendig wäre, was zu einem unerwünschten zusätzlichen Kraftaufwand beim Vortreiben der Pressvorrichtung führen würde.

Es wird ferner für die erste Ausführung vorgeschlagen, dass die Rückseite der Pressvorrichtung als Auflagefläche für einen Stößel einer Auspressvorrichtung ausgebildet ist.

Hiermit kann die Pressvorrichtung leicht mit einer herkömmlichen Auspressvorrichtung angetrieben werden.

Gemäß einer bevorzugten Weiterbildung der ersten Ausführung kann vorgesehen sein, dass der Durchmesser des Innenraums der ersten Kartusche kleiner oder gleich 35 mm ist, wobei bevorzugt der Durchmesser des Innenraums der ersten Kartusche kleiner oder gleich 20 mm ist, und/oder die erste Kartusche einen Innendurchmesser von höchstens 35 mm und die zweite Kartusche einen Innendurchmesser von höchstens 10 mm haben, bevorzugt die erste Kartusche einen Innendurchmesser von höchstens 20 mm und die zweite Kartusche einen Innendurchmesser von höchstens 5 mm haben.

Durch den erfindungsgemäßen Aufbau der ersten Kartuschen beziehungsweise der ersten und der zweiten Kartusche gelingt es, die oft besonders zähen und pastösen Ausgangskomponenten des Knochenzements, insbesondere bezüglich der ersten Ausgangskomponente, in einem einzigen Knochenzement-Applikator als Lagerungs- und Mischsystem unterzubringen, der noch durch manuelle Krafteinwirkung auspressbar ist und der aber auch gleichzeitig noch mit herkömmlichen Techniken befüllt werden kann. Bei größeren Durchmessern reicht eine manuelle Krafteinwirkung nicht mehr ohne weiteres aus, um die zähflüssigen pastösen Ausgangskomponenten des Knochenzements aus der Kartusche beziehungsweise den Kartuschen zu pressen. Bei den angegebenen Durchmessern wirken sich die Vorteile der vorliegenden Erfindung also besonders stark aus.

Esa kann des Weiteren vorgesehen sein, dass beim Vortreiben der Pressvorrichtung in Richtung Kartuschenkopf die Klemmkante durch Quetschung der zweiten Kartusche die so deformierte Wand der zweiten Kartusche gegen die Unterseite des zweiten Austragskolbens gepresst wird und so den zweiten Austragskolben in Richtung Kartuschenkopf schiebt.

Hiermit kann ein separater oder komplexer Antrieb für den zweiten Austragskolben vermieden werden. Ohne den zweiten Austragskolben käme es aber bei einem reinen Ausquetschen der zweiten Ausgangskomponente aus der sich verformenden zweiten Kartusche zu ungewollten Variationen in der Zusammensetzung des Knochenzements, da sich beim Verformen der zweiten Kartusche unkontrolliert Falten bilden, in denen unvorherbestimmbare Reste der zweiten Ausgangskomponente verbleiben, die nicht mit der ersten Ausgangskomponente gemischt werden. Dagegen wird beim Vortreiben des zweiten Austragskolbens, der mit Dichtungen und/oder Abstreiflippen gegen die Innenwand der zweiten Kartusche abgedichtet werden kann, immer der gesamte Inhalt der zweiten Kartusche gefördert und ausgetrieben.

Bevorzugte Ausführungsformen der vorliegenden Erfindung können sich dadurch auszeichnen, dass die zumindest eine Kartusche, der zumindest eine Austragskolben und der Schlauch und gegebenenfalls der Mischer aus Kunststoff gefertigt sind, wobei als Kunststoffe Polyethylen-co-vinylalkohol (EVOH), Polybutylenterephthalat (PBT), Polyethylenterephthalat (PET) und Poly-methacrylsäuremethylester-co-acrylnitril bevorzugt werden.

Der Aufbau mit Kunststoffen ist kostengünstig und einfach umsetzbar. Die bevorzugten Kunststoffe sind aufgrund ihrer Resistenz gegenüber den in den Ausgangskomponenten enthaltenen Chemikalien besonders gut geeignet.

Es wird mit der ersten Ausführung der vorliegenden Erfindung vorgeschlagen, dass die Pressvorrichtung oder die Klemmkante aus Metall und/oder Kunststoff und/oder glasfaserverstärktem Kunststoff gefertigt ist, wobei die Pressvorrichtung oder die Klemmkante aus Stahl, Aluminiumlegierungen, Zinklegierungen, Polyamid, glasfaserverstärktem Polyamid, Polyetherketon, Polysulfon oder Kombinationen dieser Werkstoffe gefertigt ist.

Durch die Härte dieser Materialien und einen hohen Widerstand gegen elastische Verformungen (was insbesondere auch durch eine geeignete Formgebung erreicht werden kann) kann eine ausreichende Umformung der zweiten Kartusche erreicht werden, ohne dass sich die Klemmkante selbst zu stark verformt.

Bevorzugte Knochenzement-Applikatoren der ersten Ausführung können sich auch dadurch auszeichnen, dass das Verhältnis des Volumens der ersten Kartusche zum Volumen der zweiten Kartusche mindestens 95 zu 5 ist, bevorzugt mindestens 98 zu 2 ist.

Hierdurch werden kleine beziehungsweise geringe Mengen einer zweiten Ausgangskomponente beigemischt und so die Vorteile des erfindungsgemäßen Aufbaus besonders gut genutzt. Der erfindungsgemäße Knochenzement-Applikator zeichnet sich nämlich dadurch aus, dass auch solche stark unterschiedlichen Mischungsverhältnisse noch homogen erzeugt werden können.

Des Weiteren kann vorgesehen sein, dass die zweite Kartusche bei einer Beaufschlagung der Pressvorrichtung von mindestens 0,5 kN in Richtung des Kartuschenkopfs so gequetscht wird, dass die gequetschte zweite Kartusche durch einen Spalt zwischen der Klemmkante und der Innenwand der ersten Kartusche passt.

Bevorzugt wird dies durch die Wahl einer geeigneten Dicke der Wand der zweiten Kartusche erreicht sowie durch die Auswahl eines geeigneten Materials für die zweite Kartusche. Hiermit wird erreicht, dass der Knochenzement-Applikator mit manuellem Kraftaufwand auspressbar beziehungsweise verwendbar ist.

Ferner kann vorgesehen sein, dass das Verhältnis der Dicke der Wand der ersten Kartusche zu der Dicke der Wand der zweiten Kartusche mindestens 11 zu 10 ist, und wobei bevorzugt das Verhältnis der Dicke der Wand der ersten Kartusche zu der Dicke der Wand der zweiten Kartusche mindestens 2 zu 1 ist, besonders bevorzugt mindestens 3 zu 1 ist.

Dies gilt für den Fall, dass die erste und die zweite Kartusche aus dem gleichen Material bestehen. Sofern die innere zweite Kartusche aus einem Material mit einem geringeren Elastizitäts-Modul beziehungsweise einem leichter umformbaren Material besteht, kann die Wandstärke der zweiten inneren Kartusche auch gleich groß oder sogar größer gewählt werden, als die Wandstärke der äußeren Kartusche. Hiermit wird erreicht, dass sich die innere zweite Kartusche verformen lässt, ohne, dass sich die äußere erste Kartusche mit verformt und dadurch die Bewegung der Pressvorrichtung und der Austragskolben behindert wird.

Die innere zweite Kartusche kann als fertiges Teil in die erste Kartusche eingeschweißt werden. Dies ist insbesondere dann wichtig, wenn die beiden Kartuschen nicht aus dem gleichem Material bestehen.

Mit einer Weiterbildung der ersten Ausführung der vorliegenden Erfindung wird vorgeschlagen, dass ein Teil der Wand der ersten Kartusche einen Teil der Innenwand der zweiten Kartusche bildet, bevorzugt über die gesamte Länge der zweiten Kartusche oder über zumindest 80% der gesamten Länge der zweiten Kartusche, und/oder dass ein Teil der Wand der ersten Kartusche einen Teil des zweiten Innenraums des zweiten Kartusche begrenzt, bevorzugt über die gesamte Länge der zweiten Kartusche oder über zumindest 80% der gesamten Länge der zweiten Kartusche.

Hiermit ist eine Bewegung der zweiten Kartusche gegen die erste Kartusche besonders effektiv eingeschränkt. Zudem kann so die Verformung der Wand der zweiten Kartusche besonders gut vorgegeben werden.

Es kann vorgesehen sein, dass die Dicke der Wand der ersten Kartusche mindestens 1 mm ist und die Wand der zweiten Kartusche höchstens 0,5 mm ist.

Hiermit wird erreicht, dass sich nur die zweite Kartusche plastisch verformt, während die erste Kartusche plastisch und weitgehend auch elastisch unverformt bleibt.

Es kann erfindungsgemäß bei der ersten Ausführung vorgesehen sein, dass die zweite Kartusche von der Klemmkante plastisch verformt wird, beziehungsweise plastisch verformbar ist.

Es kann erfindungsgemäß ferner vorgesehen sein, dass die erste Kartusche an der dem Kartuschenkopf gegenüberliegenden Seite außen an der Wand ein Befestigungsmittel aufweist, mit der der Knochenzement-Applikator mit einer Auspressvorrichtung verbindbar ist.

Gemäß einer Weiterbildung der ersten Ausführung kann erfindungsgemäß vorgesehen sein, dass eine dritte röhrenförmige Kartusche innerhalb der ersten röhrenförmigen Kartusche angeordnet ist, wobei die dritte Kartusche mit ihrer Außenwand an der Innenwand der ersten Kartusche anliegt und an der Innenwand der ersten Kartusche befestigt ist, wobei in der dritten Kartusche die zweite Ausgangskomponente oder eine dritte Ausgangskomponente des Mehrkomponenten-Knochenzements enthalten ist und ein dritter Austragskolben angeordnet ist, wobei mit dem dritten Austragskolben die zweite Ausgangskomponente oder die dritte Ausgangskomponente aus der dritten Kartusche durch eine gegenüberliegende Öffnung in der dritten Kartusche im Bereich des Kartuschenkopfs der ersten Kartusche auszutreiben ist, wobei die Pressvorrichtung vom Kartuschenkopf aus gesehen hinter dem dritten Austragskolben angeordnet ist und die Pressvorrichtung eine Klemmkante zum Zusammenpressen der dritten Kartusche aufweist, wobei die Pressvorrichtung derart in Richtung des Kartuschenkopfs vortreibbar ist, dass während der Bewegung der Pressvorrichtung die dritte Kartusche axial fortlaufend zusammengepresst wird und dabei der erste Austragskolben, der zweite Austragskolben und der dritte Austragskolben in Richtung des Kartuschenkopfs vorgetrieben werden.

Hiermit kann eine weitere Ausgangskomponente in den Knochenzementteig gemischt werden. Wenn die dritte Kartusche mit der zweiten Ausgangskomponente befüllt ist, hat dies den Vorteil, dass hierdurch die zweite Ausgangskomponente an unterschiedlichen Stellen der ersten Ausgangskomponente beigemischt werden kann und so eine homogenere Mischung der Ausgangskomponenten erreicht werden kann.

Bevorzugt ist die dritte röhrenförmige Kartusche an der zur zweiten Kartusche gegenüberliegenden Innenwand der ersten Kartusche angeordnet. Durch diese Symmetrie wird erreicht, dass auch die Krafteinwirkung durch die Verformung der zweiten und dritten Kartusche symmetrisch auf die Pressvorrichtung einwirkt. Dadurch kann ein gleichmäßigerer Vortrieb erreicht werden. Insbesondere ist aber die Gefahr reduziert, dass die Pressvorrichtung in der ersten Kartusche verkantet, wodurch zumindest die notwendige Kraft zum Antreiben des Knochenzement-Applikators reduziert wird, ein gleichmäßigerer Austrag des gemischten Knochenzementteigs erreicht wird und die Gefahr einer vollständigen Blockade des Knochenzement-Applikators reduziert oder verhindert wird.

Dabei kann vorgesehen sein, dass die dritte Kartusche und/oder der dritte Austragskolben die gleichen Merkmale wie die zweite Kartusche und/oder der zweite Austragskolben nach einem der erfindungsgemäßen Knochenzement-Applikatoren mit innerer zweiter Kartusche aufweist.

Dies gilt insbesondere auch in Bezug auf die Wechselwirkung der dritten Kartusche und/oder des dritten Austragskolbens zu anderen Bauteilen erfindungsgemäßer Knochenzement-Applikatoren. Hieraus ergeben sich die gleichen Vorteile, wie für die zweite Kartusche und/oder den zweiten Austragskolben.

Ferner kann vorgesehen sein, dass zumindest eine vierte röhrenförmige Kartusche innerhalb der ersten röhrenförmigen Kartusche angeordnet ist, wobei die zumindest eine vierte Kartusche mit ihrer Außenwand an der Innenwand der ersten Kartusche anliegt und an der Innenwand der ersten Kartusche befestigt ist, wobei in der zumindest einen vierten Kartusche die zweite, die dritte, eine vierte und/oder jeweils zumindest eine weitere Ausgangskomponente des Knochenzements enthalten ist und in der zumindest einen vierten Kartusche jeweils ein vierter Austragskolben angeordnet ist, wobei mit dem vierten Austragskolben die zweite, die dritte, die vierte und/oder die jeweilige weitere Ausgangskomponente aus der zumindest einen vierten Kartusche durch eine gegenüberliegende Öffnung in der zumindest einen vierten Kartusche im Bereich des Kartuschenkopfs der ersten Kartusche auszutreiben ist, wobei die Pressvorrichtung vom Kartuschenkopf aus gesehen hinter dem oder den vierten Austragskolben angeordnet ist und die Pressvorrichtung zumindest eine Klemmkante zum Zusammenpressen der zumindest einen vierten Kartusche aufweist, wobei die Pressvorrichtung derart in Richtung des Kartuschenkopfs vortreibbar ist, dass während der Bewegung der Pressvorrichtung die zumindest eine vierte Kartusche axial fortlaufend zusammengepresst wird und dabei der erste Austragskolben, der zweite Austragskolben, der dritte Austragskolben und der vierte oder die vierten Austragskolben in Richtung des Kartuschenkopfs vorgetrieben werden.

Auch hierbei kann bevorzugt vorgesehen sein, dass die zumindest eine vierte Kartusche und/oder der oder die jeweiligen vierten Austragskolben die gleichen Merkmale wie die zweite Kartusche und/oder der zweite Austragskolben nach einem der erfindungsgemäßen Knochenzement-Applikatoren mit innerer zweiter Kartusche aufweist.

Dies gilt insbesondere auch in Bezug auf die Wechselwirkung der zumindest einen vierten Kartusche und/oder des vierten Austragskolbens oder der jeweiligen vierten Austragskolben zu anderen Bauteilen erfindungsgemäßer Knochenzement-Applikatoren. Auch kann erfindungsgemäß vorgesehen sein, dass die zumindest eine vierte Kartusche zusammen mit der zweiten und dritten Kartusche symmetrisch bezüglich der Achse der ersten Kartusche angeordnet sind, um eine gleichmäßige Durchmischung aller Ausgangskomponenten und eine gleichmäßige Krafteinwirkung auf die Kartuschen und die Pressvorrichtung zu erreichen.

Gemäß einer bevorzugten zweiten Ausführung der vorliegenden Erfindung, die eine Alternative zur ersten Ausführung ist, wird ein Knochenzement-Applikator vorgeschlagen, aufweisend
eine röhrenförmige Kartusche, wobei der Innenraum der Kartusche zylindrisch ist, einen Kartuschenkopf, der ein Ende der röhrenförmigen Kartusche begrenzt, eine axial in dem zylindrischen Innenraum der Kartusche angeordnete Trennwand, wobei die Trennwand mit der Mantelfläche des zylindrischen Innenraums der Kartusche verbunden ist und wobei die Trennwand den durch den Kartuschenkopf begrenzten zylindrischen Innenraum der Kartusche in zwei räumlich voneinander getrennte Hohlräume teilt, wobei in dem ersten Hohlraum eine erste pastenförmige Ausgangskomponente des Knochenzements enthalten ist und in dem separaten zweiten Hohlraum eine zweite pastenförmige Ausgangskomponente des Knochenzements enthalten ist,
zwei axial in beiden Hohlräumen der Kartusche verschiebbar angeordnete Austragskolben, wobei die Austragskolben die beiden Hohlräume auf der dem Kartuschenkopf gegenüberliegenden Seite der Hohlräume abschließen,
wobei die Austragskolben an der dem Kartuschenkopf gegenüberliegenden Rückseite über ein Verbindungsmittel miteinander verbunden sind, wobei an dem Verbindungsmittel ein Keil oder Kegel mit einer Schneide an der zum Kartuschenkopf weisenden Vorderseite des Keils oder Kegels angeordnet ist, so dass beim Vortreiben der Austragskolben in den Hohlräumen in Richtung des Kartuschenkopfs die Schneide die Trennwand aufschneidet und der Keil oder Kegel die aufgeschnittene Teile der Trennwand in Richtung der Innenwand der Kartusche drückt.

So gelingt es, dass die Ausgangskomponenten in einer einzigen gemeinsamen Kartusche mit zylindrischem Innenraum gelagert werden können, wenn die Ausgangskomponenten in der Kartusche über eine Trennwand, die im Innenraum der Kartusche angeordnet ist, voneinander getrennt werden, wobei beide Austragskolben über ein gemeinsames Verbindungsmittel ausgetrieben werden können, das die Trennwand aufschneidet und die aufgeschnittenen Teile der Trennwand aufbiegt, so dass eine weitere Bewegung eines antreibenden Stößels einer Auspressvorrichtung nicht behindert wird. Dadurch können handgetriebene Auspressvorrichtungen mit nur einem Stößel verwendet werden, da die notwendige Kraft aufgrund des erfindungsgemäßen Aufbaus dazu ausreicht, die Ausgangskomponenten anzutreiben und zu mischen sowie die Trennwand aufzuschneiden und zu verformen, beziehungsweise die notwendige Kraft vollständig in diese vier Vorgänge gesteckt werden kann. Daher ist der Kraftaufwand zum Vortreiben der Ausgangskomponenten, zum Mischen der Ausgangskomponenten und zum Aufschneiden und Trennen der Trennwand aufgrund des erfindungsgemäßen Aufbaus in der Summe nicht so groß, dass die Auspressvorrichtung insgesamt zu schwergängig wäre. Bei der zweiten Ausführung werden bevorzugt gleich große Mengen oder ähnlich große Mengen von Ausgangskomponenten des Knochenzements miteinander gemischt.

Die zumindest eine Kartusche ist bei dieser zweiten Ausführung also eine einzelne röhrenförmige Kartusche mit einem zylindrischen Innenraum. Der zumindest eine Austragskolben ist bei dieser zweiten Ausführung durch die beiden Austragskolben realisiert, die zum Austreiben der beiden Ausgangskomponenten aus den beiden Hohlräumen in der Kartusche vorgesehen sind.

Die beiden Teile der mit der Schneide aufgeschnittenen Trennwand bleiben also mit der Kartusche verbunden. Es kann erfindungsgemäß aber auch als dritte erfindungsgemäße Ausführung vorgesehen sein, dass an dem Verbindungsmittel zwei Schneiden angeordnet sind, die einen Streifen aus der Trennwand herausscheiden. Dieser Streifen wird dann vorzugsweise in Richtung der Kartuscheninnenwand gedrückt und so von dem Hub beziehungsweise von der Bewegung des Stößels zum Vortreiben der Austragskolben beziehungsweise des Verbindungsmittels weggedrückt.

Besonders bevorzugt verformt der Keil oder Kegel bei der zweiten Ausführung die beiden aufgeschnittenen Teile der Trennwand. Die Verformung bewirkt eine Verschiebung des Materials der Teile der aufgeschnittenen Trennwand in Richtung der Innenwand der Kartusche. Ganz besonders bevorzugt werden die Teile der aufgeschnittenen Teile der Kartusche in Richtung der Innenwand der Kartusche geklappt.

Der Keil oder Kegel weist erfindungsgemäß bevorzugt an der der Schneide gegenüberliegenden Basis einen Durchmesser von wenigstens 1,5 mm auf, besonders bevorzugt von wenigstens 3 mm. Hierdurch wird sichergestellt, dass einem Stößel einer Auspressvorrichtung eine ausreichende Anlagefläche zum vortreiben des Verbindungsmittels und damit der Austragskolben geboten wird.

Die beiden Hohlräume sind bevorzugt ebenfalls zylindrisch und haben besonders bevorzugt eine Grundfläche in Form eines Halbkreises oder in Form von Kreissegmenten.

Die Ausführung der zweiten Ausführung des Knochenzement-Applikators mit auftrennbarer Trennwand ist vorzugsweise für eine 1:1 Mischung der Ausgangskomponenten vorgesehen.

Es kann vorgesehen sein, dass die Trennwand zumindest halb so breit ist wie die Hälfte des maximalen Durchmessers senkrecht zur Achse der Kartusche. Es kann auch vorgesehen sein, dass die Trennwand eine ebene Fläche ist. Die Trennwand soll den zylindrischen Innenraum der Kartusche im Bereich der Achse des Innenraums der Kartusche in die zwei Hohlräume teilen, besonders bevorzugt in zwei gleich große Hohlräume teilen.

Mit einer alternativen Weiterbildung der zweiten Ausführung der vorliegenden Erfindung wird vorgeschlagen, dass die Austragskolben aus mehreren nicht miteinander verbundenen Teilen bestehen, vorzugsweise einem vorderen Teil und einem hinteren Teil, die miteinander verbindbar sind oder die aneinander anlegbar sind. Der vordere Teil wird dann einfach von dem hinteren Teil in den Hohlräumen vorangeschoben. Das Verbindungsmittel ist dabei mit den hinteren oder hintersten Teilen der Austragskolben verbunden oder verbindbar. Zur Stabilisierung der beiden Austragskolben und zur Stabilisierung der Bewegung der beiden Austragskolben in den Hohlräumen sind die beiden Austragskolben bevorzugt jeweils einteilig ausgeführt und fest mit dem Verbindungsmittel verbunden.

Bevorzugt kann vorgesehen sein, dass die Trennwand den Innenraum der Kartusche flüssigkeitsundurchlässig trennt und dadurch die beiden Hohlräume voneinander flüssigkeitsundurchlässig getrennt sind.

Hiermit wird sichergestellt, dass die beiden Ausgangskomponenten auch über längere Zeit in dem Knochenzement-Applikator beziehungsweise innerhalb der Kartusche gelagert werden können. Es soll dabei vermieden werden, dass die flüssige und sehr bewegliche Monomerkomponente in den benachbarten Hohlraum kriecht und mit der anderen Ausgangskomponente reagiert.

Gemäß einer bevorzugten Weiterbildung der zweiten Ausführung der vorliegenden Erfindung kann vorgesehen sein, dass die Trennwand mit der Mantelfläche des zylindrischen Innenraums der Kartusche entlang zwei Verbindunglinien verbunden ist, die die Mantelfläche begrenzen, wobei bevorzugt die Verbindungslinien gegenüberliegend zueinander angeordnet sind und besonders bevorzugt die Achse der Kartusche in der Trennwand liegt.

Hierdurch können ein gleichmäßiges Vortreiben der Austragskolben in den Hohlräumen und ein gleichmäßiges Aufschneiden der Trennwand mit gleichbleibender Kraft erfolgen.

Es kann auch vorgesehen sein, dass die von dem Kartuschenkopf abgewandte Seite des Keils oder Kegels eine Anlagefläche für einen Stößel einer Auspressvorrichtung bildet. Der Stößel der Auspressvorrichtung ist dazu bevorzugt in einer Richtung in der Fläche der Trennwand ausgerichtet, damit der Druck, der von dem Stößel auf die Schneide ausgeübt wird, derart auf die Schneide drückt, dass die Schneide durch die Trennwand schneidet und der Keil oder Kegel durch den Schnitt in der Trennwand getrieben werden kann. Dabei soll es möglichst nicht zu einem Verkanten des Kegels oder Keils kommen.

Des Weiteren kann auch vorgesehen sein, dass die Hohlräume einen halbkreisförmigen oder kreissegmentförmigen Querschnitt aufweisen und die Austragskolben einen dazu passenden Querschnitt aufweisen, so dass die Austragskolben die Hohlräume in jeder axialen Position in den Hohlräumen verschließen.

Hiermit wird ein besonders einfacher und kostengünstig zu realisierender Knochenzement-Applikator ermöglicht.

Es kann erfindungsgemäß auch vorgesehen sein, dass die Austragskolben über das Verbindungsmittel derart voneinander beabstandet sind, dass der zwischen den Austragskolben liegende Spalt kleiner oder gleich groß ist, wie die Stärke der Trennwand.

Hiermit wird sichergestellt, dass die Austragskolben stabil in den Hohlräumen laufen und dass die Trennwand gut von dem Keil oder Kegel aufgebogen werden kann.

Es kann bei der ersten Ausführung auch vorgesehen sein, dass die Trennwand eine Stärke von maximal 1,5 mm hat, bevorzugt von maximal 1,0 mm, und/oder die Trennwand eine solche Stärke hat, dass die Trennwand mit der Schneide, auf die eine Vortriebskraft von 1 kN einwirkt, zu schneiden und von dem Keil oder Kegel aufzubiegen ist.

Hiermit wird sichergestellt, dass die Trennwand, wenn sie aus üblichen Kunststoffen hergestellt ist, problemlos mit manuell angetriebenen Auspressvorrichtungen aufgeschnitten werden kann, während die Ausgangskomponenten von den Austragskolben aus den Hohlräumen ausgepresst werden.

Bei der zweiten Ausführung kann erfindungsgemäße ferner vorgesehen sein, dass der Durchmesser des Innenraums der Kartusche kleiner oder gleich 35 mm ist, wobei bevorzugt der Durchmesser des Innenraums der Kartusche kleiner oder gleich 20 mm ist.

Es wird erfindungsgemäß mit der zweiten Ausführung zudem vorgeschlagen, dass die Kartusche einteilig mit der darin angeordneten Trennwand aufgebaut ist, vorzugsweise die Kartusche und die Trennwand als einteiliges Spritzgussteil aus Kunststoff gefertigt ist.

Hierdurch wird erreicht, dass die beiden Hohlräume dicht voneinander getrennt sind, so dass auch eine längerfristige Lagerung der Ausgangskomponenten möglich ist.

Des Weiteren kann bei der zweiten Ausführung vorgesehen sein, dass die Schneide aus einer Stahllegierung, Aluminiumlegierung, Zinklegierung, Keramik, einem Polyamid, glasfaserverstärktem Polyamid, Polyimid, Polyamid-co-imid, Polyetherketon oder Polysulfon besteht.

Diese Materialien sind gut zum Schneiden der Trennwand geeignet und kostengünstig zu verarbeiten.

Mit einer Weiterbildung der Erfindung wird vorgeschlagen, dass das Verhältnis des Innendurchmessers der Kartusche zum Abstand des Austragskolbens zum Kartuschenkopf kleiner oder gleich 1 zu 4 ist, wobei bevorzugt das Verhältnis des Innendurchmessers der Kartusche zum Abstand des Austragskolbens zum Kartuschenkopf kleiner oder gleich 1 zu 10 ist.

Die Austragskolben können bei der zweiten Ausführung zur Befüllung der Hohlräume mit den Ausgangskomponenten verwendet werden, wenn sie zunächst an dem Kartuschenkopf anliegen. Die Ausgangskomponenten werden in die Hohlräume eingepresst und dabei die Austragskolben in Richtung des Verbindungsmittels beziehungsweise der Rückseite der Kartusche gedrückt, ohne dass im Inneren der Hohlräume unerwünschte Lufteinschlüsse verbleiben, die beim Austreiben der Ausgangskomponenten mit den Austragskolben aus den Hohlräumen stören würden. Es können Rastmittel vorgesehen sein, die die Austragskolben mit dem Verbindungsmittel verbinden.

Die der vorliegenden Erfindung zugrundeliegenden Aufgaben werden auch gelöst durch ein Verfahren zur Applikation eines Knochenzements, insbesondere eines pastenförmigen Mehrkomponenten-Polymethylmethacrylat-Knochenzementteigs, mit einem erfindungsgemäßen Knochenzement-Applikator mit den folgenden nacheinander ablaufenden Schritten:
a) Einsetzen des Knochenzement-Applikators in eine Auspressvorrichtung, die Auspressvorrichtung aufweisend einen axial vortreibbaren Stößel zum Vortreiben des zumindest einen Austragskolbens und/oder der Pressvorrichtung oder dem Verbindungsmittel im Innenraum der zumindest einen Kartusche in Richtung der Öffnung der zumindest einen Kartusche,
b) das Dreiwege-Ventil wird in die erste Stellung gebracht oder es befindet sich in der ersten Stellung und es erfolgt ein Auspressen der Ausgangskomponenten mit Hilfe der Auspressvorrichtung durch axiales Vortreiben eines Stößels der Auspressvorrichtung, wobei der zumindest eine Austragskolben vom Stößel in Richtung der Öffnung gedrückt wird wodurch die Ausgangskomponenten zum Knochenzement gemischt werden und der Knochenzementteig durch den Schlauch und aus der Applikationsöffnung gedrückt wird,
c) Überführen des Dreiwege-Ventils in die zweite Stellung,
d) durch die zweite Stellung des Dreiwege-Ventils wird der Fluss der Ausgangskomponenten aus der zumindest einen Kartusche in den Schlauch gestoppt und ein Teil des zwischen der Applikationsöffnung und dem Dreiwege-Ventil im Schlauch unter Druck stehenden Knochenzementteigs wird durch das Dreiwege-Ventil in den Auffangbehälter gedrückt.

Dabei kann vorgesehen sein, dass nach Schritt d) in einem Schritt e) das Dreiwege-Ventil wieder in die erste Stellung überführt wird und dadurch der Knochenzementteig wieder durch das Dreiwege-Ventil zur Applikationsöffnung geleitet wird, wobei bevorzugt anschließend die Schritte c), d) und e) chronologisch einmal oder mehrmals wiederholt werden.

Während das Dreiwege-Ventil geschlossen ist, das heißt, sich in der zweiten Stellung befindet, wird zweckmäßigerweise der Vortrieb des Stößels und damit der Vortrieb des zumindest einen Austragskolbens gestoppt und erst dann wieder aufgenommen, wenn das Dreiwege-Ventil sich in der geöffneten ersten Stellung befindet.

Ferner kann vorgesehen sein, dass das Verfahren mit einem Knochenzement-Applikator nach der ersten Ausführung durchgeführt wird und dass während des Auspressens der Ausgangskomponenten in Schritt b) die Pressvorrichtung mit dem Stößel in Richtung eines Mischrohrs vorgetrieben wird, dabei der erste Austragskolben von der Pressvorrichtung in Richtung des Mischrohrs geschoben wird, die Klemmkante der Pressvorrichtung die Wand der zweiten Kartusche zur Innenwand der ersten Kartusche presst, dabei die verformte Wand der zweiten Kartusche den zweiten Austragskolben in der zweiten Kartusche in Richtung des Mischrohrs schiebt, wodurch die Ausgangskomponenten des Knochenzements beider Kartuschen in das Mischrohr gedrückt werden, wobei die Ausgangskomponenten im Mischrohr zu dem pastenförmigen Zementteig gemischt werden und der gemischte Zementteig aus einer Applikationsöffnung ausfließt.

Dabei kann vorgesehen sein, dass bei Vorliegen einer ersten Ausführung mit dritter Kartusche in Schritt b) beim Vortreiben der Pressvorrichtung mit dem Stößel in Richtung des Mischrohrs die Klemmkante oder eine weitere Klemmkante der Pressvorrichtung die Wand der dritten Kartusche zur Innenwand der ersten Kartusche presst, dabei die verformte Wand der dritten Kartusche den dritten Austragskolben in der dritten Kartusche in Richtung des Mischrohrs schiebt und bevorzugt beim Vortreiben der Pressvorrichtung mit dem Stößel in Richtung des Mischrohrs die Klemmkante oder eine oder mehrere weitere Klemmkante(n) der Pressvorrichtung auch die jeweilige Wand der zumindest einen vierten Kartusche zur Innenwand der ersten Kartusche presst beziehungsweise pressen, dabei die verformte Wand oder die verformten Wände der zumindest einen vierten Kartusche den jeweiligen vierten Austragskolben in der zumindest einen vierten Kartusche in Richtung des Mischrohrs schiebt.

Hiermit können mehrere Ausgangskomponenten zu einem Zementteig gemischt werden oder es kann ein symmetrischerer Krafteintrag auf die Pressvorrichtung wirken, so dass es weniger leicht zu einer Verkantung oder Behinderung der Pressvorrichtung kommt.

Ferner kann vorgesehen sein, dass die Auspressvorrichtung manuell, mit Druckluft oder mit einem Motor angetrieben wird, wobei durch die manuelle Kraft, die Druckluft oder den Motor der Stößel in Richtung des Mischrohrs vorgetrieben wird.

Manuell antreibbare Auspressvorrichtungen sind erfindungsgemäß bevorzugt, da sie weder an eine Druckluftquelle oder eine Energiequelle angeschlossen werden müssen, noch eine solche enthalten müssen.

Ferner kann bei Anwendung der ersten Ausführung vorgesehen sein, dass der Stößel der Auspressvorrichtung auf die von den Austragskolben abgewandte Seite der Pressvorrichtung drückt und die Austragskolben über die Pressvorrichtung angetrieben werden.

Alternativ kann vorgesehen sein dass das Verfahren mit einem Knochenzement-Applikator nach der zweiten Ausführung durchgeführt wird und dass während des Auspressens der Ausgangskomponenten in Schritt b) durch Auspressen der pastenförmigen Ausgangskomponenten mit Hilfe der Auspressvorrichtung durch axiales Vortreiben der Austragskolben mit dem Stößel erfolgt, wodurch die Ausgangskomponenten in den Schlauch gedrückt werden, wobei synchron zur Bewegung der Austragskolben die Trennwand mit der Schneide in Längsrichtung der Kartusche aufgeschnitten wird und mit dem Keil oder Kegel die beiden aufgeschnittenen Teile der Trennwand in Richtung der Innenwand der Kartusche zumindest derart weit nach außen gedrückt werden, dass eine weitere Bewegung des Stößels der Auspressvorrichtung nicht durch die Teile der aufgeschnittenen Trennwand verhindert oder behindert wird.

An dem in Richtung der Kartusche weisenden Ende des Stößels der Auspressvorrichtung kann ein Teller angeordnet sein, mit dem auf die Austragskolben beziehungsweise auf das Verbindungsmittel und den Keil oder Kegel gedrückt wird, um die Austragskolben in der Kartusche vorzutreiben. Die aufgeschnittenen Teile der Trennwand müssen dann derart weit nach außen an die Innenwand der Kartusche gedrückt werden, dass sie nicht in die Bewegung des Tellers eingreifen können.

Bei erfindungsgemäßen Verfahren mit der zweiten Ausführung des Knochenzement-Applikators kann vorgesehen sein, dass die beiden aufgeschnittene Teile der Trennwand mit der Innenwand der Kartusche verbunden bleiben.

Hiermit wird sichergestellt, dass keine losen Teile der aufgeschnittenen Trennwand eine weitere Bewegung des Stößels behindern können.

Auch kann vorgesehen sein, dass der Stößel der Auspressvorrichtung auf die von den Austragskolben abgewandte Seite des Keils oder Kegels drückt und die Austragskolben über den Keil oder Kegel und das Verbindungsmittel angetrieben werden.

Dadurch wird erreicht, dass die durch den Stößel verfügbare Kraft mit einem möglichst großen Anteil zum Antreiben der Ausgangskomponenten sowie zum Schneiden und Trennen der Trennwand genutzt werden kann. Es soll dadurch vermieden werden, dass ein zu großer Teil der Kraft in eine ungewollte Verformung der Kartusche oder eine störende Verkantung der Austragskolben fließt.

Des Weiteren kann vorgesehen sein, dass die von den Austragskolben abgewandte Seite des Keils oder Kegels eine Anlagefläche zum Anlegen der Vorderseite des Stößels oder eines daran angebrachten Tellers aufweist, die gleich groß oder größer ist als der Querschnitt des Stößels oder als die Auflagefläche des Tellers, wobei beim Vortreiben des Stößels der Querschnitt des Stößels oder die Auflagefläche des Tellers vollständig von der Anlagefläche abgedeckt wird oder vorzugsweise die Anlagefläche den Querschnitt des Stößels oder die Auflagefläche des Tellers überragt.

Hiermit wird erreicht, dass die Bewegung des Stößels nicht von den aufgeschnittenen Teilen der Trennwand behindert wird.

Schließlich wird im Rahmen der vorliegenden Erfindung auch vorgeschlagen, dass die Auspressvorrichtung manuell antreibbar ist oder durch Druckluft oder elektrisch antreibbar ist.

Manuell antreibbare Auspressvorrichtungen sind erfindungsgemäß bevorzugt, da sie weder an eine Druckluftquelle oder eine Energiequelle angeschlossen werden müssen, noch eine solche enthalten müssen.

Der Erfindung liegt die überraschende Erkenntnis zugrunde, dass durch Einstellen eines Dreiwege-Ventils, das mit einem Auffangbehälter, einem Schlauch und zumindest einer Kartusche verbunden ist, der Druck von dem Knochenzement im Schlauch genommen werden kann, ohne dass eine größere Menge des Knochenzements aus der Applikationsöffnung nachfließt. Gleichzeitig kann auf diese Weise der Druck des Knochenzements und der Ausgangskomponenten in der Kartusche bis zum Dreiwege-Ventil und insbesondere auch im Mischer, sofern vorhanden, aufrechterhalten werden. Dadurch ist die Zeit, die nach dem Öffnen des Dreiwege-Ventils (nach Überführung in die erste Stellung) bis zum erneuten Austritt des Knochenzements aus der Applikationsöffnung an der Spitze des Schlauchs vergeht, sehr kurz. Die Spannung des Knochenzement-Applikators wird zwischen dem Dreiwege-Ventil und dem zumindest einen Austragskolben also gehalten, wenn das Dreiwege-Ventil geschlossen wird (in der zweiten Stellung des Dreiwege-Ventils), während eine schnelle Entspannung des Knochenzement-Applikators zwischen dem Dreiwege-Ventil und der Applikationsöffnung durch Abfließen des Knochenzements durch das Dreiwege-Ventil in der zweiten Stellung erreicht wird. Damit der Knochenzement nicht die Umgebung oder den Anwender kontaminiert ist ein Auffangbehälter vorgesehen, der ein Abtropfen des durch das Dreiwege-Ventil austretenden Knochenzementteigs verhindert. Bevorzugt ist hierzu der Auffangbehälter geschlossen. Theoretisch kann es ausreichen, wenn der Knochenzementteig zurückgehalten wird. Der Auffangbehälter kann auch flexibel und/oder elastisch sein und sich bei der Aufnahme des aus dem Dreiwege-Ventil austretenden Knochenzements ausdehnen.

Der besondere Vorteil des erfindungsgemäßen Knochenzement-Applikators besteht darin, dass mit konventionellen, manuell angetriebenen Auspressvorrichtungen, die für normale PMMA-Zemente üblich sind, der Zweikomponenten-Spinezement beziehungsweise der Zweikomponenten-Knochenzement für die Vertebroplastie über einen dünnen Schlauch in den Trokar ausgepresst werden kann. Die Augmentation von Wirbelkörpern erfolgt unter permanenter Röntgenkontrolle. Der Schlauch zwischen dem Trokar und dem Applikator ermöglicht es, dass der Arzt mit seinen Händen nicht im Bereich der Röntgenstrahlung arbeitet. Es sind dabei keine komplexen, teuren Hydraulik-Applikationsvorrichtungen notwendig. Weiterhin ist es vorteilhaft, dass der Knochenzement-Applikator ein Notablassventil in Form des Dreiwege-Ventils enthält, mit dem der Auspressvorgang sofort gestoppt werden kann, wenn der Zementteig in unerwünschte Bereiche des Wirbelkörpers zu fließen beginnt. Dieses Notablassventil druckentlastet das Applikationssystem, in dem der Nachdruck des Zementteigs aus dem statischen Mischer blockiert wird und gleichzeitig der vor dem Notablassventil befindliche Zementteig druckentlastet wird, in dem ein Kanal in den Auffangbehälter geöffnet wird, in den der Zementteig austreten kann bis der Druck im Schlauch beziehungsweise im Trokar abgebaut ist. Durch den Auffangbehälter kommt es nicht zu einer Kontamination des OPs beziehungsweise der Handschuhe des Arztes durch den beim Notablass austretenden Knochenzementteig.

Die Erfindung bezüglich der ersten Ausführung basiert auch auf der Idee, in einer röhrenförmigen äußeren ersten Kartusche zumindest eine zweite kleinere innere zweite Kartusche vorzusehen, die einen kleineren Querschnitt als die größere äußere erste Kartusche besitzt, wobei in beiden Kartuschen jeweils ein axial beweglicher Austragskolben angeordnet ist, und dass der größere Austragskolben mit einer Pressvorrichtung verbunden ist, die mit einer Klemmkante die Wand der zumindest einen kleineren inneren zweiten Kartusche bei der Bewegung des Klemmkörpers in Richtung Kartuschenkopf so gegen die Wand der größeren ersten Kartusche drückt, dass die Kartuschenwand deformiert wird und bei der Vorwärtsbewegung der Pressvorrichtung der Austragskolben der kleineren zweiten Kartusche durch die deformierte Wand beziehungsweise durch die fortlaufende Deformation der Wand in Richtung Kartuschenkopf bewegt wird. Zeitgleich wird bei der Vorwärtsbewegung auch der Austragskolben der größeren ersten Kartusche in Richtung Kartuschenkopf bewegt. Die deformierte, gequetschte Kartuschenwand der inneren zweiten Kartusche rutscht durch eine äußere seitliche Öffnung der Pressvorrichtung bei der Vorwärtsbewegung der Pressvorrichtung. Es verbleiben bei dem Austrag der pastenförmigen zweiten Ausgangskomponente aus der kleineren zweiten Kartusche durch Vorwärtsbewegung des zweiten Austragskolbens praktisch keine Reste der pastenförmigen zweiten Ausgangskomponente in der deformierten kleinen zweiten Kartusche. Wichtig ist dabei, dass die Pressvorrichtung auf ihrer Rückseite eine zentrale Auflagefläche für einen Stößel besitzt, wobei die Auflagefläche außerhalb der Öffnung der Pressvorrichtung angeordnet ist, um eine ungestörte Auspressbewegung des Stößels zu ermöglichen. Weiterhin basiert die Erfindung auf der überraschenden Beobachtung, dass hochviskose Zementpasten als Ausgangskomponenten aus einer Kartusche durch einen statischen Mischer und durch einen Schlauch mit Hilfe von manuell zu betätigenden Auspressvorrichtungen ausgepresst werden können, wenn der Querschnitt der röhrenförmigen Kartusche gleich oder kleiner 35 mm ist.

Die Erfindung der ersten und der zweiten Ausführung basiert auch auf der Idee, zur Minimierung des Strömungswiderstands beim Austrag nur eine zylindrische äußere Kartusche, anstelle von mehreren side-by-side-Kartuschen oder Koaxial-Kartuschen, für die separate Lagerung der beiden pastenförmigen Ausgangskomponenten einzusetzen. Ferner wurde überraschend gefunden, dass auf diese Weise eine schmale Kartusche mit nur einem einzigen Austragskolben zum Vortreiben der beiden Ausgangskomponenten verwendet werden kann. Dadurch kann die Kraft, die notwendig ist, um die Ausgangskomponenten zu mischen und auszutreiben, minimiert werden, so dass eine mit manueller Kraft anzutreibende Auspressvorrichtung zusammen mit dem Konchenzement-Applikator eingesetzt werden kann, um die Ausgangskomponenten aus der Kartusche auszutreiben und miteinander zu mischen und durch einen Schlauch zu applizieren.

Durch das Wegdrücken der Wand der inneren Kartusche(n) mit Hilfe der Pressvorrichtung gemäß der ersten Ausführung können auch kleinere Mengen des PMMA-Knochenzements verwendet werden und auch schmalere Kartuschen mit Innenräumen mit kleineren Innendurchmessern sind noch auspressbar.

Zur Vermeidung von zwei Schubstangen und zwei Tellern zum Antreiben von zwei Austragskolben wird die zylindrische Kartusche der zweiten Ausführung mit einer axialen und axial schneidbaren Trennwand ausgerüstet, die den Innenraum der Kartusche, der durch zwei Austragskolben und einen Kartuschenkopf begrenzt ist, in zwei Hohlräume teilt, in denen die beiden pastenförmigen Ausgangskomponenten separat gelagert werden können. Durch das Schneiden und Aufbiegen der Trennwand können auch kleinere Mengen des PMMA-Knochenzements verwendet werden und auch schmalere Kartuschen mit Innenräumen mit kleineren Innendurchmessern sind noch auspressbar.

Eigene Versuche zeigten, dass während des Auspressvorgangs der zumindest einen Kartusche auf Grund der hohen Viskosität der pastenförmigen Ausgangskomponenten ein sehr großer Druckabfall im Schlauch, aber insbesondere auch an einem statischen Mischer (sofern vorhanden) stattfindet.

Weiterhin basiert die Erfindung auf der Beobachtung, dass hochviskoser Zementteig aus zylinderförmigen Kartuschen durch einen statischen Mischer und durch den Schlauch mit handelsüblichen manuell anzutreibenden Auspressvorrichtungen in akzeptabler Zeit und mit akzeptablem, da manuell aufbringbarem Kraftaufwand ausgetragen werden kann, wenn der Austragskolben an seiner Stirnseite einen Durchmesser von maximal 35 mm hat oder alle Austragskolben zusammen an ihrer Stirnseite einen Durchmesser von maximal 35 mm haben. Mit dem erfindungsgemäßen Aufbau wird ein Knochenzement-Applikator bereitgestellt, der derartig kleine Durchmesser für die Anwendung hochviskoser Ausgangskomponenten realisieren kann. Dabei kann die zumindest eine Kartusche beziehungsweise können die Hohlräume der Kartusche dennoch ohne zu großen Aufwand mit den Ausgangskomponenten befüllt werden.

Ein beispielhafter Knochenzement-Applikator ist zusammengesetzt aus
1. einer röhrenförmigen Kartusche mit einem Innendurchmesser kleiner, gleich 20 mm (bevorzugt 15 mm)
2. einer Trennwand in Längsrichtung in der Kartusche, zur Bildung von zwei getrennten Räumen für die Lagerung einer Paste A und der Paste B,
3. ein erster Austragskolben für die Paste A und ein zweiter Austragskolben für die Paste B,
4. einen Kolben, der die beiden Austragskoben miteinander verbindet, wobei der Kolben eine Schneide besitzt, mit der bei der Vorwärtsbewegung des Kolbens die Trennwand hinter den Austragskolben aufgeschnitten wird,
5. einen entfernbaren Verschluss am Kartuschenkopf
6. einem Mischrohr, indem ein statischer Mischer angeordnet ist,
7. einem Dreiwege-Ventil am distalen Ende des Mischrohrs, mit einer seitlichen Notauslassöffnung,
8. einem hohlen Reservoir, das so um das Notablassventil (Dreiwege-Ventil) angeordnet ist, dass die Notablassöffnung mit dem Hohlraum des Reservoirs verbunden ist, und
9. einem Schlauch, der an einem Ende mit dem Notablassventil und am anderen Schlauchende mit einem Luer-System-Adapter verbunden ist.

Die Pasten A und B bilden dabei die beiden Ausgangskomponenten des Zweikomponenten-Knochenzements.

Im Folgenden werden weitere Ausführungsbeispiele der Erfindung anhand von neunzehn schematisch dargestellten Figuren erläutert, ohne jedoch dabei die Erfindung zu beschränken. Dabei zeigt:
Figur 1: eine schematische perspektivische Ansicht eines beispielhaften erfindungsgemäßen Knochenzement-Applikators;
Figur 2: eine schematische perspektivische Ansicht eines vergrößerten Ausschnitts, in dem das Dreiwege-Ventil des Knochenzement-Applikators nach Figur 1 gezeigt ist;
Figur 3: zwei schematische perspektivische Querschnittansichten durch das Dreiwege-Ventil des Knochenzement-Applikators nach den Figuren 1 und 2 und zwar das Dreiwege-Ventil in geschlossener Position beziehungsweise Stellung (Figur 3 oben) und in geöffneter Position beziehungsweise Stellung (Figur 3 unten);
Figur 4: zwei schematische Querschnittansichten in Aufsicht durch das Dreiwege-Ventil des Knochenzement-Applikators nach den Figuren 1 und 2 und zwar das Dreiwege-Ventil in geöffneter Position beziehungsweise Stellung (Figur 4 oben) und in geschlossener Position beziehungsweise Stellung (Figur 4 unten);
Figur 5: eine schematische Querschnittansicht des geöffneten Dreiwege-Ventils nach den Figuren 2, 3 und 4 des Knochenzement-Applikators, wobei die Schnittebene senkrecht zu den Schnittebenen nach den Figuren 3 und 4 gewählt ist;
Figur 6: eine schematische Querschnittansicht des Luer-System-Adapters an der Spitze des Knochenzement-Applikators als Ausschnittvergrößerung;
Figur 7: eine schematische perspektivische Ansicht einer Kartusche mit einer Innenkartusche zur Realisierung einer ersten Ausführung eines erfindungsgemäßen Knochenzement-Applikators;
Figur 8: eine schematische Querschnittansicht in Längsrichtung der Rückseite der Kartusche der ersten Ausführung nach Figur 7 vor Beginn des Auspressvorgangs;
Figur 9: zwei schematische Querschnittansichten in Längsrichtung des Mittelteils der Kartusche (Figur 9 links) und des mittleren Teils des Knochenzement-Applikators (Figur 9 rechts) der ersten Ausführung nach den Figuren 7 und 8 während des Auspressvorgangs;
Figur 10: eine schematische perspektivische Ausschnittvergrößerung als Querschnittansicht der Bewegung der Austragskolben und der Pressvorrichtung in der Kartusche des Knochenzement-Applikators nach der ersten Ausführung gemäß den Figuren 7 bis 9;
Figur 11: zwei schematische perspektivische Schnittansichten des vorderen Bereichs der Kartusche der ersten Ausführung des Knochenzement-Applikators mit einem Verschluss;
Figur 12: eine vergrößerte schematische Querschnittansicht einer Variante der ersten Ausführung des Knochenzement-Applikators mit zwei Innenkartuschen während des Austreibens der Ausgangskomponenten;
Figur 13: eine schematische perspektivische Ansicht einer Kartusche mit einer Trennwand zur Realisierung einer zweiten Ausführung eines erfindungsgemäßen Knochenzement-Applikators;
Figur 14: eine schematische Querschnittansicht in Längsrichtung der Rückseite der Kartusche der zweiten Ausführung nach Figur 13 vor Beginn des Auspressvorgangs;
Figur 15: zwei schematische Querschnittansichten in Längsrichtung des Mittelteils der Kartusche (Figur 15 links) und des mittleren Teils des Knochenzement-Applikators (Figur 15 rechts) der zweiten Ausführung nach den Figuren 13 und 14 während des Auspressvorgangs;
Figur 16: eine schematische perspektivische Darstellung der Rückseite der Kartusche der zweiten Ausführung des Knochenzement-Applikators nach den Figuren 13 bis 15;
Figur 17: eine schematische perspektivische Querschnittansicht der Rückseite der zweiten Ausführung des Knochenzement-Applikators nach Figur 16, die in eine Auspressvorrichtung eingesetzt ist;
Figur 18: eine schematische perspektivische Querschnittansicht der Rückseite der zweiten Ausführung des Knochenzement-Applikators nach Figur 16, die in eine Auspressvorrichtung eingesetzt ist, wobei die Schnittebene senkrecht zu der nach Figur 17 gewählt ist; und
Figur 19: eine schematische perspektivische Querschnittansicht der Rückseite der zweiten Ausführung des Knochenzement-Applikators nach Figur 16, die in die Auspressvorrichtung eingesetzt ist, mit der Schnittebene nach Figur 18, wobei das Verbindungsmittel mit dem Stößel der Auspressvorrichtung zum Auspressen der Ausgangskomponenten des Knochenzements nach vorne getrieben wurde.

In den Figuren werden der Einfachheit halber bei gleichen Bauteilen die gleichen Bezugszeichen auch für unterschiedliche Ausführungsformen verwendet.

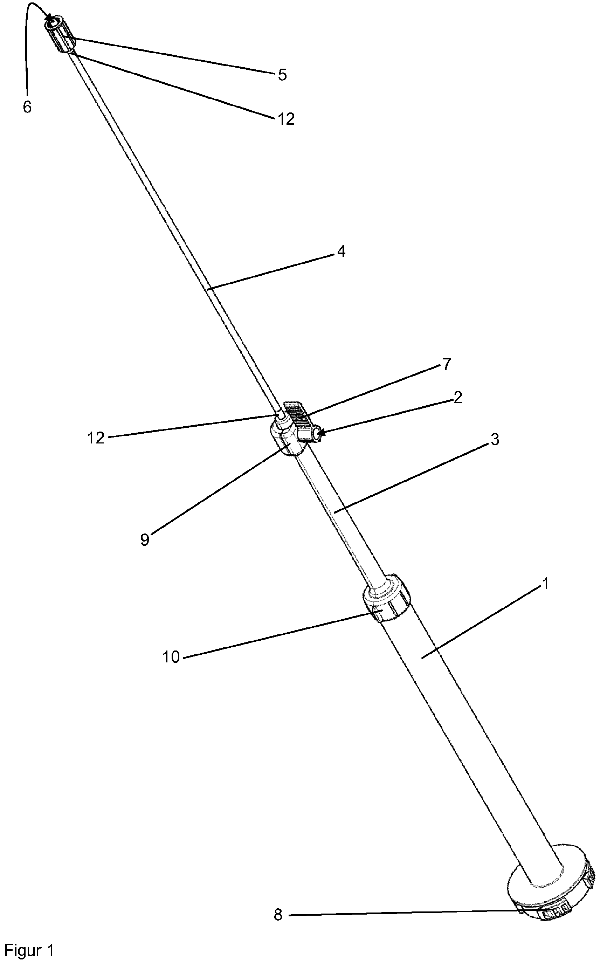

Die Figuren 1 bis 6 zeigen einen beispielhaften erfindungsgemäßen Knochenzement-Applikator für die Vertebroplastie, der in seinem Inneren zur Realisierung verschiedener Ausführungen unterschiedlich aufgebaut sein kann. Figur 1 zeigt dabei eine schematische perspektivische Ansicht eines beispielhaften erfindungsgemäßen Knochenzement-Applikators. Der Knochenzement-Applikator umfasst im Wesentlichen vier Hauptteile, nämlich eine Kartusche 1, ein Dreiwege-Ventil 2, ein Mischrohr 3 und einen Schlauch 4. In der Kartusche 1 sind die Ausgangskomponenten des herzustellenden Knochenzements gelagert. Zur Lagerung sind diese darin bevorzugt getrennt gelagert und derart aufbewahrt, dass sie über längere Zeit ohne Qualitätsverlust enthalten sein können. Hierzu können Abdichtungen verwendet werden. Bevorzugt wird dabei die Kartusche 1 erst kurz vor der Anwendung geöffnet und mit den anderen Hauptteilen des Knochenzement-Applikators verbunden. Die beiden Ausführungen des erfindungsgemäßen Knochenzement-Applikators unterscheiden sich in einem unterschiedlichen inneren Aufbau der Kartusche 1.

An der Vorderseite des Schlauchs 4 ist ein Luer-System-Adapter 5 angeordnet, in dem sich eine Applikationsöffnung 6 befindet. An diesen Luer-System-Adapter 5 kann ein Trokar (nicht gezeigt) oder auch eine Kanüle oder eine andere Verlängerung mit passendem Luer-System-Anschluss angeschlossen werden, mit dem der Knochenzementteig im Bereich der Wirbel appliziert werden kann. Der Trokar (oder die andere Verlängerung) kann beziehungsweise können auch als weiteres Teil des Knochenzement-Applikators angesehen werden. Die Applikationsöffnung 6 wird durch diese Teile entsprechend verlängert, das heißt, dass die Applikationsöffnung 6 sich durch das Anschließen des Trokars an die Spitze des Trokars verlängert. Der Knochenzement-Applikator wird üblicherweise nicht ohne den Trokar betrieben und angewendet.

Das Dreiwege-Ventil 2 kann über einen Knebelgriff 7 manuell bedient werden, in dem es um 90° gedreht wird und damit von einer geschlossenen Position beziehungsweise von einer geschlossenen Stellung in eine geöffnete Position beziehungsweise in eine geöffnete Stellung überführt wird.

An der Rückseite der Kartusche 1 beziehungsweise am Kartuschenboden ist ein Anschluss 8 zum Anschließen einer Auspressvorrichtung (nicht gezeigt) angeordnet. Die Auspressvorrichtung wird an diesem Anschluss 8 befestigt, um den Inhalt der Kartusche 1, also den Knochenzement beziehungsweise die Ausgangskomponenten des Knochenzements aus der Kartusche 1 auszupressen, dann in Flussrichtung durch das Mischrohr 3 hindurch, durch das (geöffnete) Dreiwege-Ventil 2 hindurch und durch den Schlauch 4 hindurch (und bevorzugt durch den Trokar hindurch) bis durch die Applikationsöffnung 6, über die der Knochenzement im Wirbel appliziert wird.

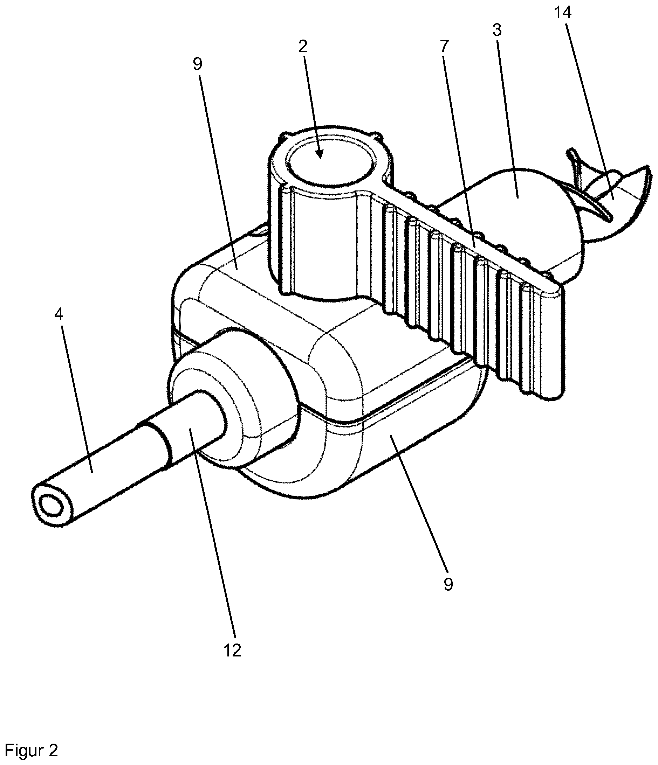

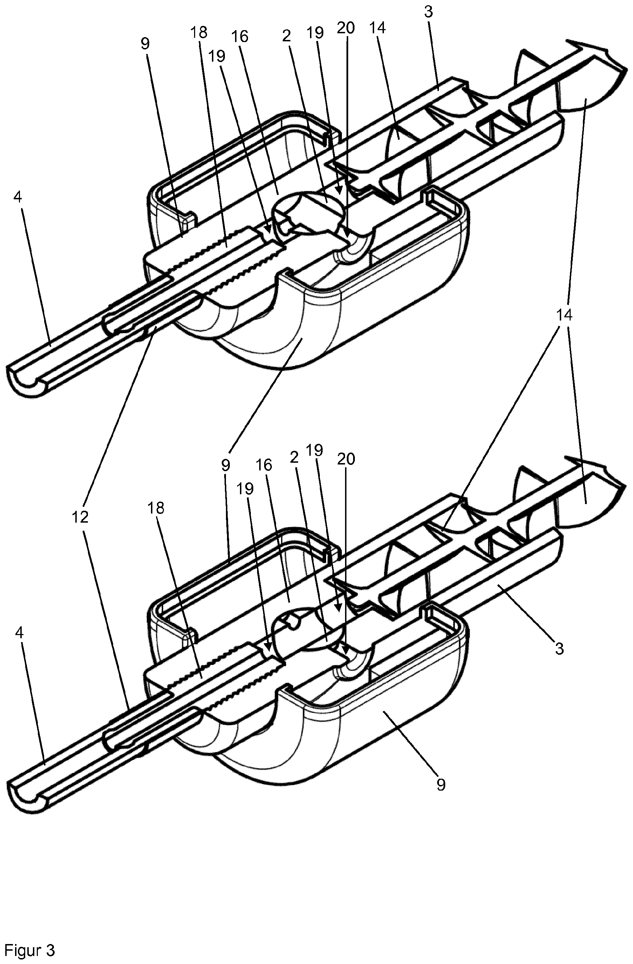

Im Bereich des Dreiwege-Ventils 2 ist ein Auffangbehälter 9 angeordnet, der das Dreiwege-Ventil 2 bezüglich der Längsachse des Knochenzement-Applikators axial umschließt. Der Auffangbehälter 9 ist aus zwei Kunststoffteilen zusammengesteckt (siehe Figuren 2 und 3).

Die Kartusche 1 und das Mischrohr 3 sind über eine Überwurfmutter 10 mit einem Innengewinde miteinander verbunden. Hierzu ist an der Kartusche 1 im Bereich des Kartuschenkopfs, der dem Kartuschenboden gegenüberliegt, ein zum Innengewinde der Überwurfmutter 10 passendes Außengewinde vorgesehen, auf das die Überwurfmutter 10 mit dem Innengewinde aufgeschraubt werden kann, um das Mischrohr 3 mit der Kartusche 1 zu verbinden. Das Mischrohr 3 weist hierzu bodenseitig eine Umfangverbreiterung nach Art eines Flanschs auf. Zwischen dem Mischrohr 3 und der Kartusche 1 ist eine Dichtung (in den Figuren 1 bis 6 nicht zu sehen) vorgesehen. Bevor die Kartusche 1 mit dem Mischrohr 3 über die Überwurfmutter 10 verschraubt ist, kann der Kartuschenkopf der Kartusche 1 mit Hilfe einer aufgeschraubten Kappe (nicht gezeigt) verschlossen sein. Dazu ist in der Kappe ein zum Außengewinde der Kartusche 1 passendes Innengewinde vorgesehen.

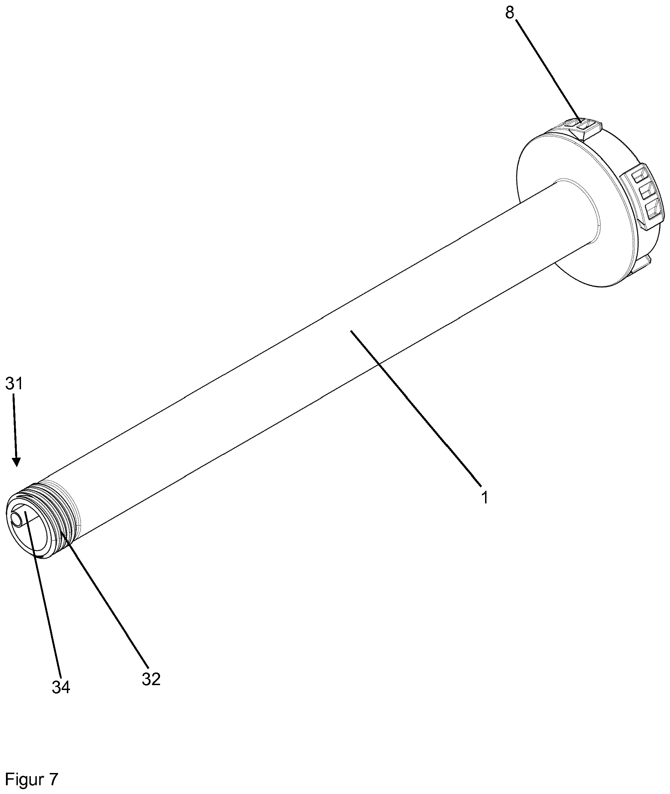

Das Dreiwege-Ventil 2 und der Luer-System-Adapter 5 sind über einen Crimpanschluss mit Hilfe von Hülsen 12 aus Metall mit dem Schlauch 4 druckdicht verbunden. Bis auf den Crimpanschluss (und gegebenenfalls bis auf Schneiden und Klemmkanten - siehe die erste und zweite Ausführung zu den Figuren 7 bis 19) können alle Teile des Knochenzement-Applikators aus Kunststoff gefertigt werden, wobei die Dichtungen bevorzugt aus elastischem Kunststoff, wie beispielsweise Gummi, gefertigt sind.

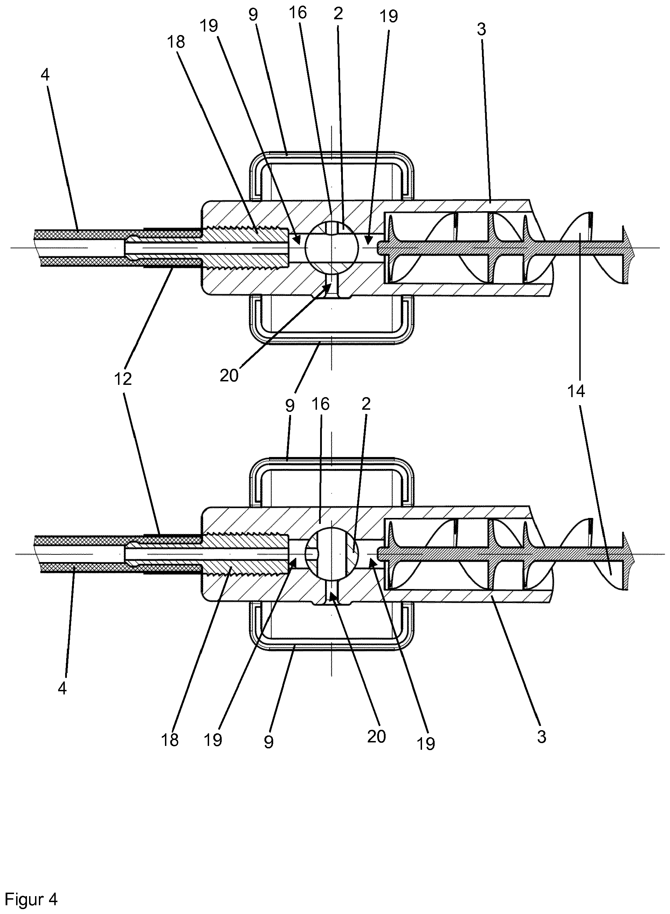

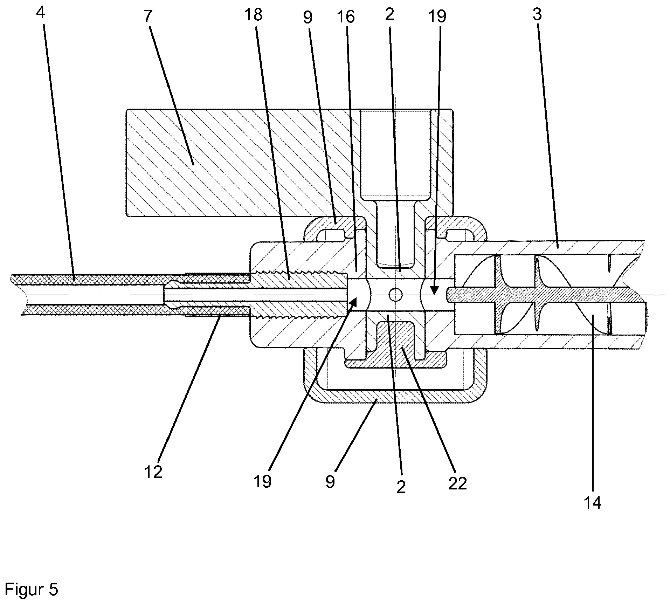

Der für die Erfindung wesentliche Hauptteil des Knochenzement-Applikators ist neben der Kartusche 1 vor allem das Dreiwege-Ventil 2, beziehungsweise insbesondere die Funktionsweise des Dreiwege-Ventils 2 zusammen mit dem Auffangbehälter 9 und mit den im Inneren des Knochenzement-Applikators gebildeten Kanälen. Figur 2 zeigt eine schematische perspektivische Ansicht eines vergrößerten Ausschnitts, in dem das Dreiwege-Ventil 2 des Knochenzement-Applikators nach Figur 1 gezeigt ist. In den Figuren 3 und 4 sind Querschnittansichten durch das Dreiwege-Ventil 2 des Knochenzement-Applikators nach den Figuren 1 und 2 dargestellt und zwar das Dreiwege-Ventil 2 in geschlossener Position beziehungsweise in geschlossener Stellung (Figur 3 oben und Figur 4 unten) und in geöffneter Position beziehungsweise in geöffneter Stellung (Figur 3 unten und Figur 4 oben), um die Funktionsweise des Dreiwege-Ventils 2 anhand des inneren Aufbaus zu erläutern. Zudem ist in Figur 5 eine schematische Querschnittansicht des geöffneten Dreiwege-Ventils nach den Figuren 2, 3 und 4 des Knochenzement-Applikators gezeigt, wobei die Schnittebene senkrecht zu den Schnittebenen nach den Figuren 3 und 4 gewählt ist.

Im Inneren des Mischrohrs 3 befindet sich ein statischer Mischer 14, der sich bis an das Dreiwege-Ventil 2 heran erstreckt. Mit Hilfe des statischen Mischers 14 werden die Ausgangskomponenten des Knochenzements durchmischt, wenn diese durch den statischen Mischer 14 im Mischrohr 3 gepresst werden.

Das drehbare Dreiwege-Ventil 2 ist in den Querschnittansichten nach den Figuren 3 und 4 in der Symmetrieebene der darin zu erkennenden Kanäle geschnitten. Die Kanäle sind also zylindrisch und setzen sich in den weggeschnittenen Teil des Dreiwege-Ventils 2 spiegelsymmetrisch fort. Die Kanäle bilden im Dreiwege-Ventil 2 ein T-Stück. Das Dreiwege-Ventil 2 sitzt in einem passenden Ventilsitz 16, der dicht an dem Dreiwege-Ventil 2 anliegt und so die Kanäle abdichtet, wenn diese in den Ventilsitz 16 gedreht sind. Im Ventilsitz 16 befinden sich zwei Durchgänge 19, durch die der größere, durchgehende Kanal im Dreiwege-Ventil 2 mit dem Mischrohr 3 auf der einen Seite und mit einem Einsatz 18 aus Metall zum Befestigen des Schlauchs 4 auf der anderen Seite fluiddurchlässig zu verbinden ist.

Senkrecht zu der Achse der beiden Durchgänge 19 befindet sich eine Durchführung 20, die den Ventilsitz 16 mit dem Inneren des nach außen geschlossenen Auffangbehälters 9 verbindet. Der Ventilsitz 16 ist einteilig aus Kunststoff mit dem Mischrohr 3 ausgeführt. In der geöffneten Position beziehungsweise Stellung des Dreiwege-Ventils 2 (Figur 3 unten, Figur 4 oben und Figur 5) ist der große durchgehende Kanal mit den beiden Durchgängen 19 verbunden und der kleine senkrechte Kanal durch den Ventilsitz 16 geschlossen. Der Knochenzement kann so aus dem Mischrohr 3 durch das Dreiwege-Ventil 2 und den Einsatz 18 in den Schlauch 4 fließen. In der geschlossenen Position beziehungsweise Stellung des Dreiwege-Ventils 2 (Figur 3 oben und Figur 4 unten) ist eine Seite des großen durchgehenden Kanals mit der Durchführung 20 zum Innenraum des Auffangbehälters 9 verbunden und der kleine senkrechte Kanal mit dem Durchgang 19 zum Schlauch 4 verbunden, während der andere Durchgang 19 zum Mischrohr 3 durch das Dreiwege-Ventil 2 verschlossen ist. Der Knochenzement kann so aus dem Schlauch 4 und gegebenenfalls dem am Luer-Adapter-Anschluss 5 angeschlossenen Trokar in den Auffangbehälter 9 fließen. Der Druck hierfür resultiert von einer elastischen Verformung des Schlauchs 4 und gegebenenfalls des Trokars, die sich beim Auspressen beziehungsweise beim Durchpressen des Knochenzements aufgebaut hat.

Das außen zylindrische Dreiwege-Ventil 2 ist durch eine zylindrische Bohrung im Ventilsitz 16 geführt und auf der dem Knebelgriff 7 gegenüberliegenden Seite mit einem Stopfen 22 verbunden und so gegen Herausfallen oder versehentliches Herausziehen aus dem Ventilsitz 16 gesichert.

Durch den erfindungsgemäßen Aufbau gelingt es, dass der Strom des Knochenzements durch Drehen und damit Schließen des Dreiwege-Ventils 2 schnell unterbrochen wird, ohne dass große Mengen des Knochenzements durch die Applikationsöffnung 6 nachströmen. Gleichzeitig werden ein Austreten des Knochenzements und damit eine Kontamination der Umgebung oder des Anwenders mit Hilfe des Auffangbehälters 9 verhindert, der überschüssigen Knochenzement aufnimmt. Des Weiteren bleibt der Druck in der Rückseite des Knochenzement-Applikators, also zwischen dem Dreiwege-Ventil 2 und dem Austragskolben oder den Austragskolben in der Kartusche 1, erhalten, so dass nach einem erneuten Öffnen des Dreiwege-Ventils 2 schnell wieder der Fluss des Knochenzements bereitgestellt werden kann, ohne dass der Druck in der Rückseite des Knochenzement-Applikators neu aufgebaut werden muss.

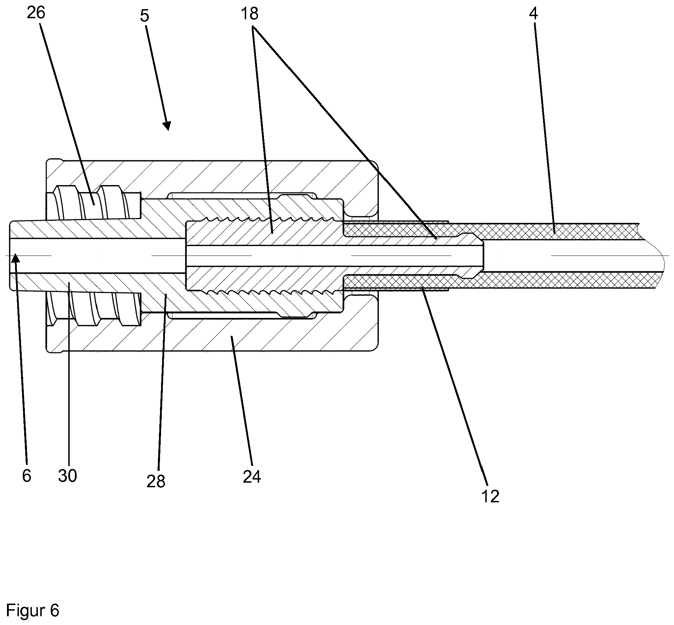

Figur 6 zeigt eine schematische Querschnittansicht des Luer-System-Adapters 5 an der Spitze des Knochenzement-Applikators als Ausschnittvergrößerung. In dem Luer-System-Adapter 5 befindet sich analog zum Anschluss des Ventilsitzes 16 an den Schlauch 4 ein Einsatz 18 aus einem metallischen Werkstoff. Der Schlauch 4 ist mit der Hülse 12 auf diesen Einsatz 18 gecrimpt, um eine druckdichte Verbindung herzustellen. Ansonsten besteht der Luer-System-Adapter 5 aus einer äußeren Hülse 24 mit einem Innengewinde 26 und einem Innenteil 28 mit einem Konus 30. Im Innenteil 28 verläuft ein Kanal, der über einen Kanal des Einsatzes 18 mit dem Schlauch 4 verbunden ist und der auf der anderen Seite in die Applikationsöffnung 6 mündet. Die äußere Hülse 24 und das Innenteil 28 sind aus Kunststoff gefertigt. Theoretisch kann auch ein anderer Adapter vorgesehen sein oder ein Trokar oder ein ähnliches Bauteil kann fest mit dem Schlauch 4 verbunden sein.

Damit der zähe Knochenzementteig mit einer manuell bedienbaren Auspressvorrichtung ausgepresst werden kann, also mit manueller Kraft ausgepresst werden kann, darf der Innendurchmesser der Kartusche 1 nicht zu groß sein. Bevorzugt ist der Innendurchmesser kleiner als 35 mm, besonders bevorzugt kleiner als 25 mm. Dadurch wird erreicht, dass der Widerstand, der durch den zähflüssigen Knochenzement im Knochenzement-Applikator verursacht wird, nicht so groß wird, dass er mit manueller Kraft nicht mehr ohne weiteres von normalen Anwendern ausgedrückt werden kann.

Theoretisch lässt sich der Knochenzement-Applikator ohne weiteres mit einer Side-by-side-Kartusche aufbauen und auch mit einem Motor- oder Druckluftgetriebenen Auspressvorrichtung bedienen. Um herkömmliche handbetriebene Auspressvorrichtungen mit einem einzelnen zentral vortreibbaren Stößel anwenden zu können, muss die Kartusche 1 des Knochenzement-Applikators geeignet aufgebaut sein. Der Vorteil des Dreiwege-Ventils 2 mit dem Auffangbehälter 9 liegt insbesondere darin, dass solche einfachen manuell antreibbaren Auspressvorrichtungen verwendet werden können, da sich der Fluss des Knochenzements schnell wieder aufbauen lässt, beziehungsweise der Druck des Knochenzements aufgebaut bleibt, ohne dass viele manuelle Hubbewegungen mit der Auspressvorrichtung durchgeführt werden müssen.

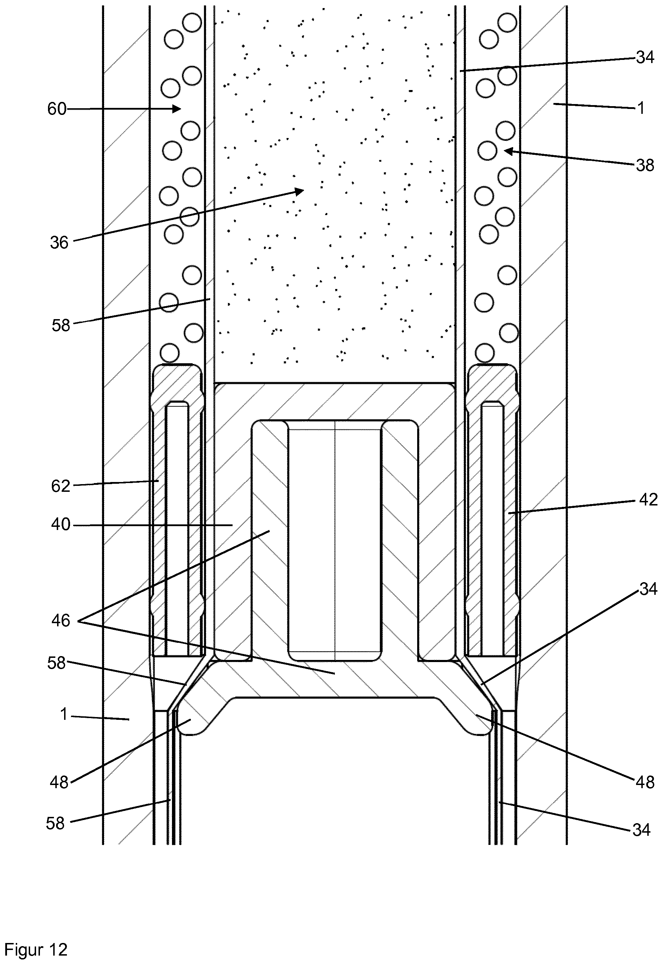

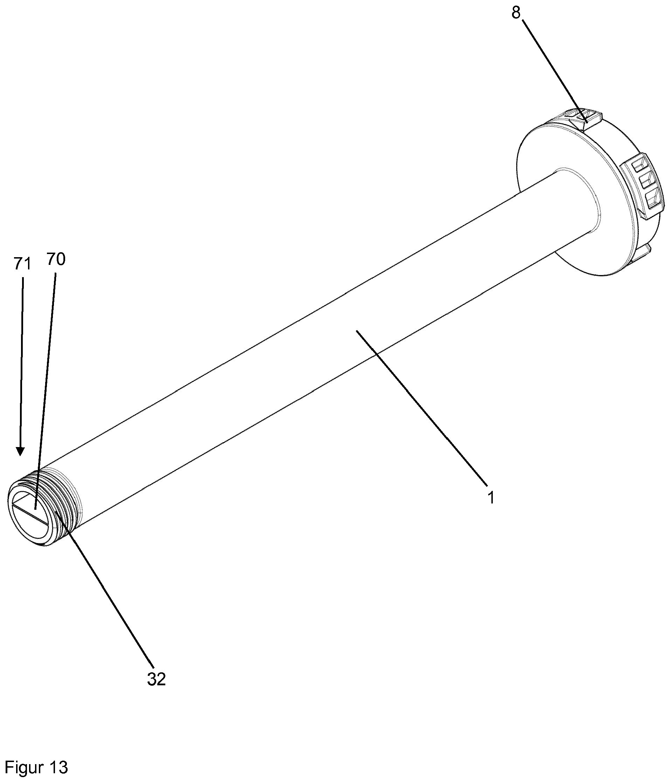

Im Folgenden werden zwei erfindungsgemäße Ausführungen vorgeschlagen, die mit einer manuell angetriebenen Auspressvorrichtung mit einem zentralen Stößel anwendbar sind. Die erste Ausführung ist in den Figuren 7 bis 11 dargestellt, wobei eine alternative Variante der ersten Ausführung in Figur 12 gezeigt ist. Die zweite Ausführung ist in den Figuren 13 bis 19 gezeigt.

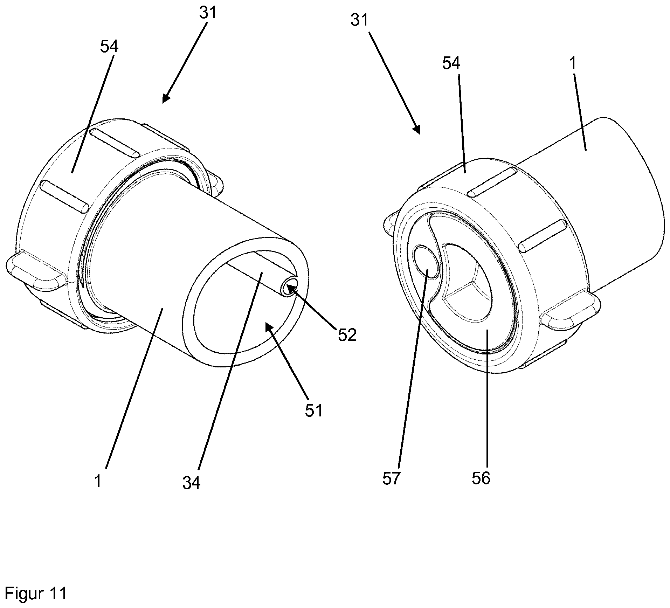

Figur 7 zeigt eine schematische perspektivische Ansicht einer Kartusche 1, die mit einer äußeren Kartusche 1 aufgebaut ist und an deren Kartuschenkopf 31 ein Außengewinde 32 vorgesehen ist. In der äußeren Kartusche 1 ist eine innere Kartusche 34 angeordnet. Diese Kartusche 1, beziehungsweise genauer das gezeigte Kartuschensystem, ist zur Realisierung einer ersten Ausführung eines erfindungsgemäßen Knochenzement-Applikators geeignet und kann in der dargestellten Form als Kartusche 1 in dem Knochenzement-Applikator verwendet werden, wie er in den Figuren 1 bis 6 dargestellt ist. In Figur 7 ist ein perspektivischer Blick auf den geöffneten Kartuschenkopf 31 dargestellt. Auf das Innengewinde 32 kann ein Verschluss geschraubt sein (siehe Figur 11), um die äußere Kartusche 1 und die innere Kartusche 34 zu verschließen, so dass deren Innenräume vollständig verschlossen sind.

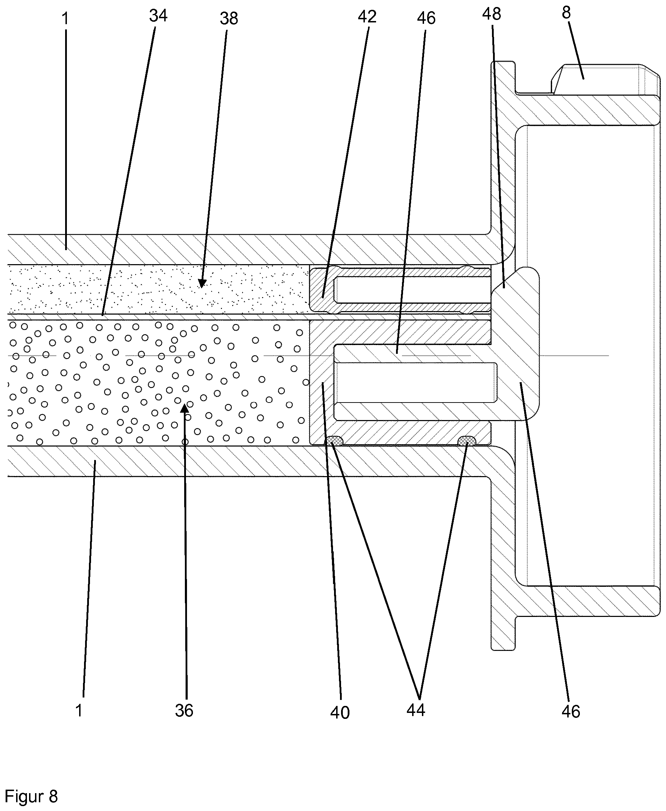

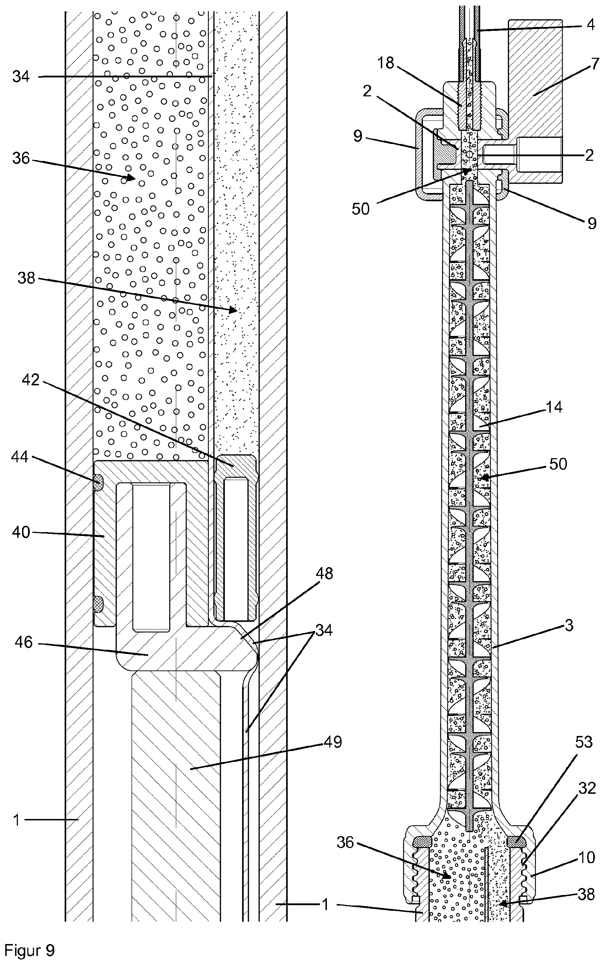

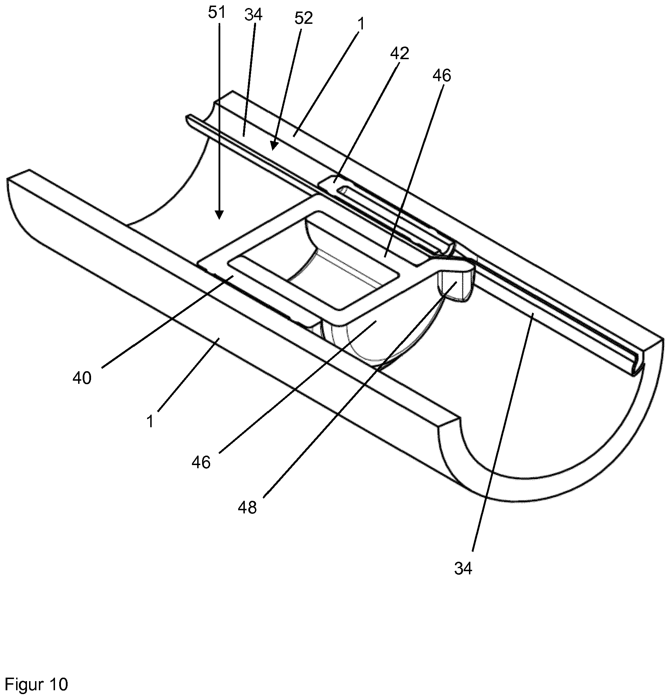

Figur 8 zeigt eine schematische Querschnittansicht in Längsrichtung der Rückseite der Kartusche 1 der ersten Ausführung nach Figur 7 vor Beginn des Auspressvorgangs. Darin ist auch der Inhalt der Kartuschen 1, 34 dargestellt, um den Mischvorgang und die Lagerung besser erklären zu können. Figur 9 zeigt zwei schematische Querschnittansichten in Längsrichtung des Mittelteils der Kartusche 1 der ersten Ausführung nach den Figuren 7 und 8 (Figur 9 links) und des mittleren Teils des Knochenzement-Applikators (Figur 9 rechts) während des Auspressvorgangs. Die Kartusche 1 nach den Figuren 7 und 8 ist an ein Mischrohr 3 mit einem Dreiwege-Ventil 2 und einem Schlauch 4 angeschlossen, wie dies in den Figuren 1 bis 6 dargestellt ist. Figur 10 zeigt eine schematische perspektivische Ausschnittvergrößerung als Querschnittansicht der Bewegung der Austragskolben und der Pressvorrichtung in der Kartusche des Knochenzement-Applikators nach der ersten Ausführung gemäß den Figuren 7 bis 9, ohne dass den Inhalt der Kartuschen 1, 34 dargestellt wäre. Figur 11 zeigt zwei schematische perspektivische Schnittansichten des vorderen Bereichs der Kartusche 1 der ersten Ausführung des Knochenzement-Applikators mit einem Verschluss, wobei der Inhalt der Kartuschen 1, 34 nicht dargestellt ist.

Der mit der Kartusche 1 nach den Figuren 7 bis 11 gezeigte Knochenzement-Applikator für die Vertebroplastie weist also die äußere erste Kartusche 1 auf, in der die innere zweite Kartusche 34 an der Innenwand der ersten Kartusche 1 über die gesamte Länge der ersten Kartusche 1 befestigt ist. Beide Kartuschen 1, 34 sind aus dem gleichen Material gefertigt. Die Wandstärke der inneren zweiten Kartusche 34 entspricht etwa einem Viertel der Wandstärke der äußeren ersten Kartusche 1. Der Innenraum der ersten Kartusche 1 ist bis auf den Raum, den die zweite Kartusche 34 einnimmt, mit einer ersten pastösen Ausgangskomponente 36 eines PMMA-Knochenzements gefüllt. Der Innenraum der zweiten Kartusche 34 ist mit einer zweiten pastösen Ausgangskomponente 38 des Zweikomponenten-PMMA-Knochenzements gefüllt. Die Innenräume der Kartuschen 1, 34 sind auf deren Rückseite (in den Figuren 8 bis 10 dargestellt) durch einen ersten Austragskolben 40 in der ersten Kartusche 1 und einen zweiten Austragskolben 42 in der zweiten Kartusche 34 begrenzt, wobei die Austragskolben 40, 42 die Innenräume der Kartuschen 1, 34 nach außen fluiddicht abschließen. Der Austragskolben 40 der ersten Kartusche 1 weist dementsprechend eine seitliche Ausnehmung auf, so dass er über die zweite Kartusche 34 gleiten kann, aber auch dort dicht abschließt. Der Austragskolben 42 der zweiten Kartusche 34 weist passend zum kleineren Innenraum einen geringeren Durchmesser auf als der Austragskolben 40 der ersten Kartusche 1.

Der Austragskolben 40 ist gegen die Innenwand der äußeren Kartusche 1 mit einer Dichtung 44 abgedichtet. An dem zweiten Austragskolben 42 zwei umlaufende Erhebungen als Dichtungen vorgesehen, mit denen der zweite Austragskolben 42 mit den Innenwänden der zweiten Kartusche 34 abschließt. Mit diesen Dichtungen 44 kann sichergestellt werden, dass der gesamte Inhalt der beiden Kartuschen 1, 34, also die beiden Ausgangskomponenten 36, 38 restlos ausgetrieben werden und so zum Herstellen einer PMMA-Knochenzement-Mischung mit dem gewünschten Verhältnis genutzt werden können. Dadurch, dass die Wand der zweiten Kartusche 34 erst nach dem Austreiben der zweiten Ausgangskomponente 38 mit dem zweiten Austragskolben 42 zusammengedrückt wird, können in den beim Zusammendrücken der Wand der zweiten Kartusche 34 entstehenden Falten keine Reste der zweiten Ausgangskomponente 38 zurückbleiben und so das Mischungsverhältnis im Zementteig nicht verfälscht werden.

Der Innenraum der zweiten Kartusche 34 ist zylindrisch mit kreisförmiger Grundfläche geformt. Der Innenraum der ersten Kartusche 1 ist ebenfalls zylindrisch mit kreisförmiger Grundfläche geformt, wobei die zweite Kartusche 34 einen Teil des Innenraums der ersten Kartusche 1 einnimmt und so eine Brechung der kreisförmigen Symmetrie des Innenraums der ersten Kartusche 1 bewirkt. Der Austragskolben 40 der ersten Kartusche 1 weist an seiner Rückseite beziehungsweise bodenseitig (in den Figuren 8 und 10 rechts, in Figur 9 (links) unten) eine Vertiefung auf, in die eine Pressvorrichtung 46 eingesteckt ist. Die Pressvorrichtung 46 weist an ihrer Rückseite eine Klemmkante 48 auf, die in den durch die zweite Kartusche 34 belegten Raum im Innenraum der ersten Kartusche 1 greift, wenn die Pressvorrichtung 46 innerhalb der ersten Kartusche 1 nach vorne (in den Figuren 8 und 10 nach links und in Figur 9 nach oben) getrieben wird. Die Klemmkante 48 weist eine abgeschrägte Oberfläche auf, die in Richtung senkrecht zur Zylinderachse der zweiten Kartusche 34 geneigt ist. Die zweite Kartusche 34 hat ein Volumen von etwa einem Zwanzigstel der ersten Kartusche 1. Dementsprechend wird der Knochenzementteig mit einem Mischungsverhältnis von etwa 20 zu 1 aus den Ausgangskomponenten 36, 38 gemischt. Aufgrund der zylindrischen Symmetrie der Innenräume der Kartuschen 1, 34 bleibt das Mischungsverhältnis während des Auspressvorgangs konstant.

Die Klemmkante 48 oder die gesamte Pressvorrichtung 46 bestehen aus einem Material und/oder sind derart geformt, dass die Pressvorrichtung 46 oder zumindest die Klemmkante 48 härter oder fester ist als die Wand der zweiten Kartusche 34. Bevorzugt besteht die Klemmkante 48 und die gesamte Pressvorrichtung 46 aus einem Metall, insbesondere einer Aluminium-Legierung, oder einem festen Kunststoff, der zumindest härter, fester und/oder zäher ist als das Material der Wand der zweiten inneren Kartusche 34.

Die Austragskolben 40, 42 sind axial in Längsrichtung in den Innenräumen der Kartuschen 1, 34 in Richtung eines Kartuschenkopfs 31 der Kartuschen 1, 34 beweglich gelagert (in den Figuren 8 und 10 von rechts nach links und in Figur 9 von unten nach oben). Wenn der Knochenzement-Applikator in eine Auspressvorrichtung eingesetzt ist, wird die Pressvorrichtung 46, und damit die Austragskolben 40, 42, mit einem Stößel 49 der Auspressvorrichtung nach vorne zum Kartuschenkopf 31 getrieben und dabei die Ausgangskomponenten 36, 38 ausgetrieben und im Mischrohr 3 zu einem Knochenzement 50 gemischt. Die erste Ausgangskomponente 36 ist in einem Innenraum 51 der ersten (äußeren) Kartusche 1 enthalten, wobei der Innenraum 51 der ersten Kartusche 1 durch die Innenwand der ersten Kartusche 1, durch die Außenwand der zweiten (inneren) Kartusche 34, durch die Vorderseite des ersten Austragskolbens 40 und gegebenenfalls durch einen Verschluss am Kartuschenkopf 31 begrenzt ist. Die zweite Ausgangskomponente 38 ist in einem Innenraum 52 der zweiten (inneren) Kartusche 34 enthalten, wobei der Innenraum 52 der zweiten Kartusche 34 durch die Innenwand der zweiten Kartusche 34, durch die Vorderseite des zweiten Austragskolbens 42 und gegebenenfalls durch einen Verschluss am Kartuschenkopf 31 begrenzt ist. Damit kein Knochenzement 50 zwischen der Kartusche 1 und dem Mischrohr 3 nach außen gedrückt wird, ist eine Dichtung 53 dazwischen angeordnet. Im Bereich des Kartuschenkopfs 31 ist im Lagerungszustand des Knochenzement-Applikators (siehe Figur 11) eine Überwurfmutter 54 aufgeschraubt, mit der eine gummielastische Platte gehalten wird, die die beiden Öffnungen der Kartuschen 1, 34 begrenzt.

Zwei Stopfen 56, 57 sind in die Öffnungen an der Vorderseite der Kartuschen 1, 34 eingesteckt, mit denen die Öffnungen und damit die Innenräume der Kartuschen 1, 34 an der Vorderseite fluiddicht verschlossen sein. Die Öffnung der ersten Kartusche 1 ist derart platziert, dass sie zu der Öffnung der zweiten Kartusche 34 hin ausgerichtet beziehungsweise benachbart ist.

An der Vorderseite der ersten Kartusche 1 ist außen das Außengewinde 32 als Befestigungselement vorgesehen, auf das die Überwurfmutter 54 aufgeschraubt werden kann beziehungsweise ist. Die Überwurfmutter 54 weist dazu ein passendes Innengewinde als Gegenbefestigungselement auf. An der Rückseite des Knochenzement-Applikators ist ein Stutzen mit einem Anschluss 8 zur Befestigung der Auspressvorrichtung vorgesehen. Die Auspressvorrichtung hält die äußere erste Kartusche 1 und weist den Stößel 49 auf, mit dem die Pressvorrichtung 46 in Richtung des Kartuschenkopfs 31 gedrückt werden kann. Die Auspressvorrichtung wird bevorzugt manuell angetrieben.

In dem Mischrohr 3 sind mehrere statische Mischer 14 angeordnet, die die Ausgangskomponenten 36, 38 miteinander durchmischen, wenn diese durch das Mischrohr 3 gepresst werden. Dadurch entsteht ein gut durchmischter Knochenzementteig 50, der über die Applikationsöffnung in einem Trokar, der an den Luer-System-Adapter 5 angeschlossen ist, oder auch über die Applikationsöffnung 6 an der Spitze des Luer-System-Adapters 5 ausgetragen beziehungsweise appliziert werden kann. Der Trokar kann Teil des erfindungsgemäßen Knochenzement-Applikators sein.

Wenn das Mischrohr 3 an der Kartusche 1 befestigt ist, wird der Knochenzement-Applikator in die Auspressvorrichtung eingesetzt und über den Anschluss 8 mit der Auspressvorrichtung verbunden. Der Stößel 49 der Auspressvorrichtung wird bodenseitig in den Knochenzement-Applikator eingetrieben und drückt so bodenseitig auf die Pressvorrichtung 46. Da der erste Austragskolben 40 mit der Pressvorrichtung 46 verbunden ist, wird dieser von der Pressvorrichtung 46 in die erste Kartusche 1 eingeschoben und dabei die erste Komponente 36 aus der ersten Kartusche 1 in das Mischrohr 3 gedrückt. Gleichzeitig wird die Wand der zweiten Kartusche 34 von der Klemmkante 48 in Richtung der Innenwand der ersten Kartusche 1 gedrückt. Durch die Verformung der Wand der zweiten Kartusche 34 wird der zweite Austragskolben 42 in Richtung des Kartuschenkopfs 31 gedrückt und so die zweite Ausgangskomponente 38 im Inneren der zweiten Kartusche 34 in das Mischrohr 3 gepresst. Diese Situation ist in den Figur 9 dargestellt.

In der Innenwand der äußeren Kartusche 1 können zwei gegenüberliegende Vertiefungen als Rastmittel (nicht gezeigt) vorgesehen sein. Dann sind in dem ersten Austragskolben 40 an der äußeren Mantelfläche zwei passende Gegenrastmittel (nicht zu sehen) vorgesehen, die in die Vertiefungen greifen können und den Austragskolben 40 dadurch in der Ausgangsposition halten, die zum Lagern der Ausgangskomponenten 36, 38 geeignet ist (siehe Figur 8). Die Rastung ist durch einen Druck auf die Rückseite des Austragskolbens 40 beziehungsweise auf die Rückseite der Pressvorrichtung 46 lösbar, so dass der erste Austragskolben 40 und damit die Pressvorrichtung 46 in Richtung des Kartuschenkopfs 31 bewegbar ist, wenn der Rastwiderstand überwunden wird.

Mit dem erfindungsgemäßen Aufbau der ersten Ausführung des Knochenzement-Applikators können auch geringe Mengen der zweiten Ausgangskomponente 38 im richtigen beziehungsweise gewünschten Mischungsverhältnis beigemischt werden. Herkömmliche Auspressvorrichtungen mit einem zentralen Stößel 49 können dazu verwendet werden, den Zementteig 50 zu mischen und auszutragen, da die Wand der zweiten Kartusche 34 nach außen in Richtung der ersten Kartusche 1 gedrückt wird und so der Bewegung des Stößels 49 nicht im Wege ist.

Die äußere erste Kartusche 1 kann dabei derart schmal gestaltet werden, erfindungsgemäß bevorzugt mit einem Innendurchmesser von maximal 35 mm oder besonders bevorzugt mit einem Innendurchmesser von maximal 20 mm, dass die zähen Ausgangskomponenten 36, 38, insbesondere die zähe erste Ausgangskomponente 36, in das Mischrohr 3 und durch die statischen Mischer 14 gedrückt werden kann, ohne dass der Widerstand der zähen Pasten 36, 38 derart groß würde, dass diese nicht mehr mit herkömmlichen, manuell angetriebenen Auspressvorrichtungen ausgetrieben werden könnten.

Mit dem Dreiwege-Ventil 2 kann der Fluss des Knochenzements 50 unterbrochen werden, ohne dass der zum Austreiben der Ausgangskomponenten 36, 38 aus den beiden Kartuschen 1, 34 und zum Zusammenpressen der Wandung der zweiten inneren Kartusche 34 notwendige Druck abgebaut werden müsste. Dadurch kann bei erneutem Öffnen des Dreiwege-Ventils 2 sofort ein weiterer Vortrieb der Pressvorrichtung 46 erfolgen. Der überschüssige Druck im Schlauch 4 und gegebenenfalls im Trokar kann durch einen Rückfluss des Knochenzementteigs 50 durch das Dreiwege-Ventil 2 in den Auffangbehälter 9 zumindest zum Teil verbraucht werden, ohne dass eine größere Menge des Knochenzementteigs 50 nach vorne austritt.

Die Figur 12 zeigt eine Alternative der ersten Ausführung des erfindungsgemäßen Knochenzement-Applikators als eine vergrößerte schematische Querschnittansicht mit zwei Innenkartuschen 34, 58 während des Austreibens der Ausgangskomponenten 36, 38, 60.

Der Knochenzement-Applikator (beziehungsweise die Kartusche 1) nach Figur 12 weist eine äußere erste Kartusche 1 auf, in der eine innere zweite Kartusche 34 an einer Innenwand der ersten Kartusche 1 über die gesamte Länge der ersten Kartusche 1 befestigt ist. Zudem ist an der gegenüberliegenden Innenwand der ersten Kartusche 1 eine innere dritte Kartusche 58 über die gesamte Länge der ersten Kartusche 1 befestigt. Alle drei Kartuschen 1, 34, 58 sind aus dem gleichen Material gefertigt. Die Wandstärken der inneren zweiten Kartusche 34 und der inneren dritten Kartusche 58 entsprechen etwa einem Viertel der Wandstärke der äußeren ersten Kartusche 1. Der Innenraum der ersten Kartusche 1 ist bis auf den Raum, den die zweite Kartusche 34 und die dritte Kartusche 58 einnehmen, mit einer ersten pastösen Ausgangskomponente 36 eines Mehrkomponenten-PMMA-Knochenzements gefüllt. Der Innenraum der zweiten Kartusche 34 ist mit einer zweiten pastösen Ausgangskomponente 38 des Mehrkomponenten-PMMA-Knochenzements gefüllt. Der Innenraum der dritten Kartusche 58 ist mit einer dritten pastösen Ausgangskomponente 60 des Mehrkomponenten-PMMA-Knochenzements gefüllt. Die Innenräume der Kartuschen 1, 34, 58 sind auf deren Rückseite (in Figur 12 unten) durch einen ersten Austragskolben 40 in der ersten Kartusche 1, einen zweiten Austragskolben 42 in der zweiten Kartusche 34 und einen dritten Austragskolben 62 in der dritten Kartusche 58 begrenzt, wobei die Austragskolben 40, 42, 62 die Innenräume der Kartuschen 1, 34, 58 nach außen fluiddicht abschließen. Der Austragskolben 40 der ersten Kartusche 1 weist dementsprechend zwei seitliche Ausnehmungen auf, so dass er über die zweite Kartusche 34 und die dritte Kartusche 58 gleiten kann. Der zweite Austragskolben 42 der zweiten Kartusche 34 und der dritte Austragskolben 62 der dritten Kartusche 58 weisen passend zum kleineren Innenraum einen geringeren Durchmesser auf als der Austragskolben 40 der ersten Kartusche 1.

Der Innenraum der zweiten Kartusche 34 und der Innenraum der dritten Kartusche 58 sind zylindrisch mit kreisförmiger Grundfläche geformt. Der Innenraum der ersten Kartusche 1 ist ebenfalls zylindrisch mit kreisförmiger Grundfläche geformt, wobei die zweite Kartusche 34 und die dritte Kartusche 58 einen Teil des Innenraums der ersten Kartusche 1 einnehmen und so eine Brechung der kreisförmigen Symmetrie des Innenraums der ersten Kartusche 1 bewirken. Der Austragskolben 40 der ersten Kartusche 1 weist an seiner Rückseite (in Figur 12 unten) eine Vertiefung auf, in die eine Pressvorrichtung 46 eingesteckt ist. Die Pressvorrichtung 46 weist an ihrer Rückseite zwei Klemmkanten 48 beziehungsweise eine Klemmkante 48 auf, die in die durch die zweite Kartusche 34 und dritte Kartusche 58 belegten Räume im Innenraum der ersten Kartusche 1 greifen beziehungsweise greift, wenn die Pressvorrichtung 46 innerhalb der ersten Kartusche 1 nach vorne (in Figur 12 nach oben) getrieben wird. Die Klemmkanten 48 weisen jeweils eine abgeschrägte Oberfläche auf, die in Richtung senkrecht zur Zylinderachse der zweiten Kartusche 34 und dritten Kartusche 58 geneigt sind. Die zweite Kartusche 34 und die dritte Kartusche 58 haben ein gleich großes Volumen und zusammen ein Volumen von etwa einem Zwanzigstel der ersten Kartusche 1. Dementsprechend wird der Zementteig mit einem Mischungsverhältnis von etwa 40 zu 1 zu 1 aus den drei Ausgangskomponenten 36, 38, 60 gemischt.

Aufgrund der zylindrischen Symmetrie der Innenräume der Kartuschen 1, 34, 58 bleibt das Mischungsverhältnis während des Auspressvorgangs konstant.

Anstatt einer dritten Ausgangskomponente 60 kann auch die zweite Komponente 38 sowohl in der zweiten Kartusche 34 als auch in der dritten Kartusche 58 enthalten sein. Dadurch wird ein Zweikomponenten-Knochenzement mit einem Mischungsverhältnis von 20 zu 1 gemischt. Der Vorteil gegenüber der mit den Figuren 7 bis 11 gezeigten Variante der ersten Ausführung ist darin zu sehen, dass durch den symmetrischen Aufbau während des Austragens der Ausgangskomponenten 36, 38 keine Kräfte senkrecht zur Zylinderachse beziehungsweise Symmetrieachse entstehen können, durch die die Pressvorrichtung 46 verkanten könnte und dadurch in ihrer Bewegung behindert wird.

Die Klemmkante 48 oder die gesamte Pressvorrichtung 46 bestehen aus einem Material und/oder sind derart geformt, dass die Pressvorrichtung 46 oder zumindest die Klemmkante 48 härter oder fester ist als die Wand der zweiten Kartusche 34 und die Wand der dritten Kartusche 58. Bevorzugt besteht die Klemmkante 48 und die gesamte Pressvorrichtung 46 aus einem Metall, insbesondere einer Aluminium-Legierung, oder einem festen Kunststoff, der zumindest härter, fester und/oder zäher ist als das Material der Wand der zweiten inneren Kartusche 34 und der dritten inneren Kartusche 58.

Die Austragskolben 40, 42, 62 sind axial in Längsrichtung in den Innenräumen der Kartuschen 1, 34, 58 in Richtung eines Kartuschenkopfs der Kartuschen 1, 34, 58 beweglich gelagert (in Figur 12 von unten nach oben). In dem Kartuschenkopf sind eine Öffnung der ersten Kartusche 1, eine Öffnung der zweiten Kartusche 34 und eine Öffnung der dritten Kartusche 58 vorgesehen (in Figur 12 nicht zu sehen). Im Bereich des Kartuschenkopfs ist im Lagerungszustand analog der ersten Variante der ersten Ausführung eine Überwurfmutter aufgeschraubt, mit der eine gummielastische Platte gehalten wird, die die drei Öffnungen begrenzt.

An der Vorderseite der ersten Kartusche 1 ist hierzu außen ein Außengewinde als Befestigungselement vorgesehen, auf das die Überwurfmutter aufgeschraubt werden kann. An der Rückseite der Kartusche 1 ist ein Stutzen mit einem Anschluss zur Befestigung einer Auspressvorrichtung (nicht gezeigt) vorgesehen. Die Auspressvorrichtung hält die äußere erste Kartusche 1 und weist einen Stößel auf, mit dem die Pressvorrichtung 46 in Richtung des Kartuschenkopfs gedrückt werden kann. Die Auspressvorrichtung wird manuell angetrieben.

Zur Anwendung des Knochenzement-Applikators nach der zweiten Variante der ersten Ausführung nach Figur 12 ist die Kartusche 1 wie in den Figuren 1 bis 6 eingebaut und mit dem Mischrohr 3 verbunden. Dann wird der Knochenzement-Applikator in die Auspressvorrichtung (nicht gezeigt) eingesetzt und über den Anschluss mit der Auspressvorrichtung verbunden. Der Stößel der Auspressvorrichtung wird bodenseitig in den Knochenzement-Applikator eingetrieben und drückt so bodenseitig auf die Pressvorrichtung 46. Da der erste Austragskolben 40 mit der Pressvorrichtung 46 verbunden ist, wird dieser von der Pressvorrichtung 46 in die erste Kartusche 1 eingeschoben und dabei die erste Komponente 36 aus der ersten Kartusche 1 in das Mischrohr 3 gedrückt. Gleichzeitig wird die Wand der zweiten Kartusche 34 von der Klemmkante 48 und die Wand der dritten Kartusche 58 von der gegenüberliegenden Klemmkante 48 in Richtung der Innenwand der ersten Kartusche 1 gedrückt. Durch die Verformung der Wand der zweiten Kartusche 34 wird der zweite Austragskolben 42 in Richtung des Kartuschenkopfs (in Figur 12 nach oben) gedrückt und so die zweite Ausgangskomponente 38 im Inneren der zweiten Kartusche 34 in das Mischrohr 3 gepresst. Ebenso wird durch die Verformung der Wand der dritten Kartusche 58 der dritte Austragskolben 62 in Richtung des Kartuschenkopfs gedrückt und so die dritte Ausgangskomponente 60 im Inneren der dritten Kartusche 58 in das Mischrohr 3 gepresst.

An dem ersten Austragskolben 40 sind zwei umlaufende Erhebungen als Dichtungen vorgesehen (in Figur 12 nicht zu sehen), mit denen der erste Austragskolben 40 mit den Innenwänden der ersten Kartusche 1 abschließt. Ebenso sind an dem zweiten Austragskolben 42 zwei umlaufende Erhebungen als Dichtungen vorgesehen, mit denen der zweite Austragskolben 42 mit den Innenwänden der zweiten Kartusche 34 abschließt. Ferner sind an dem dritten Austragskolben 62 zwei umlaufende Erhebungen als Dichtungen vorgesehen, mit denen der dritte Austragskolben 62 mit den Innenwänden der dritten Kartusche 58 abschließt. Mit den Dichtungen kann sichergestellt werden, dass der gesamte Inhalt der drei Kartuschen 1, 34, 58, also die drei Ausgangskomponenten 36, 38, 60 restlos ausgetrieben werden und so zum Herstellen einer PMMA-Knochenzement-Mischung mit dem gewünschten Verhältnis genutzt werden können. Dadurch, dass die Wand der zweiten Kartusche 34 erst nach dem Austreiben der zweiten Ausgangskomponente 42 zusammengedrückt wird und die Wand der dritten Kartusche 58 erst nach dem Austreiben der dritten Ausgangskomponente 60 (oder alternativ der zweiten Ausgangskomponente 42) zusammengedrückt wird, können in den beim Zusammendrücken der Wand der zweiten Kartusche 34 und der dritten Kartusche 58 entstehenden Falten keine Reste der zweiten Ausgangskomponente 38 und der dritten Ausgangskomponente 60 zurückbleiben und so das Mischungsverhältnis im Zementteig nicht verfälscht werden.

Mit dem erfindungsgemäßen Aufbau des Knochenzement-Applikators gemäß der zweiten Variante der ersten Ausführung können auch geringe Mengen der zweiten Ausgangskomponente 38 und der dritten Ausgangskomponente 60 im richtigen beziehungsweise gewünschten Mischungsverhältnis beigemischt werden. Herkömmliche Auspressvorrichtungen mit einem zentralen Stößel können dazu verwendet werden, den Zementteig zu mischen und auszutragen, da die Wände der zweiten Kartusche 34 und dritten Kartusche 58 nach außen in Richtung der ersten Kartusche 1 gedrückt werden und so der Bewegung des Stößels nicht im Wege sind.

Die äußere erste Kartusche 1 kann dabei derart schmal gestaltet werden, erfindungsgemäß bevorzugt mit einem Innendurchmesser von maximal 35 mm oder besonders bevorzugt mit einem Innendurchmesser von maximal 20 mm, dass die zähen Ausgangskomponenten 36, 38, 60 insbesondere die zähe erste Ausgangskomponente 36, in das Mischrohr 3 und durch die statischen Mischer 14 gedrückt werden kann, ohne dass der Widerstand der zähen Pasten 36, 38, 60 derart groß würde, dass diese nicht mehr mit herkömmlichen, manuell angetriebenen Auspressvorrichtungen ausgetrieben werden könnten.

Die Figuren 13 bis 19 zeigen eine zweite Ausführung eines erfindungsgemäßen Knochenzement-Applikators, bei der wiederum nur die Kartusche 1 mit ihrem inneren Aufbau sich von der ersten Ausführung unterscheidet. Figur 13 zeigt hierzu eine schematische perspektivische Ansicht einer Kartusche 1 mit einer Trennwand 70 zur Realisierung der zweiten Ausführung des erfindungsgemäßen Knochenzement-Applikators. An dem Kartuschenkopf 71 der Kartusche nach der zweiten Ausführung ist ein Außengewinde 32 vorgesehen. Diese Kartusche 1 ist zur Realisierung einer zweiten Ausführung eines erfindungsgemäßen Knochenzement-Applikators geeignet und kann in der dargestellten Form als Kartusche 1 in dem Knochenzement-Applikator verwendet werden, wie er in den Figuren 1 bis 6 dargestellt ist. In Figur 13 ist ein perspektivischer Blick auf den geöffneten Kartuschenkopf 71 dargestellt. Auf das Außengewinde 32 kann ein Verschluss geschraubt sein, um die durch die Trennwand 70 getrennten Bereiche der Kartusche 1 zu verschließen, so dass deren Innenräume vollständig verschlossen sind.

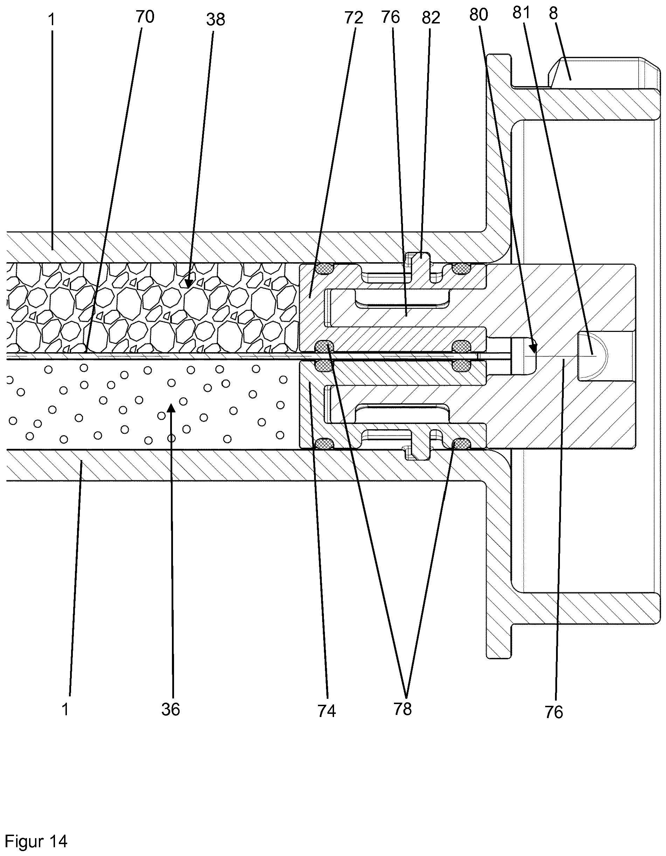

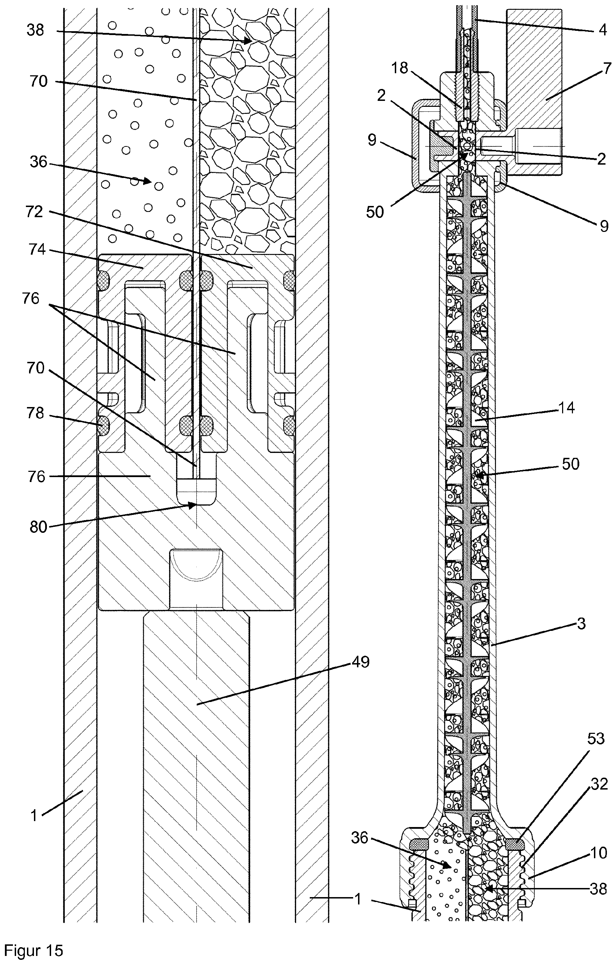

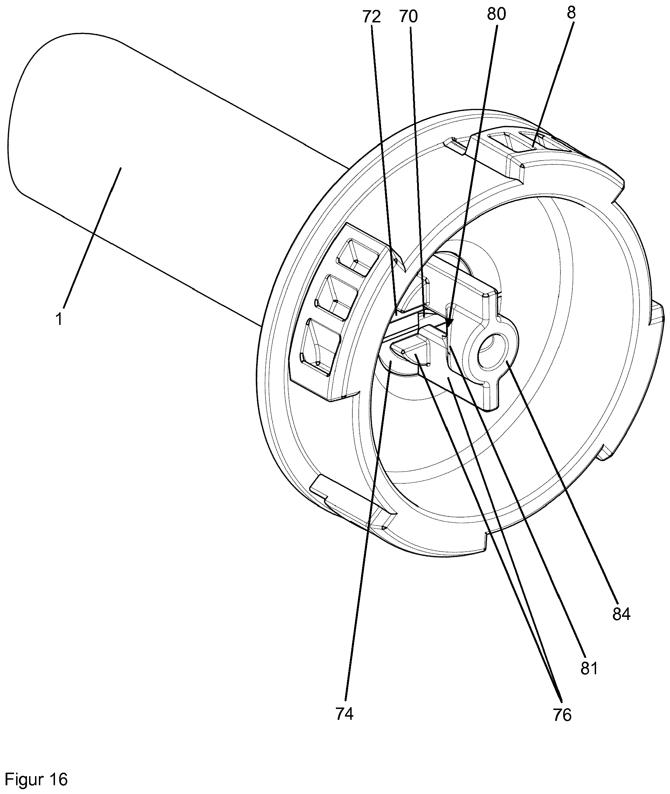

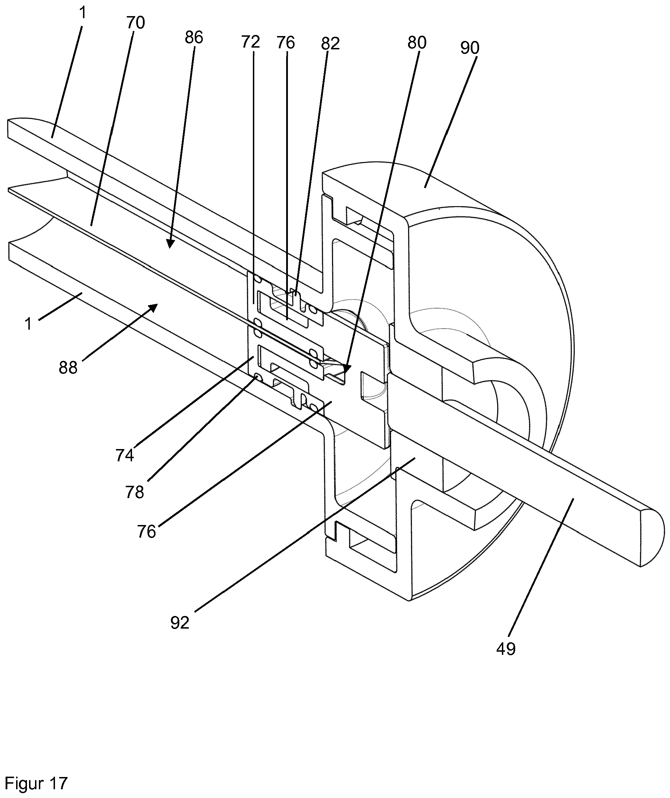

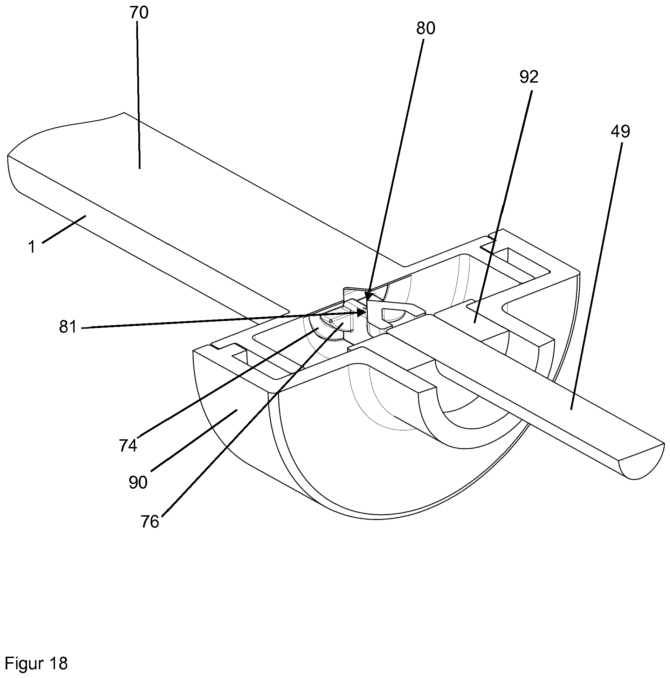

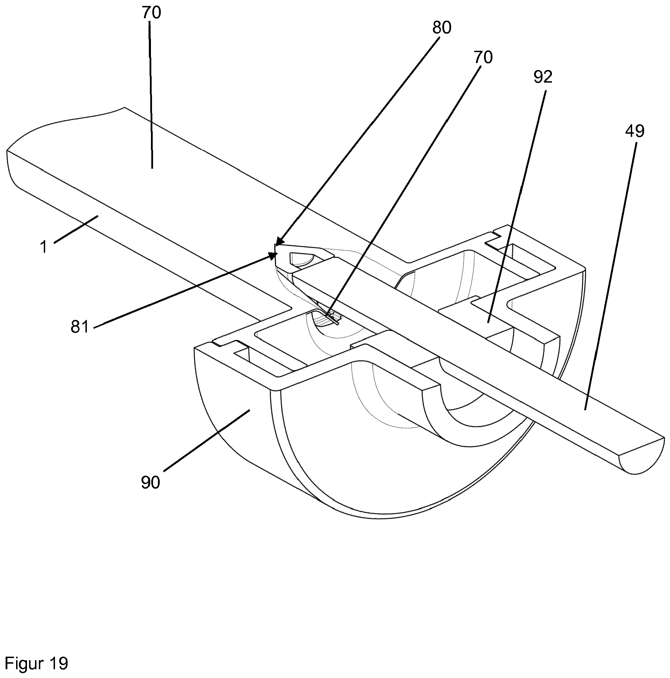

Figur 14 zeigt eine schematische Querschnittansicht in Längsrichtung der Rückseite der Kartusche 1 der zweiten Ausführung nach Figur 13 vor Beginn des Auspressvorgangs und Figur 15 zwei schematische Querschnittansichten in Längsrichtung des Mittelteils der Kartusche (Figur 15 links) und des mittleren Teils des Knochenzement-Applikators (Figur 15 rechts) der zweiten Ausführung nach den Figuren 13 und 14 während des Auspressvorgangs. Figur 16 zeigt eine schematische perspektivische Darstellung der Rückseite der Kartusche der zweiten Ausführung des Knochenzement-Applikators nach den Figuren 13 bis 15 und Figur 17 eine schematische perspektivische Querschnittansicht der Rückseite der zweiten Ausführung des Knochenzement-Applikators nach Figur 16, die in eine Auspressvorrichtung eingesetzt ist. Ferner zeigt Figur 18 eine schematische perspektivische Querschnittansicht der Rückseite der zweiten Ausführung des Knochenzement-Applikators nach Figur 16, die in eine Auspressvorrichtung eingesetzt ist, wobei die Schnittebene senkrecht zu der nach Figur 17 gewählt ist, und Figur 19 eine schematische perspektivische Querschnittansicht der Rückseite der zweiten Ausführung des Knochenzement-Applikators nach Figur 16, die in die Auspressvorrichtung eingesetzt ist, mit der Schnittebene nach Figur 18.

Der Knochenzement-Applikator gemäß der zweiten Ausführung weist als zentralen Bestandteil eine zylindrische Kartusche 1 auf, bei der eine Trennwand 70 zwei gegenüberliegende Innenseiten der Innenwand der zylindrischen Kartusche 1 verbindet. Die Kartusche 1 und die Trennwand 70 sind einteilig als gemeinsames Spitzgussteil aufgebaut. Die Trennwand 70 trennt den Innenraum der Kartusche 1 in zwei separate und fluiddicht voneinander getrennte Hohlräume 86, 88 (siehe Figur 17), in denen die beiden pastösen Ausgangskomponenten 36, 38 eines zu mischenden PMMA-Knochenzements 50 lagern.

Auf der Rückseite (in Figur 13 und 14 rechts) sind die Hohlräume 86, 88 durch zwei Austragskolben 72, 74 begrenzt, wobei die Austragskolben 72, 74 axial in den beiden Hohlräumen 86, 88 verschiebbar gelagert sind. Figur 17 zeigt hierzu eine Detailansicht in Form einer vergrößerten schematischen perspektivischen Teilquerschnittansicht des Kartuschenbodens der zweiten Ausführung des erfindungsgemäßen Knochenzement-Applikators. An der den Austragskolben 72, 74 gegenüberliegenden Vorderseite der Kartusche 1 sind die beiden Hohlräume 86, 88 durch einen Kartuschenkopf 71 mit einer gummielastischen Platte verschlossen, wenn die Kartusche 1 zum Lagern der Ausgangskomponenten 36, 38 verwendet wird. Zum Applizieren des Knochenzements 50 werden die beiden Hohlräume 86, 88 der Kartusche 1 durch Öffnen des Kartuschenkopfs 71 geöffnet und die Kartusche 1 anschließend mit dem Mischrohr 3, dem Dreiwege-Ventil 2 und dem Schlauch 4 verbunden, so wie dies in den Figuren 1 bis 6 gezeigt ist, wobei am Luer-System-Adapter 5 ein Trokar angeschlossen wird. Der Knochenzement-Applikator wird anschließend in eine Auspressvorrichtung eingesetzt, mit der die Austragskolben 72, 74 zum Austreiben der Ausgangskomponenten 36, 38 aus den Hohlräumen 86, 88 der Kartusche 1 manuell nach vorne in Richtung des Mischrohrs 3 gepresst werden können.

In dem Kartuschenkopf 71 können zwei Durchführungen vorgesehen sein, die mit jeweils einem Stopfen (nicht gezeigt) verschlossen sind. Durch die beiden Durchführungen sind die Hohlräume 86, 88 zugänglich, wenn die Stopfen nicht darin eingesteckt sind beziehungsweise wenn der Kartuschenkopf 71 geöffnet ist.

An der Rückseite der Kartusche 1 beziehungsweise bodenseitig (in Figur 13 und 14 rechts) ist an der Kartusche 1 ein Anschluss 8 mit Befestigungselementen angeordnet. Über den Anschluss 8 und die Befestigungselemente kann die Kartusche 1 an die Auspressvorrichtung beziehungsweise einen Applikator (in Figur 13 und 14 nicht gezeigt) angeschlossen werden. An der gegenüberliegenden Vorderseite (in Figur 1 links) der Kartusche 1 kann am Kartuschenkopf 71 im Lagerungszustand des Knochenzement-Applikators ein Verschluss (nicht gezeigt) mit einer Überwurfmutter (nicht gezeigt) befestigt sein, indem auf ein Außengewinde 32 an der Kartusche 1 ein Innengewinde der Überwurfmutter aufgeschraubt ist. Eine gummielastische Platte dichtet dann die Hohlräume 86, 88 nach vorne ab. Die Kartusche 1 hat einen Außendurchmesser von 27 mm, einen Innendurchmesser von 25 mm und eine Länge von etwa 18 cm.

Die beiden Austragskolben 72, 74 sind an deren Rückseite über ein Verbindungsmittel 76 miteinander verbunden. Das Verbindungsmittel 76 erstreckt sich dazu mit zwei in Richtung des Kartuschenkopfs weisenden zylindersegmentförmigen Enden in passende Hohlräume in den Rückseiten der Austragskolben 72, 74 hinein. Die Austragskolben 72, 74 sind außen gegen die Innenwand der Kartusche 1 und gegen die Trennwand 70 mit jeweils zwei umlaufenden Gummidichtungen 78 abgedichtet. Eine in Richtung der Trennwand 70 weisende vordere Kante des Verbindungsmittels 76 ist als Klinge mit einer Schneide 80 ausgeführt, die sich in Richtung der Rückseite des Knochenzement-Applikators über einen Keil 81 oder Kegel 81 beziehungsweise keilförmig und bereichsweise kegelförmig verbreitert. An der Rückseite des Keils 81 beziehungsweise Kegels 81 ist das Verbindungsmittel 76 als Anlagefläche 84 für einen Stößel 49 einer Auspressvorrichtung (nicht gezeigt) ausgeformt.

Die Austragskolben 72, 74 sind mit jeweils einem lösbaren Rastelement 82 mit passenden Gegenrastelementen (in Form von zwei Vertiefungen) in der Innenwand der Kartusche 1 verbunden. Die Austragskolben 72, 74 können durch einen Druck von der Rückseite der Kartusche 1 (in Figur 1 von rechts) in Richtung der Vorderseite der Kartusche 1 (in Figur 1 links) also in Richtung des Kartuschenkopfs 7 gedrückt werden. Die Rastelemente 82 können durch einen bodenseitigen Druck auf die Austragskolben 72, 74, wenn der Kartuschenkopf 71 geöffnet oder die Stopfen entfernt sind, ohne weiteres gelöst werden und dienen vor allem dazu, dass die Austragskolben 72, 74 beim Befüllen des Innenraums der Kartusche 1 mit den Ausgangskomponenten 36, 38 nicht bodenseitig aus der Kartusche 1 herausgedrückt werden können, beziehungsweise nicht über die gewünschte, durch die Gegenrastelemente in der Innenwand der Kartusche 1 definierte Position hinaus in Richtung des Kartuschenbodens (in Figur 1 rechts) gedrückt werden können.

Figur 14 zeigt eine schematische perspektivische Querschnittansicht durch den erfindungsgemäßen Knochenzement-Applikator gemäß der zweiten Ausführung, unmittelbar vor der Applikation des PMMA-Knochenzements. Figur 15 zeigt den Knochenzement-Applikator gemäß der zweiten Ausführung während des Austreibens der beiden Ausgangskomponenten 36, 38.

Die Ausgangskomponenten 36, 38 werden gemischt, indem sie durch das Mischrohr 3 und damit durch den statischen Mischer 14 gedrückt werden. Dadurch entsteht ein gut durchmischter Knochenzementteig 50, der über die Applikationsöffnung in einem Trokar, der an den Luer-System-Adapter 5 angeschlossen ist, oder auch über die Applikationsöffnung 6 an der Spitze des Luer-System-Adapters 5 ausgetragen beziehungsweise appliziert werden kann. Der Trokar kann Teil des erfindungsgemäßen Knochenzement-Applikators sein.

Das Mischrohr 3 weist ein Innengewinde auf, das auf das Außengewinde 32 der Kartusche 1 passt, so dass das Mischrohr 3 stabil und fest mit der Kartusche 1 verbunden werden kann. Zwischen dem Mischrohr 3 und der Vorderseite der Kartusche 1 ist ein Dichtungsring 53 angeordnet, damit die Ausgangskomponenten 36, 38 nicht zwischen dem Mischrohr 3 und der Kartusche 1 austreten können. Über das Außengewinde 32, das Innengewinde und den Dichtungsring 53 wird eine druckstabile und druckdichte Verbindung zwischen dem Mischrohr 3 und der Kartusche 1 erreicht.

Der Vortrieb der Austragskolben 72, 74 wird über eine Auspressvorrichtung erzeugt, die an den Anschluss 8 angeschlossen wird und mit der ein Stößel 49 beziehungsweise eine Schubstange der Auspressvorrichtung manuell in Richtung des Mischrohrs 3 vorgetrieben werden kann. Der Stößel 49 drückt dann auf die Anlagefläche 84, so dass einerseits die Austragskolben 72, 74 in Richtung des Mischrohrs 3 vorgetrieben werden und andererseits die Schneide 80 in die Trennwand 70 getrieben wird und diese dabei aufschneidet, so wie dies in Figur 19 dargestellt ist. Der Druck des Stößels 49 löst dabei die Rastelemente 82 und treibt die Austragskolben 72, 74 nach vorne. Die Austragskolben 72, 74 schließen dicht mit den Innenwänden der Kartusche 1 und der Trennwand 70 ab. Dadurch kann der Inhalt der Hohlräume 86, 88 der Kartusche 1, nämlich die zwei enthaltenen pastösen Ausgangskomponenten 36, 38, nach vorne in das Mischrohr 3 ausgetrieben werden, wo die Ausgangskomponenten 36, 38 zum Zementteig 50 gemischt werden.

Nach dem Öffnen der Hohlräume 86, 88 kann die Kartusche 1 mit den anderen Hauptteilen 2, 3, 4 des Knochenzement-Applikators verbunden werden und der Knochenzement-Applikator wird über den Anschluss 8 beziehungsweise über das Befestigungsmittel mit der Auspressvorrichtung verbunden.

Das Verbindungsmittel 76 wird durch einen Druck auf die Anlagefläche 84 in Richtung des Mischrohrs 3 vorgetrieben. Diese Situationen sind zusammen mit den wesentlichen Teilen einer Auspressvorrichtung zum Vortreiben des Verbindungsmittels in den Figuren 17 bis 19 gezeigt. Die Rastmittel 82 lösen sich aus den Vertiefungen und die Austragskolben 72, 74 werden in den Hohlräumen 86, 88 nach vorne gedrückt. Dabei werden die beiden Ausgangskomponenten 36, 38 nach vorne in das Mischrohr 3 gepresst und dort gemischt. Bei einem weiteren Vortreiben des Verbindungsmittels 76 werden nicht nur die Austragskolben 72, 74 weiter in den Hohlräumen 86, 88 der Kartusche 1 vorgetrieben, sondern die Schneide 80 greift auch in die Kerbe der Trennwand 70 und beginnt diese in axialer Richtung aufzuschneiden. Diese Situation ist in den Figuren 15 und 17 gezeigt.

Durch weiteres Vortreiben des Verbindungsmittels 76 drückt der hinter der Schneide 80 angeordnete Keil 81 oder Kegel 81 die aufgeschnittenen Teile der Trennwand 70, die noch immer mit der Innenwand der Kartusche 1 verbunden sind, auseinander und drückt diese in Richtung der Innenwand der Kartusche 1. Währenddessen werden die Ausgangskomponenten 36, 38 weiter aus den Hohlräumen 86, 88 in das Mischrohr 3 gedrückt und dort gemischt. Schließlich tritt der fertig gemischte Zementteig 50 durch das Dreiwege-Ventil 2, den Schlauch 4, den Luer-System-Adapter 5, gegebenenfalls den Trokar (nicht gezeigt), und die Austragsöffnung 6 aus und kann am gewünschten Ort appliziert werden. Mit dem Dreiwege-Ventil 2 kann der Fluss des Knochenzements 50 unterbrochen werden, ohne dass der zum Austreiben der Ausgangskomponenten 36, 38 aus den beiden Hohlräumen 86, 88 und zum Schneiden der Trennwand 70 notwendige Druck abgebaut werden müsste. Dadurch kann bei erneutem Öffnen des Dreiwege-Ventils 2 sofort ein weiterer Vortrieb des Verbindungsmittels 76 erfolgen. Der überschüssige Druck im Schlauch 4 und gegebenenfalls im Trokar kann durch einen Rückfluss des Knochenzementteigs 50 durch das Dreiwege-Ventil 2 in den Auffangbehälter 9 zumindest zum Teil verbraucht werden, ohne dass eine größere Menge des Knochenzementteigs 50 nach vorne austritt.

Das Verbindungsmittel 76 hat grob die Form eines Jochs und weist eine zweizählige Drehsymmetrie sowie eine Spiegelebene als Symmetrieebene auf, wobei die zweizählige Drehsymmetrieachse in der Spiegelebene liegt. Im eingebauten Zustand fällt die zweizählige Drehsymmetrieachse mit der Achse der Kartusche 1, also mit der Zylinderachse der Kartusche 1 zusammen.

Die dem Mischrohr 3 zugewandte Seite des Verbindungsmittels 76 besteht aus zwei Zylindersegmenten, die in einer Ebene parallel zu deren Zylinderachse geschnitten sind, wobei an der Mantelfläche der Zylindersegmente zwei Vertiefungen als Gegenrastungen für ein je ein Rastmittel in den Austragskolben 72, 74 angeordnet sind. Die beiden Zylindersegmente des Verbindungsmittels 76 rasten also mit den Austragskolben 72, 74, wenn sie in die hierfür vorgesehenen Öffnungen auf der dem Mischrohr 3 gegenüberliegenden Rückseite der Austragskolben 72, 74 eingesteckt werden.

Die beiden Zylindersegmente sind über eine Platte miteinander verbunden, in deren Mitte ein zentraler senkrechter Zylinder mit einer kreisförmigen Grundfläche 84 angeordnet ist, die die Anlagefläche 84 für einen Stößel 49 (siehe Figuren 15 und 17 bis 19) einer Auspressvorrichtung bildet. Auf der gegenüberliegenden Seite des zentralen Zylinders ist die Grundfläche abgeschrägt und bildet den Keil 81 beziehungsweise Kegel 81. An der Spitze des Keils 81 oder Kegels 81 ist die Schneide 80 angeordnet, die den Spalt zwischen den beiden Zylindersegmenten überbrückt. Die beiden Zylindersegmente werden über die Platte und den zentralen senkrechten Zylinder in einem festen Abstand zueinander gehalten. Der Abstand ist so gewählt, dass die beiden Austragskolben 72, 74, wenn sie auf die Zylindersegmente des Verbindungsmittels 76 aufgesteckt sind, voneinander mit einem Abstand zueinander gehalten werden, der etwas kleiner oder höchstens genauso groß ist, wie die Stärke der Trennwand 70, also beispielsweise 1 mm. Die Schneide 80 kann ein Einsatz aus einem harten Metall oder einem harten Kunststoff sein, oder das gesamte Verbindungsmittel 76 kann aus einem solchen Material gefertigt sein, das hart genug sein muss, um die Trennwand 70 aufschneiden zu können, wenn das Verbindungsmittel 76 in der Kartusche 1 in Richtung des Mischrohrs 3 vorgetrieben wird.

Von der Auspressvorrichtung ist in den Figuren 17, 18 und 19 nur ein Anschluss 90 zum Verbinden mit den Befestigungsmitteln des Knochenzement-Applikators, ein Stößel 49 und eine Lagerung 92 für den Stößel 49 dargestellt. Diese Teile und die restlichen Bauteile der Auspressvorrichtung entsprechen denen üblicher manuell oder elektrisch oder pneumatisch angetriebener Auspressvorrichtungen. Die Auspressvorrichtung hat ein Fach zur Aufnahme des Knochenzement-Applikators, wobei der Knochenzement-Applikator zumindest an der Vorderseite im Bereich des Gewindes 32 und an der Rückseite am Anschluss 8 stabil gehalten wird. Der Anschluss 90 der Auspressvorrichtung wird mit dem Anschluss 8 des Knochenzement-Applikators verbunden. Der Stößel 49, der als Schubstange 49 wirkt, kann gegen den Anschluss 90 der Auspressvorrichtung in Richtung durch den Anschluss 90 hindurch beziehungsweise in Richtung in die Kartusche 1 hinein bewegt beziehungsweise angetrieben werden, da er in der Lagerung 92 entlang seiner Längsachse beweglich gelagert ist. Dabei drückt die Spitze des Stößels 49 auf die Anlagefläche 84 des Verbindungselements 76. Dadurch wird das Verbindungselement 76 und die beiden Austragskolben 72, 74 in Richtung des Mischrohrs 3 vorgetrieben.

Durch das Aufspreizen der beiden aufgeschnittenen Trennwandteile 70 mit Hilfe des Keils 81 oder Kegels 81 des Verbindungsmittels 76, werden die Trennwandteile 70 in Richtung der Innenwand des zylindrischen Innenraums der Kartusche 1 und weg von dem sich ins Innere der Kartusche 1 bewegenden Stößel 49 gedrückt. Dadurch können die beiden aufgeschnittenen Trennwandteile 70 die weitere Bewegung des Stößels 49 nicht behindern. Hierdurch gelingt es, dass trotz der hohen Zähigkeit der pastösen Ausgangskomponenten 36, 38, trotz dem Strömungswiderstand, der durch den statischen Mischer 14, den langen Schlauch 4 und gegebenenfalls den Trokar (nicht gezeigt) verursacht wird und trotz dem zum Schneiden der Trennwand 70 durch die Schneide 80 notwendigen Kraftaufwand beziehungsweise Energieaufwand der Widerstand gegen die Bewegung des Stößels 49 nicht so groß wird, dass die Kartusche 1 nicht mit herkömmlichen und auch manuell angetriebenen Auspressvorrichtungen auszupressen ist.

Kartusche 1 und Anschluss 8 sind bei allen Ausführungen bevorzugt einteilig ausgeführt und bestehen bevorzugt aus Kunststoff. Bis auf die Dichtungen 44, 53 ,78 können alle Teile erfindungsgemäßer Knochenzement-Applikatoren durch Spritzguss aus Kunststoff gefertigt werden. Die Dichtungen 44, 53 ,78 bestehen bevorzugt aus Gummi. Die Klemmkante 48 beziehungsweise die Schneide 80 besteht bevorzugt aus einem Metall, einer Keramik, einer metallischen Legierung oder einem besonders harten Kunststoff. Die Hülsen 12 und die Einsätze 18 sowie gegebenenfalls der Trokar bestehen bevorzugt ebenfalls aus einem metallischen Werkstoff. Theoretisch können auch die anderen Teile des Knochenzement-Applikators aus metallischen Werkstoffen gefertigt sein. Als Ausgangskomponenten werden bevorzugt pastöse Ausgangskomponenten eines PMMA-Knochenzements verwendet.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Kartusche
- 2: Dreiwege-Ventil
- 3: Mischrohr
- 4: Schlauch
- 5: Luer-System-Adapter
- 6: Applikationsöffnung
- 7: Knebelgriff
- 8: Anschluss
- 9: Auffangbehälter
- 10: Überwurfmutter
- 12: Hülse
- 14: Statischer Mischer
- 16: Ventilsitz
- 18: Einsatz
- 19: Durchgang
- 20: Durchführung
- 22: Stopfen
- 24: Äußere Hülse
- 26: Innengewinde
- 28: Innenteil
- 30: Konus
- 31: Kartuschenkopf
- 32: Außengewinde
- 34: Innere Kartusche
- 36: Erste Ausgangskomponente
- 38: Zweite Ausgangskomponente
- 40: Erster Austragskolben
- 42: Zweiter Austragskolben
- 44: Dichtung
- 46: Pressvorrichtung
- 48: Klemmkante
- 49: Stößel / Schubstange
- 50: Knochenzementteig
- 51: Innenraum der ersten Kartusche
- 52: Innenraum der zweiten Kartusche
- 53: Dichtung
- 54: Überwurfmutter
- 56: Stopfen
- 57: Stopfen
- 58: Innere Kartusche
- 60: Dritte Ausgangskomponente
- 62: Dritter Austragskolben
- 70: Trennwand
- 71: Kartuschenkopf
- 72: Austragskolben
- 74: Austragskolben
- 76: Verbindungsmittel
- 78: Dichtung
- 80: Schneide
- 81: Keil / Kegel
- 82: Rastmittel
- 84: Anlagefläche
- 86: Hohlraum
- 88: Hohlraum
- 90: Anschluss
- 92: Lagerung

## Patentansprüche

1. Knochenzement-Applikator zur Applikation eines Knochenzementteigs (50) im Bereich der Wirbelsäule, der Knochenzement-Applikator aufweisend
zumindest eine röhrenförmige Kartusche (1, 34, 58) mit einem Innenraum (51, 52, 86, 88), wobei Ausgangskomponenten (36, 38, 60) des Knochenzements im Innenraum (51, 52, 86, 88) der zumindest einen Kartusche (1, 34, 58) enthalten sind,
zumindest einen Austragskolben (40, 42, 62, 72, 74) zum Austreiben der Ausgangskomponenten (36, 38, 60) aus der zumindest einen Kartusche (1, 34, 58) durch eine Öffnung der zumindest einen Kartusche (1, 34, 58), wobei der zumindest eine Austragskolben (40, 42, 62, 72, 74) in Längsrichtung im Inneren der zumindest einen Kartusche (1, 34, 58) beweglich ist,
einen Schlauch (4),
eine Applikationsöffnung (6), durch die der Knochenzementteig (50) zu applizieren ist,
ein von außen bedienbares Dreiwege-Ventil (2), das im Schlauch (4) oder an einer der zumindest einen Kartusche (1, 34, 58) zugewandten Seite des Schlauchs (4) angeordnet ist, wobei das Dreiwege-Ventil (2) mit der Öffnung der zumindest einen Kartusche (1, 34, 58) in Fluidverbindung steht,
einen Auffangbehälter (9) zum Aufnehmen von Knochenzementteig (50), der an dem Dreiwege-Ventil (2) angeordnet ist,
wobei das Dreiwege-Ventil (2) derart aufgebaut und in dem Knochenzement-Applikator angeordnet ist, dass es
in einer ersten Stellung eine Fluidverbindung zwischen der Applikationsöffnung (6) und der Öffnung der zumindest einen Kartusche (1, 34, 58) bereitstellt und eine Durchführung (20) zum Auffangbehälter (9) verschließt und
in einer zweiten Stellung eine Fluidverbindung zwischen der Applikationsöffnung (6) und dem Auffangbehälter (9) bereitstellt und einen Durchgang (19) zur Öffnung der zumindest einen Kartusche (1, 34, 58) verschließt.

2. Knochenzement-Applikator nach Anspruch 1, **dadurch gekennzeichnet, dass** zwischen der Öffnung der zumindest einen Kartusche (1, 34, 58) und dem Schlauch (4) oder zwischen der Öffnung der zumindest einen Kartusche (1, 34, 58) und dem Dreiwege-Ventil (2) ein Mischer (14) zum Mischen des Knochenzements, insbesondere ein statischer Mischer (14), angeordnet ist.

3. Knochenzement-Applikator nach Anspruch 2, **dadurch gekennzeichnet, dass** das Dreiwege-Ventil (2) zwischen dem Mischer (14) und dem Schlauch (4) angeordnet ist, wobei das Dreiwege-Ventil (2) in der ersten Stellung eine Fluidverbindung zwischen der Applikationsöffnung (6) und dem Mischer (14) bereitstellt und in der zweiten Stellung den Durchgang (19) zum Mischer (14) verschließt.

4. Knochenzement-Applikator nach Anspruch 1, 2 oder 3, **dadurch gekennzeichnet, dass**
der Knochenzement-Applikator mit Hilfe einer manuell bedienbaren Auspressvorrichtung bedienbar ist und der zumindest eine Austragskolben (40, 42, 62, 72, 74) mit manueller Kraft in der zumindest einen Kartusche (1, 34, 58) bewegbar ist, wobei der Querschnitt des Innenraums (51, 52, 86, 88) der einen Kartusche (1, 34, 58) maximal 3,5 cm² beträgt, bevorzugt maximal 2,5 cm² beträgt, oder der Querschnitt aller Innenräume (51, 52, 86, 88) der Kartuschen (1, 34, 58) zusammen maximal 3,5 cm² beträgt, bevorzugt maximal 2,5 cm² beträgt, und/oder die Schubfläche des zumindest einen Austragskolbens (40, 42, 62, 72, 74) maximal 3,5 cm² beträgt, bevorzugt maximal 2,5 cm² beträgt.

5. Knochenzement-Applikator nach einem der vorangehenden Ansprüche,
**dadurch gekennzeichnet, dass**
der Schlauch (4) zumindest bereichsweise flexibel ist.

6. Knochenzement-Applikator nach einem der vorangehenden Ansprüche,
**dadurch gekennzeichnet, dass**
die Applikationsöffnung (6) in einem Anschluss (5) mit einem Innengewinde (26), insbesondere in einem Luer-System-Adapter (5), oder in einem Trokar angeordnet ist.

7. Knochenzement-Applikator nach einem der vorangehenden Ansprüche,
**dadurch gekennzeichnet, dass**
der Auffangbehälter (9) nach außen für den Knochenzementteig (50) undurchlässig ist, vorzugsweise der Auffangbehälter (9) flüssigkeitsdicht oder flüssigkeitsdicht und gasdicht ist.

8. Knochenzement-Applikator nach einem der vorangehenden Ansprüche,
**dadurch gekennzeichnet, dass**
der Auffangbehälter (9) ein Volumen aufweist, das mindestens so groß ist wie die Hälfte des Volumens des Schlauchs (4), bevorzugt mindestens so groß ist wie das Volumen des Schlauchs (4).

9. Knochenzement-Applikator nach einem der vorangehenden Ansprüche,
**dadurch gekennzeichnet, dass**
die zumindest eine Kartusche (1, 34, 58) an der Rückseite ein Befestigungselement (8) zur Befestigung einer Auspressvorrichtung aufweist.

10. Knochenzement-Applikator nach einem der vorangehenden Ansprüche, **gekennzeichnet durch**
eine erste röhrenförmige Kartusche (1) mit einem ersten zylindrischen Innenraum (51), wobei in dem Innenraum (51) eine erste Ausgangskomponente (36) eines Knochenzements enthalten ist,
einen ersten Austragskolben (40) der axial beweglich im ersten Innenraum (51) der ersten Kartusche (1) angeordnet ist und der zum Austreiben der ersten Ausgangskomponente (36) aus der ersten Kartusche (1) durch eine dem ersten Austragskolben (40) gegenüberliegende Öffnung in einem Kartuschenkopf (31) der ersten Kartusche (1) vorgesehen ist,
eine zweite röhrenförmige Kartusche (34), die innerhalb der ersten röhrenförmigen Kartusche (1) angeordnet ist, wobei in der zweiten Kartusche (34) eine zweite Ausgangskomponente (38) des Knochenzements enthalten ist und ein zweiter Austragskolben (42) angeordnet ist, wobei mit dem zweiten Austragskolben (42) die zweite Ausgangskomponente (38) aus der zweiten Kartusche (34) durch eine gegenüberliegende Öffnung der zweiten Kartusche (34) im Bereich des Kartuschenkopfs (31) der ersten Kartusche (1) auszutreiben ist,
wobei vom Kartuschenkopf (31) aus gesehen hinter dem ersten Austragskolben (40) und dem zweiten Austragskolben (42) eine axial im Innenraum (51) der ersten Kartusche (1) vortreibbare Pressvorrichtung (46) mit einer Klemmkante (48) zum Zusammenpressen der zweiten Kartusche (34) angeordnet ist, wobei die Pressvorrichtung (46) derart in Richtung des Kartuschenkopfs (31) vortreibbar ist, dass während der Bewegung der Pressvorrichtung (46) die zweite Kartusche (34) axial fortlaufend zusammengepresst wird und dabei der erste Austragskolben (40) und der zweite Austragskolben (42) in Richtung des Kartuschenkopfs (31) vorgetrieben werden.

11. Knochenzement-Applikator nach Anspruch 10, **dadurch gekennzeichnet, dass** die zweite Kartusche (34) mit ihrer Außenwand an der Innenwand der ersten Kartusche (1) anliegt und an der Innenwand der ersten Kartusche (1) befestigt ist.

12. Knochenzement-Applikator nach Anspruch 10 oder 11, **dadurch gekennzeichnet, dass**
beim Vortreiben der Pressvorrichtung (46) der erste Austragskolben (40) und der zweite Austragskolben (42) parallel zueinander vorgetrieben werden.

13. Knochenzement-Applikator nach Anspruch 12, **dadurch gekennzeichnet, dass** der erste Austragskolben (40) und der zweite Austragskolben (42) auf gleicher Höhe in Richtung des Kartuschenkopfs (31) laufen.

14. Knochenzement-Applikator nach einem der Ansprüche 10 bis 13, **dadurch gekennzeichnet, dass**
die Klemmkante (48) gegen die Längsachse der ersten Kartusche (1) geneigt ist, vorzugsweise mit einem Winkel von wenigstens 40° senkrecht zur Längsachse in Richtung der Innenwand der ersten Kartusche (1) geneigt ist, besonders bevorzugt mit einem Winkel zwischen 40° und 80° senkrecht zur Längsachse in Richtung der Innenwand der ersten Kartusche (1) geneigt ist.

15. Knochenzement-Applikator nach einem der Ansprüche 10 bis 14, **dadurch gekennzeichnet, dass**
zwischen der Pressvorrichtung (46) und der Innenwand der ersten Kartusche (1) im Bereich der zweiten Kartusche (34) ein Spalt vorgesehen ist, der genauso breit oder breiter ist als die Dicke der Wand der zweiten Kartusche (34).

16. Knochenzement-Applikator nach einem der Ansprüche 10 bis 15, **dadurch gekennzeichnet, dass**
beim Vortreiben der Pressvorrichtung (46) in Richtung Kartuschenkopf (31) die Klemmkante (48) durch Quetschung der zweiten Kartusche (34) die so deformierte Wand der zweiten Kartusche (34) gegen die Unterseite des zweiten Austragskolbens (42) gepresst wird und so den zweiten Austragskolben (42) in Richtung Kartuschenkopf (31) schiebt.

17. Knochenzement-Applikator nach einem der Ansprüche 10 bis 16, **dadurch gekennzeichnet, dass**
eine dritte röhrenförmige Kartusche (58) innerhalb der ersten röhrenförmigen Kartusche (1) angeordnet ist, wobei die dritte Kartusche (58) mit ihrer Außenwand an der Innenwand der ersten Kartusche (1) anliegt und an der Innenwand der ersten Kartusche (1) befestigt ist, wobei in der dritten Kartusche (58) die zweite Ausgangskomponente oder eine dritte Ausgangskomponente (60) des Mehrkomponenten-Knochenzements enthalten ist und ein dritter Austragskolben (62) angeordnet ist, wobei mit dem dritten Austragskolben (62) die zweite Ausgangskomponente oder die dritte Ausgangskomponente (60) aus der dritten Kartusche (58) durch eine gegenüberliegende Öffnung in der dritten Kartusche (58) im Bereich des Kartuschenkopfs (31) der ersten Kartusche (1) auszutreiben ist, wobei die Pressvorrichtung (46) vom Kartuschenkopf (31) aus gesehen hinter dem dritten Austragskolben (62) angeordnet ist und die Pressvorrichtung (46) eine Klemmkante (48) zum Zusammenpressen der dritten Kartusche (58) aufweist, wobei die Pressvorrichtung (46) derart in Richtung des Kartuschenkopfs (31) vortreibbar ist, dass während der Bewegung der Pressvorrichtung (46) die dritte Kartusche (58) axial fortlaufend zusammengepresst wird und dabei der erste Austragskolben (40), der zweite Austragskolben (42) und der dritte Austragskolben (62) in Richtung des Kartuschenkopfs (31) vorgetrieben werden.

18. Knochenzement-Applikator nach einem der Ansprüche 1 bis 9, **gekennzeichnet durch**
eine röhrenförmige Kartusche (1), wobei der Innenraum (86, 88) der Kartusche (1) zylindrisch ist,
einen Kartuschenkopf (71), der ein Ende der röhrenförmigen Kartusche (1) begrenzt,
eine axial in dem zylindrischen Innenraum (86, 88) der Kartusche (1) angeordnete Trennwand (70), wobei die Trennwand (70) mit der Mantelfläche des zylindrischen Innenraums (86, 88) der Kartusche (1) verbunden ist und wobei die Trennwand (70) den durch den Kartuschenkopf (71) begrenzten zylindrischen Innenraum (86, 88) der Kartusche (1) in zwei räumlich voneinander getrennte Hohlräume (86, 88) teilt, wobei in dem ersten Hohlraum (86) eine erste pastenförmige Ausgangskomponente (36) des Knochenzements enthalten ist und in dem separaten zweiten Hohlraum (88) eine zweite pastenförmige Ausgangskomponente (38) des Knochenzements enthalten ist, zwei axial in beiden Hohlräumen (86, 88) der Kartusche (1) verschiebbar angeordnete Austragskolben (72, 74), wobei die Austragskolben (72, 74) die beiden Hohlräume (86, 88) auf der dem Kartuschenkopf (71) gegenüberliegenden Seite der Hohlräume (86, 88) abschließen,
wobei die Austragskolben (72, 74) an der dem Kartuschenkopf (71) gegenüberliegenden Rückseite über ein Verbindungsmittel (76) miteinander verbunden sind, wobei an dem Verbindungsmittel (76) ein Keil (81) oder Kegel (81) mit einer Schneide (80) an der zum Kartuschenkopf (71) weisenden Vorderseite des Keils (81) oder Kegels (81) angeordnet ist, so dass beim Vortreiben der Austragskolben (72, 74) in den Hohlräumen (86, 88) in Richtung des Kartuschenkopfs (71) die Schneide (80) die Trennwand (70) aufschneidet und der Keil (81) oder Kegel (81) die aufgeschnittene Teile der Trennwand (70) in Richtung der Innenwand der Kartusche (1) drückt.

19. Knochenzement-Applikator nach Anspruch 18, **dadurch gekennzeichnet, dass** die Austragskolben (72, 74) über das Verbindungsmittel (76) derart voneinander beabstandet sind, dass der zwischen den Austragskolben (72, 74) liegende Spalt kleiner oder gleich groß ist, wie die Stärke der Trennwand (70).

## Claims

1. Bone cement applicator for application of a bone cement dough (50) in the region of the spine, the bone cement applicator comprising
at least one tubular cartridge (1, 34, 58) with an internal space (51, 52, 86, 88), whereby the internal space (51, 52, 86, 88) of the at least one cartridge (1, 34, 58) contains starting components (36, 38, 60) of the bone cement;
at least one dispensing plunger (40, 42, 62, 72, 74) for expelling the starting components (36, 38, 60) from the at least one cartridge (1, 34, 58) through an opening of the at least one cartridge (1, 34, 58), whereby the at least one dispensing plunger (40, 42, 62, 72, 74) is mobile in longitudinal direction on the inside of the at least one cartridge (1, 34, 58);
a hose (4);
an application opening (6) through which the bone cement dough (50) is applicable;
a three-way valve (2) being operable from outside and being arranged in the hose (4) or on a side of the hose (4) facing the at least one cartridge (1, 34, 58), whereby the three-way valve (2) is in fluid connection with the opening of the at least one cartridge (1, 34, 58);
a collecting container (9) arranged on the three-way valve (2) for accommodation of bone cement dough (50);
whereby the three-way valve (2) is appropriately designed and is appropriately arranged in the bone cement applicator such that it,
being in a first position, provides a fluid connection between the application opening (6) and the opening of the at least one cartridge (1, 34, 58) and closes a feed-through (20) to the collecting container (9) and,
being in a second position, provides a fluid connection between the application opening (6) and the collecting container (9) and closes a passage (19) to the opening of the at least one cartridge (1, 34, 58).

2. Bone cement applicator according to claim 1, **characterised in that** a mixer (14) for mixing of the bone cement, in particular a static mixer (14), is arranged between the opening of the at least one cartridge (1, 34, 58) and the hose (4) or between the opening of the at least one cartridge (1, 34, 58) and the three-way valve (2).

3. Bone cement applicator according to claim 2, **characterised in that** the three-way valve (2) is arranged between the mixer (14) and the hose (4), whereby the three-way valve (2), being in the first position, provides a fluid connection between the application opening (6) and the mixer (14) and, being in the second position, closes the passage to the mixer (14).

4. Bone cement applicator according to claim 1, 2 or 3, **characterised in that** the bone cement applicator is operable by means of a manually operated extrusion device and **in that** the at least one dispensing plunger (40, 42, 62, 72, 74) is movable in the at least one cartridge (1, 34, 58) by manual force, whereby the cross-section of the internal space (51, 52, 86, 88) of the one cartridge (1, 34, 58) is maximally 3.5 cm², preferably is maximally 2.5 cm², or the cross-section of all internal spaces (51, 52, 86, 88) of the cartridges (1, 34, 58) taken together is maximally 3.5 cm², preferably is maximally 2.5 cm², and/or that the propulsion area of the at least one dispensing plunger (40, 42, 62, 72, 74) is maximally 3.5 cm², preferably is maximally 2.5 cm².

5. Bone cement applicator according to any one of the preceding claims,
**characterised in that**
at least part of the hose (4) is flexible.

6. Bone cement applicator according to any one of the preceding claims,
**characterised in that**
the application opening (6) is arranged in a connection (5) with an internal thread (26), in particular in a Luer system adapter (5), or in a trocar.

7. Bone cement applicator according to any one of the preceding claims,
**characterised in that**
the collecting container (9) is impermeable for the bone cement dough (50) towards the outside, preferably the collecting container (9) is fluid-tight or fluid-tight and gas-tight.

8. Bone cement applicator according to any one of the preceding claims,
**characterised in that**
the collecting container (9) has a volume that is at least as large as half the volume of the hose (4), preferably is at least as large as the volume of the hose (4).

9. Bone cement applicator according to any one of the preceding claims,
**characterised in that**
the at least one cartridge (1, 34, 58) comprises, on its rear side, an attachment element (8) for attachment of an extrusion device.

10. Bone cement applicator according to any one of the preceding claims,
**characterised by**
a first tubular cartridge (1) with a first cylindrical internal space (51), whereby a first starting component (36) of a bone cement is contained in the internal space (51);
a first dispensing plunger (40) that is arranged in the first internal space (51) of the first cartridge (1) such as to be axially mobile and that is provided for expelling the first starting component (36) from the first cartridge (1) through an opening in a cartridge head (31) of the first cartridge (1) that is situated opposite from the first dispensing plunger (40);
a second tubular cartridge (34) that is arranged inside the first tubular cartridge (1), whereby the second cartridge (34) contains a second starting component (38) of the bone cement and has a second dispensing plunger (42) arranged in it, whereby the second dispensing plunger (42) is usable to expel the second starting component (38) from the second cartridge (34) through an opposite opening of the second cartridge (34) in the region of the cartridge head (31) of the first cartridge (1);
whereby a pressing device (46) is arranged in the internal space (51) of the first cartridge (1) behind the first dispensing plunger (40) and the second dispensing plunger (42), as seen from the cartridge head (31), the pressing device (46) comprising a clamping edge (48) for compressing the second cartridge (34) and being propellable axially, whereby the pressing device (46) is propellable appropriately in the direction of the cartridge head (31) such that the second cartridge (34) is being progressively compressed axially during the motion of the pressing device (46) thereby propelling the first dispensing plunger (40) and the second dispensing plunger (42) in the direction of the cartridge head (31).

11. Bone cement applicator according to claim 10, **characterised in that**
the external wall of the second cartridge (34) touches against the internal wall of the first cartridge (1) and is attached to the internal wall of the first cartridge (1).

12. Bone cement applicator according to claim 10 or 11, **characterised in that**
the first dispensing plunger (40) and the second dispensing plunger (42) are propelled parallel with respect to each other during the propulsion of the pressing device (46).

13. Bone cement applicator according to claim 12, **characterised in that**
the first dispensing plunger (40) and the second dispensing plunger (42) run at the same level in the direction of the cartridge head (31).

14. Bone cement applicator according to any one of the claims 10 to 13,
**characterised in that**
the clamping edge (48) is inclined with respect to the longitudinal axis of the first cartridge (1), preferably is inclined at an angle of at least 40° perpendicular to the longitudinal axis in the direction of the internal wall of the first cartridge (1), particularly preferably is inclined at an angle between 40° and 80° perpendicular to the longitudinal axis in the direction of the internal wall of the first cartridge (1).

15. Bone cement applicator according to any one of the claims 10 to 14,
**characterised in that**
a gap is provided between the pressing device (46) and the internal wall of the first cartridge (1) in the region of the second cartridge (34), whereby the gap is as wide as or wider than the thickness of the wall of the second cartridge (34).

16. Bone cement applicator according to any one of the claims 10 to 15,
**characterised in that**
the clamping edge (48), by squeezing the second cartridge (34), presses the thus deformed wall of the second cartridge (34) against the underside of the second dispensing plunger (42) and thus pushes the second dispensing plunger (42) in the direction of the cartridge head (31) while the pressing device (46) is being propelled in the direction of the cartridge head (31).

17. Bone cement applicator according to any one of the claims 10 to 16,
**characterised in that**
a third tubular cartridge (58) is arranged inside the first tubular cartridge (1), whereby the external wall of the third cartridge (58) touches against the internal wall of the first cartridge (1) and is attached to the internal wall of the first cartridge (1), whereby the third cartridge (58) contains the second starting component or a third starting component (60) of the multicomponent bone cement and has a third dispensing plunger (62) arranged in it, whereby the second starting component or the third starting component (60) is expellable from the third cartridge (58), by means of the third dispensing plunger (62), through an opposite opening in the third cartridge (58) in the region of the cartridge head (31) of the first cartridge (1), whereby the pressing device (46) is arranged behind the third dispensing plunger (62) as seen from the cartridge head (31) and the pressing device (46) comprises a clamping edge (48) for compressing the third cartridge (58), whereby the pressing device (46) is propellable appropriately in the direction of the cartridge head (31) such that the third cartridge (58) is progressively compressed axially while the pressing device (46) moves and thus the first dispensing plunger (40), the second dispensing plunger (42), and the third dispensing plunger (62) are being propelled in the direction of the cartridge head (31).

18. Bone cement applicator according to any one of the claims 1 to 9, **characterised by**
a tubular cartridge (1), whereby the internal space (86, 88) of the cartridge (1) is cylindrical;
a cartridge head (71) that limits an end of the tubular cartridge (1);
a separating wall (70) in an axial arrangement in the cylindrical internal space (86, 88) of the cartridge (1), whereby the separating wall (70) is connected to the jacket surface of the cylindrical internal space (86, 88) of the cartridge (1), and whereby the separating wall (70) subdivides the cylindrical internal space (86, 88) of the cartridge (1), which is limited by the cartridge head (71), into two spatially separated hollow spaces (86, 88), whereby the first hollow space (86) contains a first pasty starting component (36) of the bone cement, and the separate second hollow space (88) contains a second pasty starting component (38) of the bone cement;
two dispensing plungers (72, 74) that are arranged in the two hollow spaces (86, 88) of the cartridge (1) such as to be axially displaceable, whereby the dispensing plungers (72, 74) close off the two hollow spaces (86, 88) on the side of the hollow spaces (86, 88) opposite from the cartridge head (71); whereby the dispensing plungers (72, 74) are connected to each other on the rear side opposite from the cartridge head (71) by means of a connecting means (76), whereby a wedge (81) or cone (81) with a blade (80) on the front side of the wedge (81) or cone (81) facing the cartridge head (71) is arranged on the connecting means (76) such that, upon propulsion of the dispensing plungers (72, 74) in the hollow spaces (86, 88) in the direction of the cartridge head (71), the blade (80) cuts open the separating wall (70) and the wedge (81) or cone (81) pushes the cut-open parts of the separating wall (70) in the direction of the internal wall of the cartridge (1).

19. Bone cement applicator according to claim 18, **characterised in that**
the dispensing plungers (72, 74) are situated at an appropriate distance from each other by means of the connecting means (76) such that the gap between the dispensing plungers (72, 74) is smaller than or equal to the thickness of the separating wall (70).

## Revendications

1. Applicateur de ciment osseux permettant l'application d'une pâte de ciment osseux (50) dans la région de la colonne vertébrale, l'applicateur de ciment osseux présentant
au moins une cartouche (1, 34, 58) en forme de tube avec un espace intérieur (51, 52, 86, 88), où des composants de départ (36, 38, 60) du ciment osseux sont contenus dans l'espace intérieur (51, 52, 86, 88) de l'au moins une cartouche (1, 34, 58),
au moins un piston de décharge (40, 42, 62, 72, 74) permettant d'expulser les composants de départ (36, 38, 60) de l'au moins une cartouche (1, 34, 58) par un orifice de l'au moins une cartouche (1, 34, 58), où l'au moins un piston de décharge (40, 42, 62, 72, 74) est mobile dans la direction longitudinale à l'intérieur de l'au moins une cartouche (1, 34, 58),
un tuyau (4),
un orifice d'application (6) par lequel la pâte de ciment osseux (50) est à appliquer,
une soupape à trois voies (2) pouvant être actionnée de l'extérieur, qui est disposée dans le tuyau (4) ou sur le côté du tuyau (4) orienté vers l'au moins une cartouche (1, 34, 58), où la soupape à trois voies (2) est en communication fluidique avec l'orifice de l'au moins une cartouche (1, 34, 58),
un récipient de récupération (9) permettant la récupération de pâte de ciment osseux (50), qui est disposé dans la soupape à trois voies (2),
où la soupape à trois voies (2) est conçue de telle manière et est disposée dans l'applicateur de ciment osseux qu'elle
établit une communication fluidique entre l'orifice d'application (6) et l'orifice de l'au moins une cartouche (1, 34, 58) dans une première position, et ferme un passage (20) vers le récipient de récupération (9), et
établit une communication fluidique entre l'orifice d'application (6) et le récipient de récupération (9) dans une deuxième position, et ferme un passage (19) vers l'orifice de l'au moins une cartouche (1, 34, 58).

2. Applicateur de ciment osseux selon la revendication 1, **caractérisé en ce qu'**un mélangeur (14) pour mélanger le ciment osseux, notamment un mélangeur statique (14), est disposé entre l'orifice de l'au moins une cartouche (1, 34, 58) et le tuyau (4) ou entre l'orifice de l'au moins une cartouche (1, 34, 58) et la soupape à trois voies (2).

3. Applicateur de ciment osseux selon la revendication 2, **caractérisé en ce que** la soupape à trois voies (2) est disposée entre le mélangeur (14) et le tuyau (4), où la soupape à trois voies (2) établit une communication fluidique entre l'orifice d'application (6) et le mélangeur (14) dans la première position et ferme le passage (19) vers le mélangeur (14) dans la deuxième position.

4. Applicateur de ciment osseux selon la revendication 1, la revendication 2 ou la revendication 3, **caractérisé en ce que**
l'applicateur de ciment osseux peut être actionné à l'aide d'un dispositif de pressage pouvant être actionné manuellement et l'au moins un piston de décharge (40, 42, 62, 72, 74) est mobile dans l'au moins une cartouche (1, 34, 58) avec une force manuelle, où la section transversale de l'espace intérieur (51, 52, 86, 88) de la cartouche (1, 34, 58) est d'au maximum 3,5 cm², de préférence, est au maximum de 2,5 cm², ou la section transversale de tous les espaces intérieurs (51, 52, 86, 88) des cartouches (1, 34, 58) dans leur ensemble est au maximum de 3,5 cm², est de préférence au maximum de 2,5 cm², et/ou la surface de déplacement de l'au moins un piston de décharge (40, 42, 62, 72, 74) est d'au maximum 3,5 cm², de préférence, est d'au maximum 2,5 cm².

5. Applicateur de ciment osseux selon l'une des revendications précédentes, **caractérisé en ce que**
le tuyau (4) est souple au moins par endroits.

6. Applicateur de ciment osseux selon l'une des revendications précédentes, **caractérisé en ce que**
l'orifice d'application (6) est disposé dans un raccordement (5) avec un filetage intérieur (26), notamment dans un adaptateur de système Luer (5), ou dans un trocart.

7. Applicateur de ciment osseux selon l'une des revendications précédentes, **caractérisé en ce que**
le récipient de récupération (9) ne laisse pas passer la pâte de ciment osseux (50) vers l'extérieur, le récipient de récupération (9) est de préférence étanche vis-à-vis des liquides ou étanche vis-à-vis des liquides et des gaz.

8. Applicateur de ciment osseux selon l'une des revendications précédentes, **caractérisé en ce que**
le récipient de récupération (9) présente un volume qui est au moins aussi grand que la moitié du volume du tuyau (4), de préférence, est au moins aussi grand que le volume du tuyau (4).

9. Applicateur de ciment osseux selon l'une des revendications précédentes, **caractérisé en ce que**
l'au moins une cartouche (1, 34, 58) présente au niveau de son côté arrière un élément de fixation (8) pour la fixation d'un dispositif de pressage.

10. Applicateur de ciment osseux selon l'une des revendications précédentes, **caractérisé par**
une première cartouche (1) en forme de tube avec un premier espace intérieur (51) cylindrique, où un premier composant de départ (36) d'un ciment osseux est contenu dans l'espace intérieur (51),
un premier piston de décharge (40), qui est disposé mobile axialement dans le premier espace intérieur (51) de la première cartouche (1) et qui est prévu pour l'expulsion du premier composant de départ (36) de la première cartouche (1) par un orifice situé en face du premier piston de décharge (40) dans une tête de cartouche (31) de la première cartouche (1),
une deuxième cartouche (34) en forme de tube, qui est disposée à l'intérieur de la première cartouche (1) en forme de tube, où, un deuxième composant de départ (38) du ciment osseux est contenu dans la deuxième cartouche (34) et un deuxième piston de décharge (42) est disposé, où le deuxième composant de départ (38) est expulsé avec le deuxième piston de décharge (42) hors de la deuxième cartouche (34) par un orifice de la deuxième cartouche (34) situé en face dans la région de la tête de cartouche (31) de la première cartouche (1),
où, vu à partir de la tête de cartouche (31), un dispositif de pressage (46), pouvant être avancé axialement dans l'espace intérieur (51) de la première cartouche (1), doté d'un bord de pincement (48) pour le pressage de la deuxième cartouche (34), est disposé derrière le premier piston de décharge (40) et le deuxième piston de décharge (42), où le dispositif de pressage (46) peut être avancé en direction de la tête de cartouche (31) de telle manière que, pendant le déplacement du dispositif de pressage (46), la deuxième cartouche (34) est pressée axialement de manière continue et ainsi le premier piston de décharge (40) et le deuxième piston de décharge (42) sont avancés en direction de la tête de cartouche (31).

11. Applicateur de ciment osseux selon la revendication 10, **caractérisé en ce que**
la deuxième cartouche (34) est plaquée contre la paroi intérieure de la première cartouche (1) avec sa paroi extérieure et est fixée sur la paroi intérieure de la première cartouche (1).

12. Applicateur de ciment osseux selon la revendication 10 ou la revendication 11,
**caractérisé en ce que**,
lors du déplacement vers l'avant du dispositif de pressage (46), le premier piston de décharge (40) et le deuxième piston de décharge (42) sont déplacés vers l'avant parallèlement l'un par rapport à l'autre.

13. Applicateur de ciment osseux selon la revendication 12, **caractérisé en ce que**
le premier piston de décharge (40) et le deuxième piston de décharge (42) s'étendent sur le même niveau en direction de la tête de cartouche (31).

14. Applicateur de ciment osseux selon l'une des revendications 10 à 13, **caractérisé en ce que**
le bord de pincement (48) est incliné contre l'axe longitudinal de la première cartouche (1), de préférence, est incliné avec un angle d'au moins 40 ° perpendiculairement par rapport à l'axe longitudinal en direction de la paroi intérieure de la première cartouche (1), de manière particulièrement préférée, est incliné avec un angle entre 40 ° et 80 ° perpendiculairement par rapport à l'axe longitudinal en direction de la paroi intérieure de la première cartouche (1).

15. Applicateur de ciment osseux selon l'une des revendications 10 à 14, **caractérisé en ce**
**qu'**une fente, qui est exactement aussi large ou plus large que l'épaisseur de la paroi de la deuxième cartouche (34), est prévue entre le dispositif de pressage (46) et la paroi intérieure de la première cartouche (1) dans la région de la deuxième cartouche (34).

16. Applicateur de ciment osseux selon l'une des revendications 10 à 15, **caractérisé en ce que**,
lors du déplacement vers l'avant du dispositif de pressage (46) en direction de la tête de cartouche (31), le bord de pincement (48), par un pincement de la deuxième cartouche (34), la paroi de la deuxième cartouche (34) ainsi déformée est pressée contre la face inférieure du deuxième piston de décharge (42) et pousse ainsi le deuxième piston de décharge (42) en direction de la tête de cartouche (31).

17. Applicateur de ciment osseux selon l'une des revendications 10 à 16, **caractérisé en ce**
**qu'**une troisième cartouche (58) en forme de tube est disposée à l'intérieur de la première cartouche (1) en forme de tube, où la troisième cartouche (58) est plaquée avec sa paroi extérieure contre la paroi intérieure de la première cartouche (1) et est fixée avec sa paroi extérieure sur la paroi intérieure de la première cartouche (1), où le deuxième composant de départ ou un troisième composant de départ (60) du ciment osseux à plusieurs composants est contenu dans la troisième cartouche (58), et un troisième piston de décharge (62) est disposé, où le deuxième composant de départ ou le troisième composant de départ (60) peut être expulsé de la troisième cartouche (58) par un orifice se situant en face dans la troisième cartouche (58) dans la région de la tête de cartouche (31) de la première cartouche (1), où le dispositif de pressage (46) est disposé derrière le troisième piston de décharge (62), vu à partir de la tête de cartouche (31), et le dispositif de pressage (46) présente un bord de pincement (48) pour le pressage de la troisième cartouche (58), où le dispositif de pressage (46) peut être avancé en direction de la tête de cartouche (31) de telle manière que, pendant le déplacement du dispositif de pressage (46), la troisième cartouche (58) est constamment pressée axialement et ainsi le premier piston de décharge (40), le deuxième piston de décharge (42) et le troisième piston de décharge (62) sont avancés vers l'avant en direction de la tête de cartouche (31).

18. Applicateur de ciment osseux selon l'une des revendications 1 à 9, **caractérisé par**
une cartouche (1) en forme de tube, où l'espace intérieur (86, 88) de la cartouche (1) est cylindrique,
une tête de cartouche (71) qui délimite une extrémité de la cartouche (1) en forme de tube,
une paroi de séparation (70) disposée axialement dans l'espace intérieur (86, 88) cylindrique de la cartouche (1), où la paroi de séparation (70) est reliée avec la surface d'enveloppe de l'espace intérieur (86, 88) cylindrique de la cartouche (1), et où la paroi de séparation (70) divise l'espace intérieur (86, 88) cylindrique de la cartouche (1) délimité par la tête de cartouche (71) en deux espaces creux (86, 88) séparés l'un de l'autre, où un premier composant de départ (36) du ciment osseux en forme de pâte est contenu dans le premier espace creux (86), et un deuxième composant de départ (38) du ciment osseux en forme de pâte est contenu dans le deuxième espace creux (88) séparé, deux pistons de décharge (72, 74) pouvant être déplacés axialement dans les deux espaces creux (86, 88) de la cartouche (1), où les pistons de décharge (72, 74) ferment les deux espaces creux (86, 88) sur le côté des espaces creux (86, 88) situés en face de la tète de cartouche (71),
où les pistons de décharge (72, 74) sont reliés l'un à l'autre sur le côté arrière se situant en face de la tête de cartouche (71) par le biais d'un système de liaison (76), où un coin (81), ou un cône (81), avec un système coupant (80) sur le côté avant du coin (81), ou du cône (81), pointant vers la tête de cartouche (71), est disposé sur le système de liaison (76), de sorte que, lors du déplacement vers l'avant des pistons de décharge (72, 74) dans les espaces creux (86, 88) en direction de la tête de cartouche (71), le système coupant (80) ouvre la paroi de séparation (70) en la coupant et le coin (81), ou le cône (81), presse les parties coupées de la paroi de séparation (70) en direction de la paroi intérieure de la cartouche (1).

19. Applicateur de ciment osseux selon la revendication 18, **caractérisé en ce que**
les pistons de décharge (72, 74) sont espacés l'un de l'autre de telle manière, par le biais du système de liaison (76), que la fente située entre les pistons de décharge (72, 74) est plus petite ou de taille égale à l'épaisseur de la paroi de séparation (70).
